# EUROPEAN PATENT SPECIFICATION

(11) **EP 1 896 422 B1**
(45) Date of publication and mention of the grant of the patent: **17.08.2011**
(21) Application number: 06785319.2
(22) Date of filing: 22.06.2006
(51) Int. Cl.: C07D 221/16, C07D 401/04, C07D 401/12, C07D 401/14, C07D 405/14, C07D 409/04, C07D 417/04, C07D 417/14, C07D 471/04, A61K 31/435, A61P 35/00

(54) **TYROSINE KINASE INHIBITORS**
TYROSINKINASE-HEMMER
INHIBITEURS DE TYROSINE KINASE

(30) Priority: 23.06.2005 US 693229 P; 21.10.2005 US 729061 P; 05.04.2006 US 789473 P
(43) Date of publication of application: 12.03.2008
(73) Proprietor: Merck Sharp & Dohme Corp., Rahway, NJ 07065 (US)
(72) Inventor: DINSMORE, Christopher, J., Rahway, NJ 07065-0907 (US); JEWELL, James, P., Rahway, NJ 07065-0907 (US); KATZ, Jason, D., Rahway, NJ 07065-0907 (US); MACHACEK, Michelle, R., Rahway, NJ 07065-0907 (US); OTTE, Ryan, D., Rahway, NJ 07065-0907 (US); YOUNG, Jonathan, R., Rahway, NJ 07065-0907 (US)
(74) Representative: Hussain, Deeba
(86) International application number: PCT/US2006/024256
(87) International publication number: WO 2007/002258

(56) References cited:
- WO-A-03/084931
- US-A1- 2003 114 432
- CHRISTENSEN J G ET AL: "A selective small molecule inhibitor of c-Met kinase inhibits c-Met-dependent phenotypes in vitro and exhibits cytoreductive antitumor activity in vivo" CANCER RESEARCH, AMERICAN ASSOCIATION FOR CANCER RESEARCH, BALTIMORE, MD, US, vol. 63, no. 21, 1 November 2003 (2003-11-01), pages 7345-7355, XP002328699 ISSN: 0008-5472
- CHRISTENSEN J G ET AL: "c-Met as a target for human cancer and characterization of inhibitors for therapeutic intervention" CANCER LETTERS, NEW YORK, NY, US, vol. 225, no. 1, 8 July 2005 (2005-07-08), pages 1-26, XP004939070 ISSN: 0304-3835

### SEQUENCE LISTING

<110> Merck & Co., Inc.
   Dinsmore, Christopher J.
   Jewell, James P.
   Katz, Jason D.
   Machacek, Michelle R.
   Otte, Ryan D.
   Young, Jonathan R.
<120> TYROSINE KINASE INHBITORS
<130> 21943YS
<150> 60/693,229
   <151> 2005-06-23
<150> 60/729,061
   <151> 2005-10-21
<150> 60/789,473
   <151> 2006-04-05
<160> 1
<170> FastSEQ for Windows Version 4.0
<210> 1
   <211> 15
   <212> PRT
   <213> Artificial Sequence
<220>
   <223> Completely Synthetic Amino Acid Sequence
<400> 1
   Glu Gln Glu Asp Glu Pro Glu Gly Asp Tyr Phe Glu Trp Leu Glu
    1               5                  10                  15

## Description

### BACKGROUND OF THE INVENTION

This invention relates to 5H-benzo[4,5]cyclohepta[1,2-b]pyridine compounds that are inhibitors of tyrosine kinases, in particular the receptor tyrosine kinase MET, and are useful in the treatment of cellular proliferative diseases, for example cancer, hyperplasias, restenosis, cardiac hypertrophy, immune disorders and inflammation.

Recently, members of the MET proto-oncogene family, a subfamily of receptortyrosine kinases, have drawn special attentionto the association between invasion and metastasis. The MET family, including MET (also referred to as c-Met) and RON receptors, can function as oncogenes like most tyrosine kinases. MET has been shown to be overexpressed and/or mutated in a variety of malignancies. A number of MET activating mutations, many of which are located in the tyrosine kinase domain, have been detected in various solid tumors and have been implicated in invasion and metastasis of tumor cells.

The c-Met proto-oncogene encodes the MET receptor tyrosine kinase. The MET receptor is a 190kDa glycosylated dimeric complex composed of a 50kDa alpha chain disulfide-linked to a 145kDa beta chain. The alpha chain is found extracellularly while the beta chain contains extracellular, transmembrane and cytosolic domains. MET is synthesized as a precursor and is proteolytically cleaved to yield mature alpha and beta subunits. It displays structural similarities to semaphoring and plexins, a ligand-receptor family that is involved in cell-cell interaction.

It is known that stimulation of MET via hepatocyte growth factor (also known as scatter factor, HGF/SF) results in a plethora of biological and biochemical effects in the cell. Activation of c-Met signaling can lead to a wide array of cellular responses including proliferation, survival, angiogenesis, wound healing, tissue regeneration, scattering, motility, invasion and branching morphogenesis. HGF/MET signaling also plays a major role in the invasive growth that is found in most tissues, including cartilage, bone, blood vessels, and neurons.

Various c-Met mutations have been well described in multiple solid tumors and some hematologic malignancies. The prototypic c-Met mutation examples are seen in hereditary and sporadic human papillary renal carcinoma (Schmidt, L. et al., Nat. Tenet. 1997, 16, 68-73; Jeffers, M. et al., Proc. Nat. Acad. Sci. 1997, 94, 11445-11500). Other reported examples of c-Met mutations include ovarian cancer, childhood hepatocellular carcinoma, metastatic head and neck squamous cell carcinomas and gastric cancers. HGF/MET has been shown to inhibit anoikis, suspension- induced programmed cell death (apoptosis), in head and neck squamous cell carcinoma cells.

MET signaling is implicated in various cancers, especially renal. The nexus between MET and colorectal cancer has also been established. In addition, when compared to the primary tumor, 70% of colorectal cancer liver metastasis showed MET overexpression. MET is also implicated in glioblastoma. Glioma MET expression correlates with glioma grade, and an analysis of human tumor specimens showed that malignant gliomas have a 7-fold higher HGF content than low-grade gliomas. Multiple studies have demonstrated that human gliomas frequently co-express HGF and MET and that high levels of expression are associated with malignant progression. It was further shown that HGF-MET is able to activate Akt and protect glioma cell lines from apoptotic death, both in vitro and in vivo.

RON shares a similar structure, biochemical features, and biological properties with MET. Studies have shown RON overexpression in a significant fraction of breast carcinomas and colorectal adenocarcinomas, but not in normal breast epithelia or benign lesions. Cross-linking experiments have shown that RON and MET form a non-covalent complex on the cell surface and cooperate in intracellular signaling. RON and MET genes are significantly co-expressed in ovarian cancer cell motility and invasiveness. This suggests that co-expression of these two related receptors might confer a selective advantage to ovarian carcinoma cells during either tumor onset or progression.

A number of reviews on MET and its function as an oncogene have recently been published: Cancer and Metastasis Review 22:309-325 (2003); Nature Reviews/Molecular Cell Biology 4:915-925 (2003); Nature Reviews/Cancer 2:289-300 (2002).

Since dysregulation of the HGF/MET signaling has been implicated as a factor in tumorgenesis and disease progression in many tumors, different strategies for therapeutic inhibition of this important RTK molecule should be investigated. Specific small molecule inhibitors against HGF/MET signaling and against RON/ MET signaling have important therapeutic value for the treatment of cancers in which Met activity contributes to the invasive/metastatic phenotype.

Tricyclic pyrazoles which may inhibit MET are described in US 2003/114432 A. The pyrrolyl methylene indolone PHA-665752 as MET inhibitor is described in Cancer Research 63:7345-7355 (2003).

### SUMMARY OF THE INVENTION

The present invention relates to 5H-benzo[4,5]cyclohepta[1,2-b]pyridine derivatives, that are useful for treating cellular proliferative diseases, for treating disorders associated with MET activity, and for inhibiting the receptor tyrosine kinase MET. The compounds of the invention may be illustrated by the Formula I:

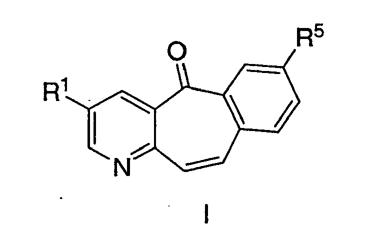

### DETAILED DESCRIPTION OF THE INVENTION

The compounds of this invention are useful in the inhibition of tyrosine kinses, in particular the receptor tyrosine kinase MET, and are illustrated by a compound of Formula I: 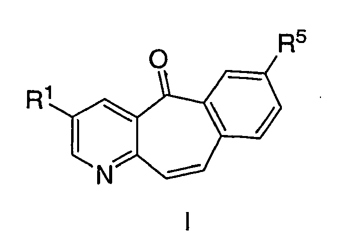 or a pharmaceutically acceptable salt or stereoisomer thereof, wherein

| | |
|---|---|
| a is independently | 0 or 1; |
| b is independently | 0 or 1; |
| m is independently | 0, 1, or 2; |

R¹ is selected from aryl, heterocyclyl and NR¹⁰R¹¹; said aryl and heterocyclyl group optionally substituted with one to five substituents, each substituent independently selected from R⁸;
R⁵ is selected from C₁₋₆alkyl, C₂₋₆ alkenyl, OH, -O-C₁₋₆alkyl, - O-C(=O)C₁₋₆ alkyl, -O-aryl, S(O)ₘR^{a}, -C(=O)NR¹⁰R¹¹, -NHS(O)₂NR¹⁰R¹¹ and NR¹⁰R¹¹, each alkyl, alkenyl and aryl optionally substituted with one to five substituents, each substituent independently selected from R⁸;
R8 independently is: (C=O)ₐO_{b}C₁-C₁₀ alkyl, (C=O)ₐO_{b}aryl, C₂-C₁₀ alkenyl, C₂-C₁₀ alkynyl, (C=O)ₐO_{b} heterocyclyl, CO₂H, halo, CN, OH, O_{b}C₁-C₆ perfluoroalkyl, Oₐ(C=O)_{b}NR¹⁰R¹¹, S(O)ₘR^{a}, S(O)₂NR¹⁰R¹¹, OS(=O)Ra, oxo, CHO, (N=O)R¹⁰R¹¹, or (C=O)ₐO_{b}C₃-C₈ cycloalkyl,
said alkyl, aryl, alkenyl, alkynyl, heterocyclyl, and cycloalkyl optionally substituted with one, two or three substituents selected from R⁹;
R⁹ is independently selected from: (C=O)ₐ0_{b}(C₁-C₁₀)alkyl, O_{b}(C₁-C₃)perfluoroalkyl, oxo, OH, halo, CN, (C₂-C₁₀)alkenyl, (C₂-C₁₀)alkynyl, (C=O)ₐO_{b}(C₃-C₆)cycloalkyl, (C=O)ₐO_{b}(C₀-C₆)alkylene-aryl, (C=O)ₐO_{b}(C₀-C₆)alkylene-heterocyclyl, (C=O)ₐO_{b}(C₀-C₆)alkylene-N(R^{b})₂, C(O)R^{a}, (C₀-C₆)alkylene-CO₂R^{a}, C(O)H, (C₀-C₆)alkylene-CO₂H, C(O)N(R^{b})₂, S(O)mR^{a}, and S(O)₂NR¹⁰R¹¹; said alkyl, alkenyl, alkynyl, cycloalkyl, aryl, and heterocyclyl is optionally substituted with one, two or three substituents selected from R^{b}, OH, (C₁-C₆)alkoxy, halogen, CO₂H, CN, O(C=O)C₁-C₆ alkyl, oxo, and N(R^{b})₂;
R¹⁰ and R¹¹ are independently selected from: H, (C=O)O_{b}C₁-C₁₀ alkyl, (C=O)O_{b}C₃-C₈ cycloalkyl, (C=O)O_{b}aryl, (C=O)O_{b}heterocyclyl, C₁-C₁₀ alkyl, aryl, C₂-C₁₀ alkenyl, C₂-C₁₀ alkynyl, heterocyclyl, C₃-C₈ cycloalkyl, SO₂R^{a} and (C₌O)NR^{b}₂,
said alkyl, cycloalkyl, aryl, heterocylyl, alkenyl, and alkynyl is optionally substituted with one, two or three substituents selected from R⁸, or
R¹⁰ and R¹¹ can be taken together with the nitrogen to which they are attached to form a monocyclic or bicyclic heterocycle with 5-7 members in each ring and optionally containing, in addition to the nitrogen, one or two additional heteroatoms selected from N, O and S, said monocyclic or bicyclic heterocycle optionally substituted with one, two or three substituents selected from R⁹;
R^{a} is independently selected from: (C₁-C₆)alkyl, (C₂-C₆)alkenyl, (C₃-C₆)cycloalkyl, aryl, -(C₁-C₆)alkylenearyl, heterocyclyl and -(C₁-C₆)alkyleneheterocyclyl; and
R^{b} is independently selected from: H, (C₁-C₆)alkyl, aryl, -(C₁-C₆)alkylenearyl, heterocyclyl, -(C₁-C₆)alkyleneheterocyclyl, (C₃-C₆)cycloalkyl, (C=O)OC₁-C₆ alkyl, (C=O)C₁-C₆ alkyl or S(O)₂R^{a}.

Specific examples of the compounds of the instant invention include:
3-phenyl-7-vinyl-5*H*-benzo[4,5]cyclohepta[1,2-*b*]pyridin-5-one;
7-ethyl-3-phenyl-5*H*-benzo[4,5]cyclohepta[1,2-*b*]pyridin-5-one;
7-[(2,4-dimethoxybenzyl)amino]-3-phenyl-5*H*-benzo[4,5]cyclohepta[1,2-*b*]pyridin-5-one;
7-amino-3-phenyl-5*H*-benzo[4,5]cyclohepta[1,2-*b*]pyridin-5-one;
2-hydroxy-N-(5-oxo-3-phenyl-5*H*-benzo[4,5]cyclohepta[1,2-*b*]pyridin-7-yl)propanamide; *N*-methyl-5-oxo-3-phenyl-5*H*-benzo[4,5]cyclohepta[1,2,*b*]pyridine-7-carboxamide;
7-isobutyl-3-phenyl-5*H*-benzo[4,5]cyclohepta[1,2-*b*]pyridin-5-one;
*N*-(5-oxo-3-phenyl-5*H*-benzo[4,5]cyclohepta[1,2-*b*]pyridin-7-yl)methanesulfonamide;
*N*-[5-oxo-3-(3-thienyl)-5*H*-benzo[4,5]cyclohepta[1,2-*b*]pyridin-7-yl]methanesulfonamide;
7-(isopropylamino)-3-(1-methyl-1*H*-pyrazol-4-yl)-5*H*-benzo[4,5]cyclohepta[1,2-*b*]pyridin-5-one;
*N*-[(2*R*)-1,4-dioxan-2-ylmethyl]-*N*-methyl-*N*-[3-(1-methyl-1*H*-pyrazol-4-yl)-5-oxo-5*H-*benzo[4,5]cyclohepta[1,2-*b*]pyridin-7-yl]sulfamide;
*N*-[(2*S*)-1,4-dioxan-2-ylmethyl]-*N*-methyl-*N*'-[3-(1-methyl-1*H*-pyrazol-4-yl)-5-oxo-5*H-*benzo[4,5]cyclohepta[1,2-*b*]pyridin-7-yl]sulfamide;
*N*-[1,4-dioxan-2-ylmethyl]-*N*-methyl-*N'*-[3-(1-methyl-1*H*-pyrazol-4-yl)-5-oxo-5*H-*benzo[4,5]cyclohepta[1,2-*b*]pyridin-7-yl]sulfamide racemic;
*N*-methyl-*N'*-[3-(1-methyl-1*H*-pyrazol-4-yl)-5-oxo-5*H*-benzo[4,5]cyclohepta[1,2-*b*]pyridin-7-yl]-*N-*(tetrahydrofuran-3-yl)sulfamide;
*N*-methyl-*N'*-[3-(1-methyl-1*H*-pyrazol-4-yl)-5-oxo-5*H*-benzo[4,5]cyclohepta[1,2-*b*]pyridin-7-yl]-*N*-({3R)-tetrahydrofuran-3-yl)sulfamide;
*N*-methyl-*N*'-[3-(1-methyl-1*H*-pyrazol-4-yl)-5-oxo-5*H*-benzo[4,5]cyclohepta[1,2-*b*]pyridin-7-yl]-*N*-({3*S*}-tetrahydrofuran-3-yl)sulfamide;
*N*-(5-oxo-3-pyridin-4-yl-5*H*-benzo[4,5]cyclohepta[1,2-*b*]pyridin-7-yl)methanesulfonamide;
*N*-[5-oxo-3-(1*H*-pyrazol-3-yl)-5*H*-benzo[4,5]cyclohepta[1,2-*b*]pyridin-7-yl]methanesulfonamide;
*N*-[5-oxo-3-(1,3-thiazol-4-yl)-5*H*-benzo[4,5]cyclohepta[1,2-*b*]pyridin-7-yl]methanesulfonamide;
*N*-[3-(1-methyl-1*H*-pyrazol-4-yl)-5-oxo-5*H*-benzo[4,5]cyclohepta[1,2-*b*]pyridin-7-yl]methanesulfonamide;
*N*-[5-oxo-3-(1*H*-pyrazol-4-yl)-5*H*-benzo[4,5]cyclohepta[1,2-*b*]pyridin-7-yl]methanesulfonamide;
*N*-(3-{1-[2-(dimethylamino)ethyl]-1*H*-pyrazol-4-yl}-5-oxo-5*H*-benzo[4,5]cyclohepta[1,2-*b*]pyridin-7-yl)methanesulfonamide;
*N*-{3-[1-(2-morpholin-4-yl-2-oxoethyl)-1*H*-pyrazol-4-yl]-5-oxo-5*H*-benzo[4,5]cyclohepta[1,2-*b*]pyridin-7-yl methanesulfonamide;
*N*-(4-{7-[(methylsulfonyl)amino]-5-oxo-5*H*-benzo[4,5]cyclohepta[1,2-*b*]pyridin-3-yl}phenyl)methanesulfonamide;
*N*-[3-(1-cyclopentyl-1*H*-pyrazol-4-yl)-5-oxo-5*H*-benzo[4,5]cyclohepta[1,2-*b*]pyridin-7-yl]methanesulfonamide;
*N-*{3-[1-(3,3-dimethyl-2-oxobutyl)-1*H*-pyrazol-4-yl]-5-oxo-5*H*-benzo[4,5]cyclohepta[1,2-*b*]pyridin-7-yl}methanesulfonamide;
*N*-[2-(1-methylpyrrolidin-2-yl)ethyl]-3-{7-[(methylsulfonyl)amino]-5-oxo-5*H-*benzo[4,5]cyclohepta[1,2-*b*]pyridin-3-yl}benzamide;
*N*,*N*-dimethyl-N-[3-(1-methyl-1*H*-pyrazol-4-yl)-5-oxo-5*H*-benzo[4,5]cyclohepta[1,2-*b*]pyridin-7-yl]sulfamide;
7-(5-methyl-1,1-dioxido-1,2,5-thiadiazelidin-2-yl)-3-(1-methyl-1*H*-pyrazol-4-yl)-5*H-*benzo[4,5]cyclohepta[1,2-*b*]pyridin-5-one;
7-[(2,4-dimethoxybenzyl)amino]-3-(3-thienyl)-5*H*-benzo[4,5]cyclohepta[1,2-*b*]pyridin-5-one; 7-amino-3-(1-methyl-1*H*-pyrazol-4-yl)-5*H*-benzo[4,5]cyclohepta[1,2-*b*]pyridin-5-one;
7-[(2,4-dimethoxybenzyl)amino]-3-(1*H*-pyrazol-3-yl)-5*H*-benzo[4,5]cyclohepta[1,2-*b*]pyridin-5-one;
N-methyl-N'-[3-(1-methyl-1H-pyrazol-4-yl)-5-oxo-5H-benzo[4,5]cyclohepta[1,2-*b*]pyridin-7-yl]-N-(tetrahydrofuran-3-yl)sulfamide;
7-[(imidazo[1,2-a]pyridin-3-ylmethyl)amino]-3-(1-methyl-1*H*-pyrazol-4-yl)-5*H-*benzo[4,5]cyclohepta[1,2-*b*]pyridin-5-one;
7-{[(1-methyl-5-oxopyrrolidin-2-yl)methyl]amino}-3-(1-methyl-1*H*-pyrazol-4-yl)-5*H-*benzo[4,5]cyclohepta[1,2-*b*]pyridin-5-one;
*N*-methyl-*N*'-[3-(1-methyl-1*H*-pyrazol-4-yl)-5-oxo-5*H*-benzo[4,5]cyclohepta[1,2-*b*]pyridin-7-yl]-*N*-(tetrahydro-2*H*-pyran-2-ylmethyl)sulfamide;
*N*-[3-(1-methyl-1*H*-pyrazol-4-yl)-5-oxo-5*H*-benzo [4,5]cyclohepta[1,2-*b*]pyridin-7-yl]-*N*'-(tetrahydrofuran-3-yl)sulfamide;
*N*-[3-(1-methyl-1*H*-pyrazol-4-yl)-5-oxo-5*H*-benzo[4,5]cyclohepta[1,2-*b*]pyridin-7-yl]morpholine-4-sulfonamide;
*N*-[3-(4-isopropylpiperazin-1-yl)-5-oxo-5*H*-benzo[4,5]cyclohepta[1,2-*b*]pyridin-7-yl]methanesulfonamide;
3-(4-isopropylpiperazin-1-yl)-7-phenyl-5H-benzo[4,5]cyclohepta[1,2-b]pyridin-5-one;
*N*-(3-morpholin-4-yl-5-oxo-5*H*-benzo[4,5]cyclohepta[1,2-*b*]pyridin-7-yl)methanesulfonamide;
*N*-(3-anilino-5-oxo-5*H*-benzo[4,5]cyclohepta[1,2-*b*]pyridin-7-yl)methanesulfonamide;
*N*-[3-(cyclohexylamino)-5-oxo-5*H*-benzo[4,5]cyclohepta[1,2-*b*]pyridin-7-yl]methanesulfonamide;
*N*-[5-oxo-3-(pyridin-4-ylamino)-5*H*-benzo[4,5]cyclohepta[1,2-*b*]pyridin-7-yl]methanesulfonamide;
*N*-(2,4-dimethoxybenzyl)-*N*-(5-oxo-3-phenyl-5H-benzo[4,5]cyclohepta[1,2-*b*]pyridin-7-yl)ethylenesulfonamide;
*N*-(5-oxo-3-phenyl-5*H*-benzo[4,5]cyclohepta[1,2-*b*]pyridin-7-yl)ethylenesulfonamide;
*N*-(5-oxo-3-phenyl-5*H*-benzo[4,5]cyclohepta[1,2-*b*]pyridin-7-yl)-2-pyrrolidin-1-ylethanesulfonamide;
dimethyl [3-(1-methyl-1H-pyrazol-4-yl)-5-oxo-5H-benzo[4,5]cyclohepta[1,2-*b*]pyridin-7-yl]amidophosphate;
7-[(1*R*)-1-hydroxyethyl]-3-(1-methyl-1*H*-pyrazol-4-yl)-5*H*-benzo[4,5]cyclohepta[l,2-*b*]pyridin-. 5-one;
7-[(1*S*)-1-hydroxyethyl]-3-(1-methyl-1*H*-pyrazol-4-yl)-5*H*-benzo[4,5]cyclohepta[1,2-*b*]pyridin-5-one;
7-(2-{[*tert*-butyl(dimethyl)silyl]oxy}ethyl)-3-(1-methyl-1*H*-pyrazol-4-yl)-5*H*-benzo[4,5]cyclohepta[1,2-*b*]pyridin-5-one;
7-(2-hydroxyethyl)-3-(1-methyl-1H-pyrazol-4-yl)-5*H*-benzo[4,5]cyclohepta[1,2-*b*]pyridin-5-one;
7-(1,2-dihydroxyethyl)-3-(1-methyl-1*H*-pyrazol-4-yl)-5*H*-benzo[4,5]cyclohepta[1,2-*b*]pyridin-5-one;
7-[(1*R*)-1-methoxyethyl]-3-(1-methyl-1*H*-pyrazol-4-yl)-5*H*-benzo[4,5]cyclohepta[1,2-*b*]pyridin-5-one;
7-[(1*S*)-1-methoxyethyl]-3-(1-methyl-1*H*-pyrazol-4-yl)-5*H*-benzo[4,5]cyclohepta[1,2-*b*]pyridin-5-one;
tert-butyl 4-[2-(3-chloro-5-oxo-5*H*-benzo[4,5]cyclohepta[1,2-*b*]pyridin-7-yl)-2-hydroxyethyl]piperazine-1-carboxylate;
tert-butyl 4-{2-hydroxy-2-[3-(1-methyl-1*H*-pyrazol-4-yl)-5-oxo-5*H*-benzo[4,5]cyclohepta[1,2-*b*]pyridin-7-yl]ethyl} piperazine-1-carboxylate;
7-(1-hydroxy-2-piperazin-1-ylethyl)-3-(1-methyl-1*H*-pyrazol-4-yl)-5*H*-benzo[4,5]cyclohepta[1,2-*b*]pyridin-5-one;
or a pharmaceutically acceptable salt or stereoisomer thereof.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and both tautomeric forms are intended to be encompassed by the scope of the invention, even though only one tautomeric structure is depicted.

It is understood that one or more silicon (Si) atoms can be incorporated into the compounds of the instant disclosure in place of one or more carbon atoms by one of ordinary skill in the art to provide compounds that are chemically stable and that can be readily synthesized by techniques known in the art from readily available starting materials. Carbon and silicon differ in their covalent radius leading to differences in bond distance and the steric arrangement when comparing analogous C-element and Si-element bonds. These differences lead to subtle changes in the size and shape of silicon-containing compounds when compared to carbon. One of ordinary skill in the art would understand that size and shape differences can lead to subtle or dramatic changes in potency, solubility, lack of off target activity, packaging properties, and so on. (Diass, J. O. et al. Organometallics (2006) 5:1188-1198; Showell, G.A. et al. Bioorganic & Medicinal Chemistry Letters (2006) 16:2555-2558).

When any variable (e.g. R⁷, R⁸, R^{b}, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted with one or more substituents" should be taken to be equivalent to the phrase "optionally substituted with at least one substituent" and in such cases another embodiment will have from zero to three substituents.

As used herein, "alkyl" is intended to include both branched and straight-chain saturated aliphatic hydrocarbon groups having the specified number of carbon atoms. For example, C₁-C₁₀, as in "C₁-C₁₀ alkyl" is defined to include groups having 1, 2, 3, 4, 5, 6, 7, 8, 9 or 10 carbons in a linear or branched arrangement. For example, "C₁-C₁₀ alkyl" specifically includes methyl, ethyl, n-propyl, *i-*propyl, n-butyl, t-butyl, i-butyl, pentyl, hexyl, heptyl, octyl, nonyl, decyl, and so on. The term "cycloalkyl" means a monocyclic saturated aliphatic hydrocarbon group having the specified number of carbon atoms. For example, "cycloalkyl" includes cyclopropyl, methyl-cyclopropyl, 2,2-dimethyl-cyclobutyl, 2-ethyl-cyclopentyl, cyclohexyl, and so on. In an embodiment of the invention the term "cycloalkyl" includes the groups described immediately above and further includes monocyclic unsaturated aliphatic hydrocarbon groups. For example, "cycloalkyl" as defined in this embodiment includes cyclopropyl, methyl-cyclopropyl, 2,2-dimethyl-cyclobutyl, 2-ethyl-cyclopentyl, cyclohexyl, cyclopentenyl, cyclobutenyl and so on.

The term "alkylene" means a hydrocarbon diradical group having the specified number of carbon atoms. For example, "alkylene" includes - CH₂-, -CH₂CH₂- and the like.

When used in the phrases "C₁-C₆ aralkyl" and "C₁-C₆ heteroaralkyl" the term "C₁-C₆" refers to the alkyl portion of the moiety and does not describe the number of atoms in the aryl and heteroaryl portion of the moiety.

"Alkoxy" represents either a cyclic or non-cyclic alkyl group of indicated number of carbon atoms attached through an oxygen bridge. "Alkoxy" therefore encompasses the definitions of alkyl and cycloalkyl above.

If no number of carbon atoms is specified, the term "alkenyl," refers to a non-aromatic hydrocarbon radical, straight, branched or cyclic, containing from 2 to 10 carbon atoms and at least one carbon to carbon double bond. Preferably one carbon to carbon double bond is present, and up to four non-aromatic carbon-carbon double bonds may be present. Thus, "C₂-C₆ alkenyl" means an alkenyl radical having from 2 to 6 carbon atoms. Alkenyl groups include ethenyl, propenyl, butenyl, 2-methylbutenyl and cyclohexenyl. The straight, branched or cyclic portion of the alkenyl group may contain double bonds and may be substituted if a substituted alkenyl group is indicated.

The term "alkynyl" refers to a hydrocarbon radical straight, branched or cyclic, containing from 2 to 10 carbon atoms and at least one carbon to carbon triple bond. Up to three carbon-carbon triple bonds may be present. Thus, "C₂-C₆ alkynyl" means an alkynyl radical having from 2 to 6 carbon atoms. Alkynyl groups include ethynyl, propynyl, butynyl, 3-methylbutynyl and so on. The straight, branched or cyclic portion of the alkynyl group may contain triple bonds and may be substituted if a substituted alkynyl group is indicated.

In certain instances, substituents may be defined with a range of carbons that includes zero, such as (C₀-C₆)alkylene-aryl. If aryl is taken to be phenyl, this definition would include phenyl itself as well as -CH₂Ph, -CH₂CH₂Ph, CH(CH₃)CH₂CH(CH₃)Ph, and so on.

As used herein, "aryl" is intended to mean any stable monocyclic or bicyclic carbon ring of up to 7 atoms in each ring, wherein at least one ring is aromatic. Examples of such aryl elements include phenyl, naphthyl, tetrahydronaphthyl, indanyl and biphenyl. In cases where the aryl substituent is bicyclic and one ring is non-aromatic, it is understood that attachment is via the aromatic ring.

The term heteroaryl, as used herein, represents a stable monocyclic or bicyclic ring of up to 7 atoms in each ring, wherein at least one ring is aromatic and contains from 1 to 4 heteroatoms selected from the group consisting of O, N and S. Heteroaryl groups within the scope of this definition include but are not limited to: acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetrahydroquinoline. As with the definition of heterocycle below, "heteroaryl" is also understood to include the N-oxide derivative of any nitrogen-containing heteroaryl. In cases where the heteroaryl substituent is bicyclic and one ring is non-aromatic or contains no heteroatoms, it is understood that attachment is via the aromatic ring or via the heteroatom containing ring, respectively.

The term "heterocycle" or "heterocyclyl" as used herein is intended to mean a 3- to 10-membered aromatic or nonaromatic heterocycle containing from 1 to 4 heteroatoms selected from the group consisting of O, N and S, and includes bicyclic groups. For the purposes of this invention, the term "heterocyclic" is also considered to be synonymous with the terms "heterocycle" and "heterocyclyl" and is understood as also having the definitions set forth herein. "Heterocyclyl" therefore includes the above mentioned heteroaryls, as well as dihydro and tetrathydro analogs thereof. Further examples of "heterocyclyl" include, but are not limited to the following: azetidinyl, benzoimidazolyl, benzofuranyl, benzofurazanyl, benzopyrazolyl, benzotriazolyl, benzothiophenyl, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrahydrothiopyranyl, tetrahydroisoquinolinyl, tetrazolyl, tetrazolopyridyl, thiadiazolyl, thiazolyl, thienyl, triazolyl, 1,4-dioxanyl, hexahydroazepinyl, piperazinyl, piperidinyl, pyridin-2-onyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl, and N-oxides thereof. Attachment of a heterocyclyl substituent can occur via a carbon atom or via a heteroatom.

In an embodiment, the term "heterocycle" or "heterocyclyl" as used herein is intended to mean a 5- to 10-membered aromatic or nonaromatic heterocycle containing from 1 to 4 heteroatoms selected from the group consisting of O, N and S, and includes bicyclic groups. "Heterocyclyl" in this embodiment therefore includes the above mentioned heteroaryls, as well as dihydro and tetrathydro analogs thereof. Further examples of "heterocyclyl" include, but are not limited to the following: benzoimidazolyl, benzofuranyl, benzofurazanyl, benzopyrazolyl, benzotriazolyl, benzothiophenyl, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrahydrothiopyranyl, tetrahydroisoquinolinyl, tetrazolyl, tetrazolopyridyl, thiadiazolyl, thiazolyl, thienyl, triazolyl, azetidinyl, 1,4-dioxanyl, hexahydroazepinyl, piperazinyl, piperidinyl, pyridin-2-onyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl, and N-oxides thereof. Attachment of a heterocyclyl substituent can occur via a carbon atom or via a heteroatom.

In another embodiment, heterocycle is selected from 2-azepinone, benzimidazolyl, 2-diazapinone, imidazolyl, 2-imidazolidinone, indolyl, isoquinolinyl, morpholinyl, piperidyl, piperazinyl, pyridyl, pyrrolidinyl, 2-piperidinone, 2-pyrimidinone, 2-pyrollidinone, quinolinyl, tetrahydrofuryl, tetrahydroisoquinolinyl, and thienyl.

As appreciated by those of skill in the art, "halo" or "halogen" as used herein is intended to include chloro, fluoro, bromo and iodo.

The alkyl, alkenyl, alkynyl, cycloalkyl, aryl, heteroaryl and heterocyclyl substituents may be substituted or unsubstituted, unless specifically defined otherwise. For example, a (C₁-C₆)alkyl may be substituted with one, two or three substituents selected from OH, oxo, halogen, alkoxy, dialkylamino, or heterocyclyl, such as morpholinyl, piperidinyl, and so on. In this case, if one substituent is oxo and the other is OH, the following are included in the definition:
-C=O)CH₂CH(OH)CH₃, -(C=O)OH, -CH₂(OH)CH₂CH(O), and so on.

The moiety formed when, in the definition of two R⁸s or two R⁹s on the same carbon atom are combined to form -(CH₂)ᵤ- is illustrated by the following:

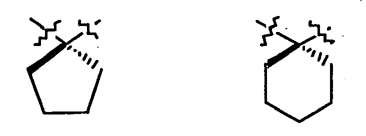

In addition, such cyclic moieties may optionally include one or two heteroatom(s). Examples of such heteroatom-containing cyclic moieties include, but are not limited to:

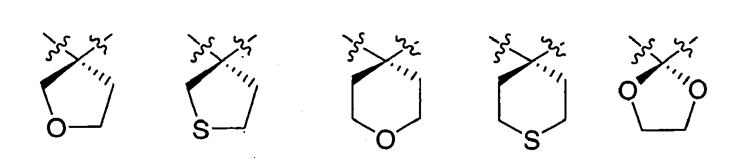

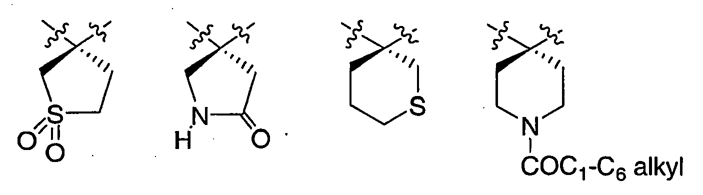

In certain instances, R¹⁰ and R¹¹ are defined such that they can be taken together with the nitrogen to which they are attached to form a monocyclic or bicyclic heterocycle with 5-7 members in each ring and optionally containing, in addition to the nitrogen, one or two additional heteroatoms selected from N, O and S, said heterocycle optionally substituted with one or more substituents selected from R⁸. Examples of the heterocycles that can thus be formed include, but are not limited to the following, keeping in mind that the heterocycle is optionally substituted with one or more (and in another embodiment, one, two or three) substituents chosen from R⁸:

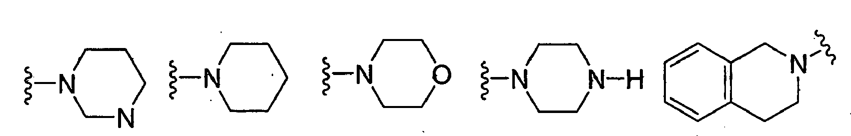

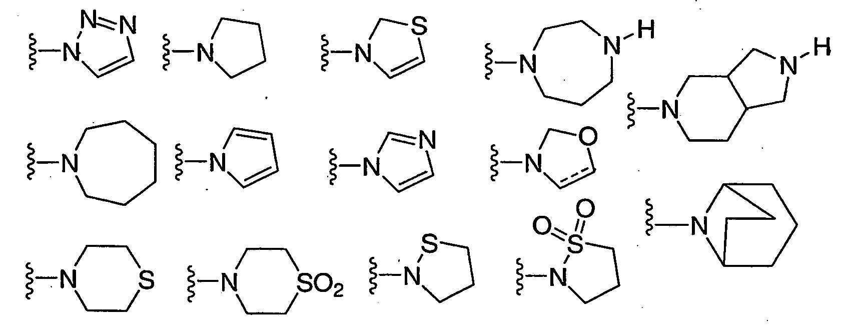

In an embodiment of the Formula I, R¹ is selected from aryl and heterocyclyl; said aryl and heterocyclyl group optionally substituted with one to five substituents, each substituent independently selected from R⁸.

In an embodiment of the compound of the Formula I, R⁵ is selected from C₁₋₆alkyl, C₂₋₆ alkenyl, C₂₋₆ alkynyl, OH, -O-C₁₋₆alkyl, - O-C(=O)C₁₋₆ alkyl, -O-aryl,
S(O)ₘR^{a}, -C(=O)NR¹⁰R¹¹, -NHS(O)₂NR¹⁰R¹¹ and NR¹⁰R¹¹, each alkyl, alkenyl, alkynyl and aryl optionally substituted with one to five substituents, each substituent independently selected from R⁸.

Included in the instant invention is the free form of compounds of Formula I, as well as the pharmaceutically acceptable salts and stereoisomers thereof. Some of the specific compounds exemplified herein are the protonated salts of amine compounds. The term "free form" refers to the amine compounds in non-salt form. The encompassed pharmaceutically acceptable salts not only include the salts exemplified for the specific compounds described herein, but also all the typical pharmaceutically acceptable salts of the free form of compounds of Formula I. The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reacting a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous; lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamin, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like. When the compound of the present invention is acidic, the term "free form" refers to the compound in its non-salt form, such that the acidic functionality is still protonated.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention may potentially be internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom. An isolated compound having internally balance charges, and thus not associated with a intermolecular counterion, may also be considered the "free form" of a compound.

Certain abbreviations, used in the Schemes and Examples, are defined below:
- APCI: Atmospheric pressure chemical ionization
- DMF: Dimethylformamide
- DMSO: Dimethyl sulfoxide
- EtOAc: Ethyl acetate
- LCMS: Liquid chromatographic mass spectrometry
- MPLC: Medium pressure liquid chromatography
- NBS: N-bromosuccinamide
- TFA: Trifluoroacetic acid
- TFAA: Trifluoroacetic anhydride

The compounds of this invention may be prepared by employing reactions as shown in the following schemes, in addition to other standard manipulations that are known in the literature or exemplified in the experimental procedures. The illustrative schemes below, therefore, are not limited by the compounds listed or by any particular substituents employed for illustrative purposes. Substituent numbering as shown in the schemes does not necessarily correlate to that used in the claims and often, for clarity, a single substituent is shown attached to the compound where multiple substituents are allowed under the definitions of Formula I hereinabove.

### SCHEMES

As shown in Scheme A, reaction of a suitably substituted 2-methylnicotinate A-1 with strong base followed by reaction with a suitably substituted bromobenzaldehyde provides the olefin intermediate A-2. Subequent polyphosphonic acid mediated cyclization provides the intermediate/compound of the invention A-3.

Scheme B illustrates the use of intermediate A-3 in the preparation of instant compounds having a variety of amine and sulfide substituents.

Scheme C illustrates the incorporation of R¹ by a Suzuki coupling of an appropriately substituted boronic acid or boronic ester with the chloride of the fused pyridyl ring of the instant compounds.

Scheme D illustrates an alternative series of reactions to the instant compounds having substituted amine substituents on the phenyl ring.

Preparation of the instant compounds wherein R⁵ is a functionalized methyl is illustrated in Scheme E. Thus the ester E-1 is reduced to provide the diol E-2, which is selectively protected and then oxidized to provide the instant compound E-4. Deprotection provides the alcohol E-5 which may then be converted to a variety of other functional groups by techniques well known in the art.

Scheme F illustrates an alternative procedure for forming the tricyclic ring system of the instant compounds. Thus a suitably substituted nicotinoyl chloride F-1 is converted to intermediate F-2, which reacts with a suitably substituted boronic acid to provide the benzaldehyde F-3. Intermediate F-3 can then undergo base mediated cyclization to provide the instant compound F-4.

Preparation of a hydroxyl bearing alkyl sidechain for substituent R⁵ is illustrated in Scheme G starting with the vinyl substituent.

Scheme H illustrates the preparation of suitably substituted amide moieties for substituent R¹.

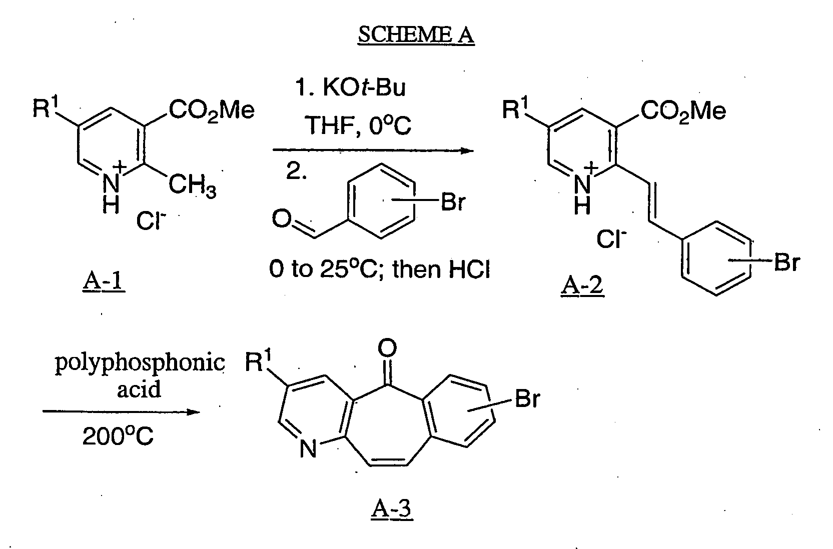

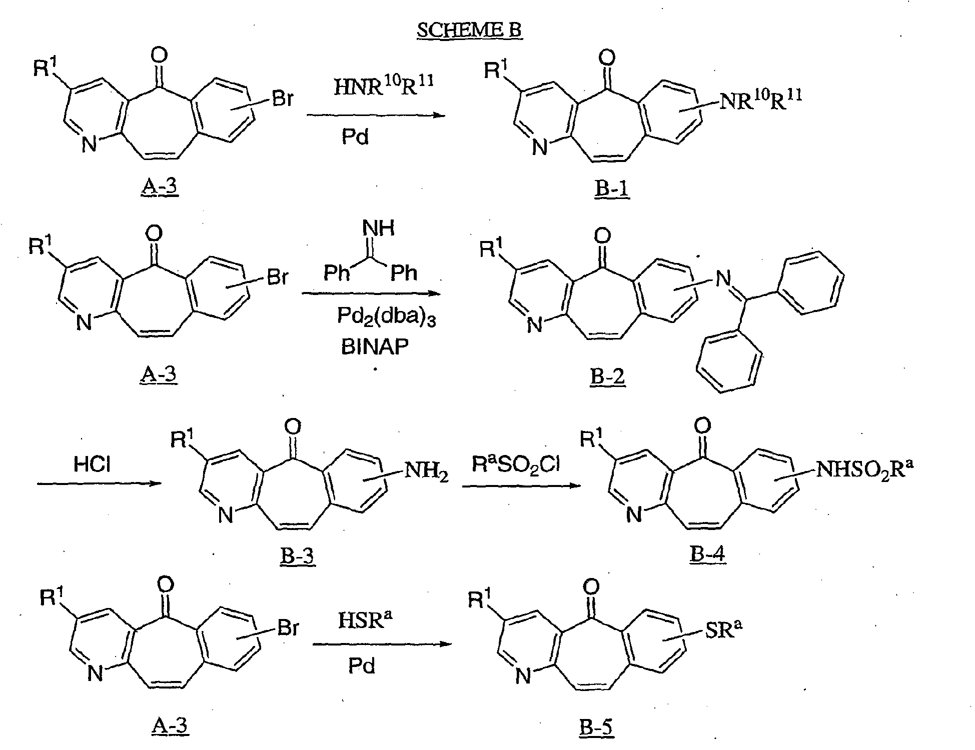

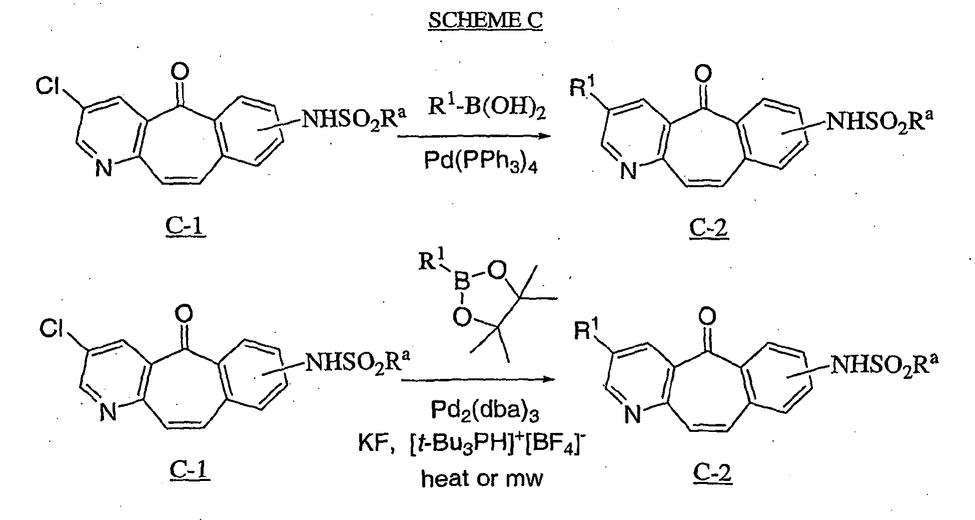

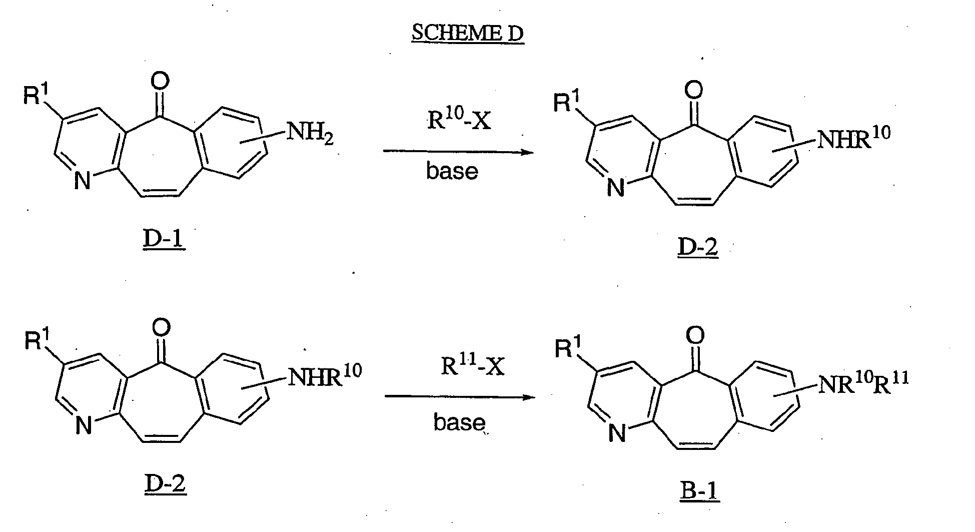

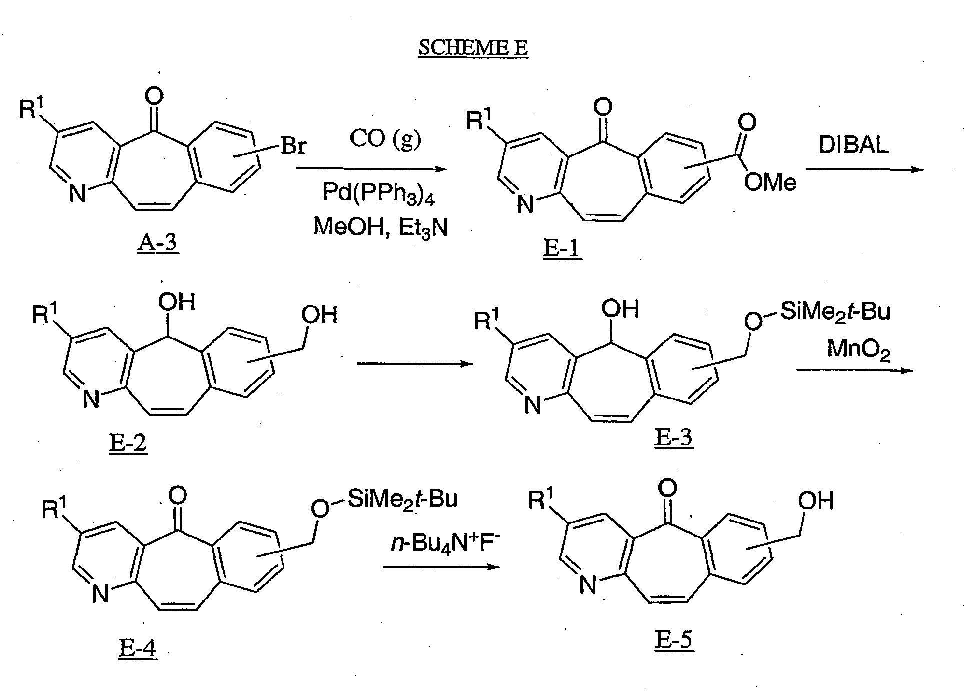

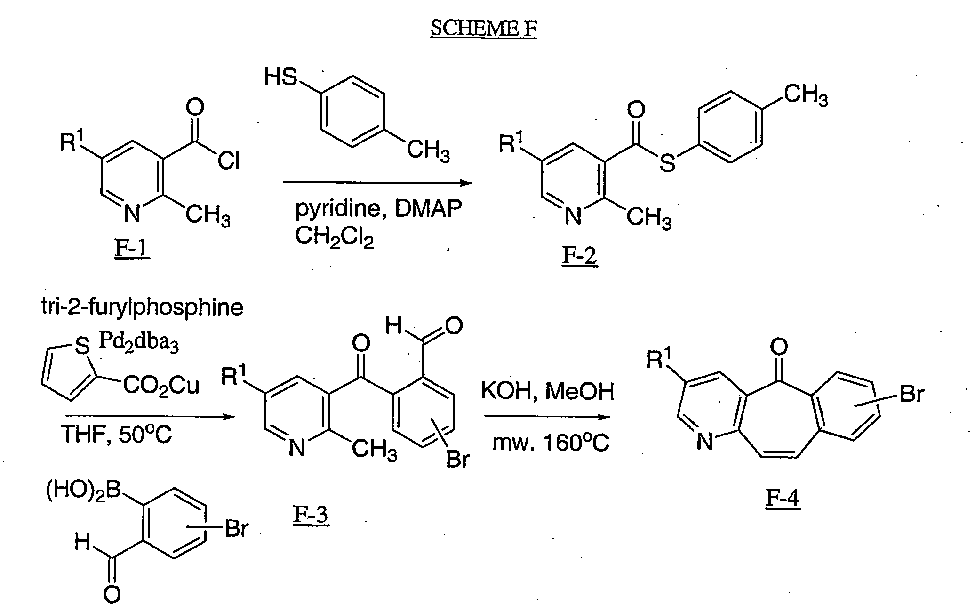

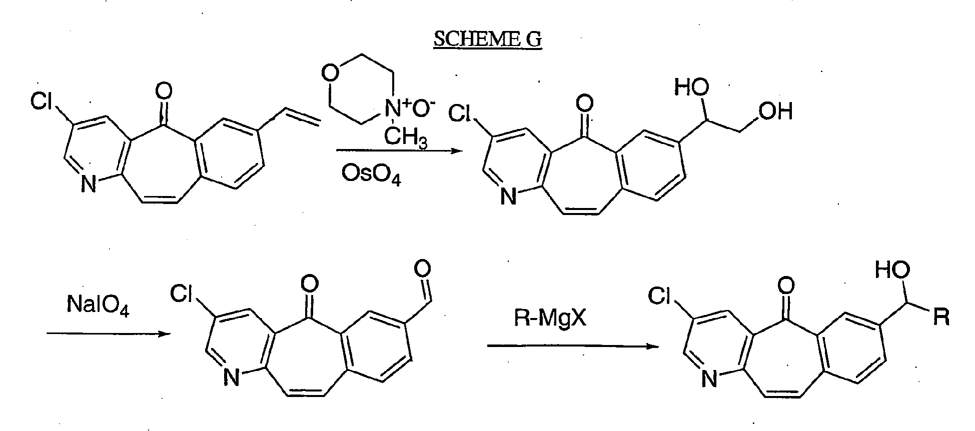

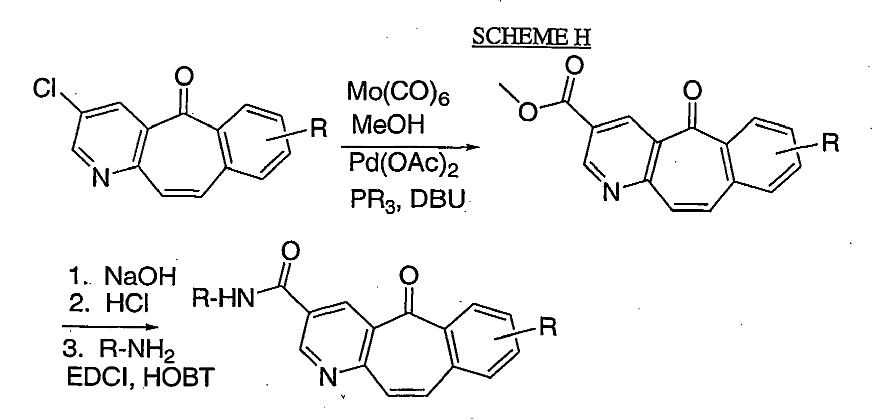

### Utilities

The compounds of the invention are useful to bind to and/or modulate the activity of a tyrosine kinase, in particular, a receptor tyrosine kinase. In an embodiment, the receptor tyrosine kinase is a member of the MET subfamily. In a further embodiment, the MET is human MET, although the activity of receptor tyrosine kinases from other organisms may also be modulated by the compounds of the present invention. In this context, modulate means either increasing or decreasing kinase activity of MET. In an embodiment, the compounds of the instant invention inhibit the kinase activity of MET.

The compounds of the invention find use in a variety of applications. As will be appreciated by those skilled in the art, the kinase activity of MET may be modulated in a variety of ways; that is, one can affect the phosphorylation/activation of MET either by modulating the initial phosphorylation of the protein or by modulating the autophosphorylation of the other active sites of the protein. Alternatively, the kinase activity of MET may be modulated by affecting the binding of a substrate of MET phosphorylation.

The compounds of the invention are used to treat or prevent cellular proliferation diseases. Disease states which can be treated by the methods and compositions provided herein include, but are not limited to, cancer (further discussed below), autoimmune disease, arthritis, graft rejection, inflammatory bowel disease, proliferation induced after medical procedures, including, but not limited to, surgery, angioplasty, and the like. It is appreciated that in some cases the cells may not be in a hyper- or hypoproliferation state (abnormal state) and still require treatment. Thus, in one embodiment, the invention herein includes application to cells or individuals which are afflicted or may eventually become afflicted with any one of these disorders or states.

The compounds, compositions and methods provided herein are particularly deemed useful for the treatment and prevention of cancer including solid tumors such as skin, breast, brain, cervical carcinomas, testicular carcinomas, etc. In an embodiment, the instant compounds are useful for treating cancer. In particular, cancers that may be treated by the compounds, compositions and methods of the invention include, but are not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, non-small cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma), rectal, colorectal and colon; Genitourinary tract: kidney (adenocarcinoma, Wilm's tumor [nephroblastoma], lymphoma, leukemia, papillary renal carcinoma), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, head and neck squamous cell carcinomas, Karposi's sarcoma, moles dysplastic nevi, lipoma, angioma, dermatofibroma, keloids, psoriasis; and Adrenal glands: neuroblastoma. Thus, the term "cancerous cell" as provided herein, includes a cell afflicted by any one of the above-identified conditions. In another embodiment, the compounds of the instant invention are useful for treating or preventing cancer selected from: head and neck squamous cell carcinoma, histiocytic lymphoma, lung adenocarcinoma, small cell lung cancer, non-small cell lung cancer, pancreatic cancer, papillary renal carcinoma, liver cancer, gastric cancer, colon cancer, multiple myeloma, glioblastoma and breast carcinoma. In still another embodiment, the compounds of the instant invention are useful for treating cancer selected from: histiocytic lymphoma, lung adenocarcinoma, small cell lung cancer, pancreatic cancer, liver cancer, gastric cancer, colon cancer, multiple myeloma, glioblastoma and breast carcinoma. , In yet another embodiment, the compounds of the instant invention are useful for treating cancer selected from: ovarian cancer, childhood hepatocellular carcinoma, metastatic head and neck squamous cell carcinomas, gastric cancer, breast cancer, colorectal cancer, cervical cancer, lung cancer, nasopharyngeal cancer, pancreatic cancer, glioblastoma and sarcoma

In another embodiment, the compounds of the instant invention are useful for the prevention or modulation of the metastases of cancer cells and cancer. In particular, the compounds of the instant invention are useful to prevent or modulate the metastases of ovarian cancer, childhood hepatocellular carcinoma, metastatic head and neck squamous cell carcinomas, gastric cancers, breast cancer, colorectal cancer, cervical cancer, lung cancer, nasopharyngeal cancer, pancreatic cancer, glioblastoma and sarcomas.

The compounds of this invention may be administered to mammals, such as humans, either alone or in combination with pharmaceutically acceptable carriers, excipients or diluents, in a pharmaceutical composition, according to standard pharmaceutical practice. The compounds can be administered orally or parenterally, including the intravenous, intramuscular, intraperitoneal, subcutaneous, rectal and topical routes of administration..

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate; sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulation.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butane diol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of Formula I may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of Formula I are employed. (For purposes of this application, topical application shall include mouth washes and gargles.)

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

The dosage regimen utilizing the compounds of the instant invention can be selected in accordance with a variety of factors including type, species, age, weight, sex and the type of cancer being treated; the severity (i.e., stage) of the cancer to be treated; the route of administration; the renal and hepatic function of the patient; and the particular compound or salt thereof employed. An ordinarily skilled physician or veterinarian can readily determine and prescribe the effective amount of the drug required to treat, for example, to prevent inhibit (fully or partially) or arrest the progress of the disease.

In one exemplary application, a suitable amount of compound is administered to a mammal undergoing treatment for cancer. Administration occurs in an amount between about 0.1 mg/kg of body weight to about 60 mg/kg of body weight per day, preferably of between 0.5 mg/kg of body weight to about 40 mg/kg of body weight per day.

For example, compounds of the instant invention can be administered in a total daily dose of up to 1000 mg. Compounds of the instant invention can be administered once daily (QD), or divided into multiple daily doses such as twice daily (BID), and three times daily (TID). Compounds of the instant invention can be administered at a total daily dosage of up to 1000 mg, e.g., 200 mg, 300 mg, 400 mg, 600 mg, 800 mg or 1000 mg, which can be administered in one daily dose or can be divided into multiple daily doses as described above.

In addition, the administration can be continuous, i.e., every day, or intermittently. The terms "intermittent" or ''intermittently" as used herein means stopping and starting at either regular or irregular intervals. For example, intermittent administration of a compound of the instant invention may be administration one to six days per week or it may mean administration in cycles (e.g. daily administration for two to eight consecutive weeks, then a rest period with no administration for up to one week) or it may mean administration on alternate days.

In addition, the compounds of the instant invention may be administered according to any of the schedules described above, consecutively for a few weeks, followed by a rest period. For example, the compounds of the instant invention may be administered according to any one of the schedules described above from two to eight weeks, followed by a rest period of one week, or twice daily at a dose of 100 - 500 mg for three to five days a week. In another particular embodiment, the compounds of the instant invention may be administered three times daily for two consecutive weeks, followed by one week of rest.

The instant compounds are also useful in combination with known therapeutic agents and anti-cancer agents. For example, instant compounds are useful in combination with known anti-cancer agents. Examples of such agents can be found in Cancer Principle and Practice of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Such anti-cancer agents include, but are not limited to, the following: estrogen receptor modulators, androgen receptor modulators, retinoid receptor modulators, cytotoxic/cytostatic agents, antiproliferative agents, prenyl-protein transferase inhibitors, HMG-CoA reductase inhibitors and other angiogenesis inhibitors, inhibitors of cell proliferation and survival signaling, apoptosis inducing agents and agents that interfere with cell cycle checkpoints. The instant compounds are particularly useful when co-administered with radiation therapy.

"Cytotoxic/cytostatic agents" refer to compounds which cause cell death or inhibit cell proliferation primarily by interfering directly with the cell's functioning or inhibit or interfere with cell mytosis, including alkylating agents, tumor necrosis factors, intercalators, hypoxia activatable compounds, microtubule inhibitors/microtubule-stabilizing agents, inhibitors of mitotic kinesins, =inhibitors of kinases involved in mitotic progression, antimetabolites; biological response modifiers; hormonal/anti-hormonal therapeutic agents, haematopoietic growth factors, monoclonal antibody targeted therapeutic agents, topoisomerase inhibitors, proteasome inhibitors and ubiquitin ligase inhibitors.

Examples of cytotoxic agents include, but are not limited to, sertenef, cachectin, ifosfamide, tasonermin, lonidamine, carboplatin, altretamine, prednimustine, dibromodulcitol, ranimustine, fotemustine, nedaplatin, oxaliplatin, temozolomide, heptaplatin, estramustine, improsulfan tosilate, trofosfamide, nimustine, dibrospidium chloride, pumitepa, lobaplatin, satraplatin, profiromycin, cisplatin, irofulven, dexifosfamide, cis-aminedichloro(2-methyl-pyridine)platinum, benzylguanine, glufosfamide, GPX100, (trans, trans, trans)-bis-mu-(hexane-1,6-diamine)-mu-[diamine-platinum(II)]bis[diamine(chloro)platinum (II)]tetrachloride, diarizidinylspermine, arsenic trioxide, 1-(11-dodecylamino-10-hydroxyundecyl)-3,7-dimethylxanthine, zorubicin, idarubicin, daunorubicin, bisantrene, mitoxantrone, pirarubicin, pinafide, valrubicin, amrubicin, antineoplaston, 3'-deamino-3'-morpholino-13-deoxo-10-hydroxycarminomycin, annamycin, galarubicin, elinafide, MEN10755, and 4-demethoxy-3-deamino-3-aziridinyl-4-methylsulphonyl-daunorubicin (see WO 00/50032).

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

The term "treating cancer" or "treatment of cancer" refers to administration to a mammal afflicted with a cancerous condition and refers to an effect that alleviates the cancerous condition by killing the cancerous cells, but also to an effect that results in the inhibition of growth and/or metastasis of the cancer.

In an embodiment, the angiogenesis inhibitor to be used as the second compound is selected from a tyrosine kinase inhibitor, an inhibitor of epidermal-derived growth factor, an inhibitor of fibroblast-derived growth factor, an inhibitor of platelet derived growth factor, an MMP (matrix metalloprotease) inhibitor, an integrin blocker, interferon-α, interleukin-12, pentosan polysulfate, a cyclooxygenase inhibitor, carboxyamidotriazole, combretastatin A-4, squalamine, 6-O-chloroacetyl-carbonyl)-fumagillol, thalidomide, angiostatin, troponin-1, or an antibody to VEGF. In an embodiment, the estrogen receptor modulator is tamoxifen or raloxifene.

Also included in the scope of the claims is a method of treating cancer that comprises administering a therapeutically effective amount of a compound of Formula I in combination with radiation therapy and/or in combination with a compound selected from: an estrogen receptor modulator, an androgen receptor modulator, a retinoid receptor modulator, a cytotoxic/cytostatic agent, an antiproliferative agent, a prenyl-protein transferase inhibitor, an HMG-CoA reductase inhibitor, an HIV protease inhibitor, a reverse transcriptase inhibitor, an angiogenesis inhibitor, PPAR-γ agonists, PPAR-δ agonists, an inhibitor of inherent multidrug resistance, an anti-emetic agent, an agent useful in the treatment of anemia, an agent useful in the treatment of neutropenia, an immunologic-enhancing drug, an inhibitor of cell proliferation and survival signaling, an agent that interfers with a cell cycle checkpoint, and an apoptosis inducing agent.

And another embodiment of the invention is a method of treating cancer that comprises administering a therapeutically effective amount of a compound of Formula I in combination with one or more of the following therapeutic agents: abarelix (Plenaxis depot®); aldesleukin (Prokine®); Aldesleukin (Proleukin®); Alemtuzumabb (Campath®); alitretinoin (Panretin®); allopurinol (Zyloprim®); altretamine (Hexalen®); amifostine (Ethyol®); anastrozole (Arimidex®); arsenic trioxide (Trisenox®); asparaginase (Elspar®); azacitidine (Vidaza®); bevacuzimab (Avastin®); bexarotene capsules (Targretin®); bexarotene gel (Targretin®); bleomycin (Blenoxane®); bortezomib (Velcade®); busulfan intravenous (Busulfex®); busulfan oral (Myleran®); calusterone (Methosarb®); capecitabine (Xeloda®); carboplatin (Paraplatin®); carmustine (BCNU®, BiCNU®); carmustine (Gliadel®); carmustine with Polifeprosan 20 Implant (Gliadel Wafer®; celecoxib (Celebrex®); cetuximab (Erbitux®); chlorambucil (Leukeran®); cisplatin (Platinol®); cladribine (Leustatin®, 2-CdA®); clofarabine (Clolar®); cyclophosphamide (Cytoxan®, Neosar®); cyclophosphamide (Cytoxan Injection®); cyclophosphamide (Cytoxan Tablet®); cytarabine (Cytosar-U®); cytarabine liposomal (DepoCyt®); dacarbazine (DTIC-Dome®); dactinomycin, actinomycin D (Cosmegen®); Darbepoetin alfa (Aranesp®); daunorubicin liposomal (DanuoXome®); daunorubicin, daunomycin (Daunorubicin®; daunorubicin, daunomycin (Cerubidine®); Denileukin diftitox (Ontak®); dexrazoxane (Zinecard®); docetaxel (Taxotere®); doxorubicin (Adriamycin PFS®); doxorubicin (Adriamycin®, Rubex®); doxorubicin (Adriamycin PFS Injection®); doxorubicin liposomal (Doxil®); DROMOSTANOLONE PROPIONATE (DROMOSTANOLONE®); DROMOSTANOLONE PROPIONATE (MASTERONE INJECTION®); Elliott's B Solution (Elliott's B Solution®); epirubicin (Ellence®); Epoetin alfa (epogen®); erlotinib (Tarceva®); estramustine (Emcyt®); etoposide phosphate (Etopophos®); etoposide, VP-16 (Vepesid®); exemestane (Aromasin®); Filgrastim (Neupogen®); floxuridine (intraarterial) (FUDR®); fludarabine (Fludara®); fluorouracil, 5-FU (Adrucil®); fulvestrant (Faslodex®); gefitinib (Iressa®); gemcitabine (Gemzar®); gemtuzumab ozogamicin (Mylotarg®); goserelin acetate (Zoladex Implant®); goserelin acetate (Zoladex®); histrelin acetate (Histrelin implant®); hydroxyurea (hydra®); Ibritumomab Tiuxetan (Zevalin®); idarubicin (Idamycin®); ifosfamide (IFEX®); imatinib mesylate (Gleevec®); interferon alfa 2a (Roferon A®; Interferon alfa-2b (Intron A®); irinotecan (Camptosar®); lenalidomide (Revlimid®); letrozole (Femara®); leucovorin (Wellcovorin®, Leucovorin®); Leuprolide Acetate (Eligard®); levamisole (Ergamisol®); lomustine, CCNU (CeeBU®); meclorethamine, nitrogen mustard (Mustargen®); megestrol acetate (Megace®); melphalan, L-PAM (Alkeran®); mercaptopurine, 6-MP (Purinethol®); mesna (Mesnex®); mesna (Mesnex tabs®); methotrexate (Methotrexate®); methoxsalen (Uvadex®); mitomycin C (Mutamycin®); mitotane (Lysodren®); mitoxantrone (Novantrone®); nandrolone phenpropionate (Durabolin-50®); nelarabine (Arranon®); Nofetumomab (Verluma®); Oprelvekin (Neumega®); oxaliplatin (Eloxatin®); paclitaxel (Paxene®); paclitaxel (Taxol®); paclitaxel protein-bound particles (Abraxane®); palifermin (Kepivance®); pamidronate (Aredia®); pegademase (Adagen (Pegademase Bovine®); pegaspargase (Oncaspar®); Pegfilgrastim (Neulasta®); pemetrexed disodium (Alimta®); pentostatin (Nipent®); pipobroman (Vercyte®); plicamycin, mithramycin (Mithracin®); porfimer sodium (Photofrin®); procarbazine (Matulane®); quinacrine (Atabrine®); Rasburicase (Elitek®); Rituximab (Rituxan®); sargramostim (Leukine®); Sargramostim (Prokine®); sorafenib (Nexavar®); streptozocin (Zanosar®); sunitinib maleate (Sutent®); talc (Sclerosol®); tamoxifen (Nolvadex®); temozolomide (Temodar®); teniposide, VM-26 (Vumon®); testolactone (Teslac®); thioguanine, 6-TG (Thioguanine®); thiotepa (Thioplex®); topotecan (Hycamtin®); toremifene (Fareston®); Tositumomab (Bexxar®); Tositumomab/I-131 tositumomab (Bexxar®); Trastuzumab (Herceptin®); tretinoin, ATRA (Vesanoid®); Uracil Mustard (Uracil Mustard Capsules®); valrubicin (Valstar®); vinblastine (Velban®); vincristine (Oncovin®); vinorelbine (Navelbine®);and zoledronate (Zometa®)..

And yet another embodiment of the disclosure is a method of treating cancer that comprises administering a therapeutically effective amount of a compound of Formula I in combination with paclitaxel or trastuzumab.

The instant invention also includes a pharmaceutical composition useful for treating or preventing cancer that comprises a therapeutically effective amount of a compound of Formula I and a compound selected from: an estrogen receptor modulator, an androgen receptor modulator, a retinoid receptor modulator, a cytotoxic/cytostatic agent, an antiproliferative agent, a prenyl-protein transferase inhibitor, an HMG-CoA reductase inhibitor, an HIV protease inhibitor, a reverse transcriptase inhibitor, an angiogenesis inhibitor, a PPAR-γ agonist, a PPAR-δ agonists; an inhibitor of cell proliferation and survival signaling, an agent that interfers with a cell cycle checkpoint, and an apoptosis inducing agent.

Any one or more of the specific dosages and dosage schedules of the compounds of the instant invention, may also be applicable to any one or more of the therapeutic agents to be used in the combination treatment (hereinafter refered to as the "second therapeutic agent").

Moreover, the specific dosage and dosage schedule of this second therapeutic agent can further vary, and the optimal dose, dosing schedule and route of administration will be determined based upon the specific second therapeutic agent that is being used.

Of course, the route of administration of the compounds of the instant invention is independent of the route of administration of the second therapeutic agent. In an embodiment, the administration for a compound of the instant invention is oral administration. In another embodiment, the administration for a compound of the instant invention is intravenous administration. Thus, in accordance with these embodiments, a compound of the instant invention is administered orally or intravenously, and the second therapeutic agent can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form.

In addition, a compound of the instant invention and second therapeutic agent may be administered by the same mode of administration, i.e. both agents administered e.g. orally, by IV. However, it is also within the scope of the present invention to administer a compound of the instant invention by one mode of administration, e.g. oral, and to administer the second therapeutic agent by another mode of administration, e.g. IV or any other ones of the administration modes described hereinabove.

These and other aspects of the invention will be apparent from the teachings contained herein.

### ASSAYS

The compounds of the instant invention described in the Examples were tested by the assays described below and were found to have MET inhibitory activity. Other assays are known in the literature and could be readily performed by those of skill in the art (see, for example, U.S. Patent Application Publications US 2005/0075340 A1, April 7, 2005, pages 18-19; and PCT Publication WO 2005/028475, March 31, 2005, pages 236-248).

### I. In vitro kinase assays

Recombinant GST-tagged cytosolic domains of human c-Met and other receptor tyrosine kinases including mouse c-Met, human Ron, KDR, IGFR, EGFR, FGFR, Mer, TrkA and Tie2 are used to determine whether the compounds of the instant invention modulate the enzymatic activities of these kinases.

Soluble recombinant GST-tagged cytosolic domains of c-Met and other receptor tyrosine kinases are expressed in a baculovirus system (Pharmingen) according to a protocol recommended by the manufacturer. The c-DNA encoding each cytosolic domain is subcloned into a baculovirus expression vector (pGcGHT-A, B or C, Pharmingen) containing an in frame 6x histidine tag and a GST tag. The resulting plasmid construct and BaculoGold baculovirus DNA (Pharmingen) are used to co-transfect Sf9 or Sf21 insect cells. After confirming expression of GST-tagged kinase fusion, a high titer recombinant baculovirus stock is produced, expression conditions are optimized, and a scaled up expression of rat KDR-GST fusion is performed. The fusion kinase is then purified from the insect cell lysate by affinity chromatography using glutathione agarose (Pharmingen). The purified protein is dialyzed against 50% glycerol, 2 mM DTT, 50 mM Tris-HCl (pH 7.4) and stored at -20°C. The protein concentrations of the fusion proteins are determined using Coomassie Plus Protein Assay (Pierce) with BSA as standard.

The kinase activities of c-Met and other kinases are measured using a modified version of the homogeneous time-resolved tyrosine kinase assay described by Park et al. (1999, Anal. Biochem. 269:94-104).

The procedure for determining the potency of a compound to inhibit c-Met kinase comprises the following steps:
1. Prepare 3-fold serial diluted compound solutions in 100% dimethyl sulfoxide (DMSO) at 20X of the desired final concentrations in a 96 well plate.
2. Prepare a master reaction mix containing 6.67 mM MgCl₂, 133.3 mM NaCl, 66.7 mM Tris-HCl (pH 7.4), 0.13 mg/ml BSA, 2.67 mM dithiothreitol, 0.27 nM recombinant c-Met and 666.7 nM biotinylated synthetic peptide substrate (biotin-ahx-EQEDEPEGDYFEWLE-CONH₂) (SEQ.ID.NO.:1).
3. In a black assay plate, add 2.5 µl of compound solution (or DMSO) and 37.5 µl of master reaction mix per well. Initiate the kinase reaction by adding 10 µl of 0.25. mM MgATP per well. Allow the reactions to proceed for 80 min at room temperature. The final conditions for the reaction are 0.2 nM c-Met, 0.5 µM substrate, 50 µM MgATP, 5 mM MgCl₂, 100 mM NaCl, 2 mM DTT, 0.1 mg/ml BSA, 50 mM Tris (pH 7.4) and 5% DMSO.
4. Stop the kinase reaction with 50 µl of Stop/Detection buffer containing 10 mM EDTA, 25 mM HEPES, 0.1 % TRITON X-100, 0.126 µg/ml Eu-chelate labeled anti-phosphotyrosime antibody PY20 (cat. # AD0067, PerkinElmer) and 45 µg/ml Streptavidin-allophycocyanin conjugate (cat. # PJ25S, Prozyme).
5. Read HTRF signals on a Victor reader (PerkinElmer) in HTRF mode after 60 min.
6. IC₅₀ is determined by fitting the observed relationship between compound concentration and HTRF signal with a 4-parameter logistic equation.
Essentially the same procedure was used to determine the potency of compounds to inhibit mouse c-Met, human Ron, KDR, IGFR, EGFR, FGFR, Mer, TrkA and Tie2 except that the concentration of enzyme varied in individual assays (0.2 nM mouse c-Met; 2.5 nM Ron, 8 nM KDR; 0.24 nM IGFR; 0.24 nM EGFR; 0.14 nM FGFR;16 nM Mer; 8 nM TrkA; 8 nM Tie2).

The compounds 3 to 54 in the Examples were tested in the above assay and found to have an IC₅₀ ≤ 50µM.

### II. Cell based-c-Met autophosphorylation assay

A sandwich ELISA assay is used to assess MET autophosphorylation in MKN45 gastric cancer cells, in which MET is constitutively activated. Briefly a monolayer of cells was pre-treated with compounds or the vehicle and then lysed. The MET in a cell lysate was captured by an anti-MET antibody immobilized on a plastic surface. A generic anti-phosphotyrosine antibody or one of several specific anti-phospho-MET antibodies is then allowed to bind captured MET and is detected using HRP-conjugated secondary antibody.
The procedure for determining the potency of a compound to inhibit MET autophosphorylation in MKN45 cells comprises the following steps:
Day 1
   1. Coat a 96-well ELISA plate overnight at 4°C with 100 µl/well of 1 µg/ml capture antibody solution (Af276, R&D).
   2. Seed a separate 96-well culture plate with MKN45 cells at 90,000 cells/well in 0.1 ml of growth media (RPMI 1640, 10% FBS, 100 µg/mL Pen-Strep, 100ug/mL L-glutamine, and 10mM HEPES) and culture overnight at 37°C/5% CO₂ to 80-90% confluence.
Day 2
   1. Wash the ELISA plate 4 X with 200 µl/well of wash buffer (TBST+,0.25% BSA). Incubate the ELISA plate with 200 µl/well of blocking buffer (TBST + 1.5% BSA) for 3-5 hrs at RT.
   2. Prepare a half-long dilution series of of 200X compound in DMSO. Dilute the series to10X with assay media (RPMI 1640,10% FBS, and 10mM HEPES).
   3. Add 10X compound solutions (11 µl/well) to the culture plate containing MKN45 cells. Incubate the plate at 37°C/5% CO₂ for 60 min.
   4. Lyse the cells with 100 µl/well of lysis buffer (30 mM Tris, pH 7.5, 5 mM EDTA, 50 mM NaCl, 30 mM sodium pyrophosphate, 50 mM NaF, 0.5 mM Na₃VO₄, 0.25 mM potassium bisperoxo(1,10-phenanthroline)-oxovanadate, 0.5% NP40, 1 % Triton X-100, 10% glycerol, and a protease inhibitor cocktail) at 4°C for 90 min.
   5. Remove blocking buffer from the ELISA plate, wash the plate 4X with 200 µl/well of wash buffer. Transfer 90 µl/well of MKN45 cell lysate from the culture plate to the ELISA plate. Incubate sealed assay plate at 4°C with gentle shaking overnight.
Day 3
   1. Wash the ELISA plates 4 times with 200 µl/well wash buffer.
   2. Incubate with 100 µl/well primary detection antibody (1 µg/ml in TBST + 1% BSA) for 1.5 hours at ambient temperature. The following primary antibodies have been used: 4G10 from UpState, anti-pMet(1349) and anti-pMet(1369), both from Biosource.
   3. Wash the ELISA plates 4 times with wash buffer. Add 100 µl/well of secondary antibody (1:1000 anti-mouse IgG-HRP diluted in TBST + 1% BSA for 4G10, or 1:1000 anti-rabbit IgG-HRP for anti-pMet(1349) and anti-pMet(1365)). Incubate at room temperature with gentle mixing for 1.5 hours. Wash 4 X with 200 µl/well wash buffer.
   4. Add 100 µl/well of Quanta Blu reagent (Pierce) and incubate at room temperature for 8 minutes. Read fluorescence (Excitation wavelength: 314 nm, emission wavelength: 425 nm) on a Spectramax Gemini EM plate reader (Molecular Devices).
   5. IC₅₀ is calculated by fitting the relationship between compound concentration and fluorescence signal with a 4-parameter logistic equation.

### III. MKN45 cell proliferation/viability assay

MKN45 human gastric cancer cells are known to over-express constitutively activated c-met. siRNA-mediated partial knock down of c-Met was found to induce pronounced growth inhibition and apoptosis in MKN45 cells, suggesting a vital role of c-Met in this cell line. The assay described here measures the effect of c-Met inhibitors on proliferation/viability of MKN45 cells. The procedure for determining the potency of a compound to inhibit MKN45 proliferation/viability comprises the following steps.

On day 1, plate MKN45 cells at 3000 cells/95 µl medium (RPMI/10% FCS, 100 mM HEPES, penicillin and streptomycin) per well in a 96 well plate. Maintain the plate in an incubator at 37°C/5%CO₂. Prepare 3-fold serial diluted compound solutions at 1000X of desired final concentrations in DMSO.

On day 2, prepare 50X compound solutions by diluting the 1000X compound solutions with the medium. Add 5 µl 20X compound solution per well to the MKN45 cell culture described above. Return the plate to the incubator.

On day 5, add 50 µl lysis buffer (ViaLight Reagents Kit, Catalog No. LT07-221, Cambrex): per well. Lyse the cells at room temperature for 15 minutes. Then add 50 µl detection reagent (ViaLight Reagents Kit) and incubate for 3 minutes. The plate is read on a TOPCOUNT (PerkinElmer) in luminescence mode. IC₅₀ is calculated by fitting the relationship between compound concentration and luminescence signal with a 4-parameter logistic equation.

### IV. HGF-induced cell migration assay

The HGF-induced migration of HPAF pancreatic cancer cells was assessed using BD Falcon Fluoroblock 96-Multiwell Insert plates (Cat # 351164, BD Discovery Labware). The plate consists of wells each of which is partitioned by a micro-porous membrane into the top and bottom chambers. Pancreatic cancer cells are plated on the top side of the membrane and migrate to the underside of the membrane in response to chemo-attractant added to the lower chamber. The cells on the under side of the membrane are labeled with a fluorescent dye and detected by a fluorescence plate reader. The procedure for determining the potency of a compound to inhibit cell migration comprises the following steps.
1. Prepare test compound solutions of 1000X final concentrations in 100% DMSO
2. Dilute the above solutions 50X with DMEM/10% FCS to obtain compound solutions 20X of the final concentrations.
3. Fill each lower chamber of a Fluoroblock 96-Muntiwell Insert plate with 180 µl DMEM/10% FCS, and plate 8,000 HPAF pancreatic cancer cells in 50ul DMEM/10% FCS in each upper chamber.
4. 1-2 hours after plating, add 2.5 µl and 10 µl of a 20X compound solution to the upper and the lower chamber respectively. Incubate the plate at 37°C for 60 min, and then add concentrated HGF to lower chamber to a final HGF concentration of 15ng/ml. The insert plates are incubated overnight for 20 hours.
5. An aliquot of a concentrated Calcein dye (Molecular Probes) is added to each lower chamber to give 5 µg/ml final dye concentration and the cells are labeled for 1 hour. Wash each lower chamber with 200 µl DMEM/10% FCS
6. Read fluorescence on a Victor reader (PerkinElmer) in bottom read mode (Excitation wave length: 485 nm, emission wavelength: 535 nm).
7. IC₅₀ is calculated by fitting the relationship between compound concentration and fluorescence signal with a 4-parameter logistic equation.

### EXAMPLES

Examples provided are intended to assist in a further understanding of the invention. Particular materials employed, species and conditions are intended to be illustrative of the invention and not limiting of the reasonable scope thereof.

### Example 1

### Stop1: 2-[(E/Z)-2-(4-bromophenyl)vinyl]-3-carboxy-5-chloropyridinium chloride.

Potassium tert-butoxide (1M solution in THF, 60 mL, 60 mmol) was added to a solution of 4-bromobenzaldehyde (5.6 g, 30 mmol) and methyl 5-chloro-2-methylnicotinate (Marcoux, J.-F.; Marcotte, F.-A.; Wu, J.; Dormer, P.G.; Davies, I.W.; Hughes, D.; Reider, P.J. J. Org. Chem. 2001, 66, 4194-4199) (5.6 g, 30 mmol) in 200 mL THF at 0°C. The mixture was allowed to warm to ambient temperature and stirred for 12 hours. The reaction slurry was concentrated to give yellow/orange solids, then 50 mL of water and 50 mL of 6N HCl were added. After stirring the resulting slurry for 30 minutes, 200 mL of EtOH was added and the slurry was stirred for 4 hours. The slurry was filtered and dried to afford the title compound. ¹H NMR (600 MHz, DMSO-D₆) δ 8.76 (d, 1H); 8.22 (d, 1H); 8.02 (d, 1H); 7.79 (d, 1H); 7.60-7.54 (m, 4H). LRMS (APCI) calculated for C₁₄H₁₀BrClNO₂ [M+H]+, 338.0; found 337.9.

### Step 2: 7-bromo-3-chloro-5H-benzo[4,5]cyclohepta[1,2-b]pyridin-5-one (Compound 1).

2-[(*E*/*Z*)-2-(4-bromophenyl)vinyl]-3-carboxy-5-chloropyridinium chloride (11.2 g, 29.9 mmol) was added to 50 mL of polyphosphoric acid and heated to 200°C. After 12 hours, the solution was poured into ice and 250 mL of 5N sodium hydroxide solution, then 5N sodium hydroxide solution was added to adjust to pH 10. The mixture was diluted in 2 L of dichloromethane, 100 g of Celite were added and the suspension was stirred for 15 minutes. The solids were filtered through a sintered glass funnel and discarded. The liquid phase was poured into a separatory funnel and the organic layer was isolated. The organic layer was dried with magnesium sulfate, filtered, and concentrated to afford Compound 1. ¹H NMR (600 MHz, CDCl₃) δ 8.82 (d, 1H); 8.50 (d, 1H); 8.41 (d, 1H); 7.80 (dd, 1H); 7.48 (d, 1H); 7.35 (d, 1H); 7.20 (d, 1H). LRMS (APCI) calculated for C₁₄H₈BrCINO [M+H]+, 320.0; found 320.0.

### Example 2

### Step1: S-(4-methylphenyl) 2-methyl-5-phenylpyridine-3-carbothioate.

To a 0°C solution of 2-methyl-5-phenylnicotinic acid (100 mg, 0.40 mmol) in CH₂Cl₂ (4 mL) was added oxalyl chloride (344 µL, 4.0 mmol). The mixture was stirred at 40°C. After 3 hours, the mixture was concentrated to dryness, dissolved in benzene (2x5 mL) and concentrated again. After dissolving the crude residue in CH₂Cl₂ (2 mL) at 0 °C, pyridine (1mL, 0.92 M in CH₂Cl₂), 4-dimethylaminopyridine (10 mg, 0.08 mmol) and 4-methylthiophenol (60 mg, 0.48 mmol) were added. The mixture was then allowed to warm to room temperature. After stirring for 2 hours, the mixture was diluted with EtOAc , washed with 1N HCl, brine, dried over sodium sulfate, filtered, and concentrated. The crude residue was purified by flash chromatography (100-80% hexanes/EtOAc gradient) to afford the title compound. LRMS (APCI) calculated for C₂₀H₁₈NOS [M+H]+, 320.1; found 320.1

### Step 2: 2-[(2-methyl-5-phenylpyridin-3-yl)carbonyl]benzaldehyde.

S-(4-methylphenyl) 2-methyl-5-phenylpyridine-3-carbothioate (100 mg, 0.31 mmol), copper (I) thiophene-2-carboxylate (89.6 mg, 0.47 mmol), Pd₂dba₃CHCl₃ (26 mg, 0.025 mmol), tri-2-furylphosphine (17.3 mg, 0.074 mmol) and 2-formylphenylboronic acid (51.7 mg, 0.34 mmol) were combined in a dry flask. The flask was purged with argon and 3.0 mL of THF were added. Argon was bubbled through the solution for 5 minutes and the solution was stirred and heated to 50°C. After 18 hours, the reaction mixture was diluted with EtOAc, washed with 1N HCl, brine, dried over sodium sulfate, filtered, concentrated and purified by flash chromatography (100-70% hexanes/EtOAc gradient) to afford the title compound. LRMS (APCI) calculated for C₂₀H₁₆NO₂ [M+H]⁺, 302.1; found 302.1

### Step 3: 3-phenyl-5H-benzo[4,5]cyclohepta[1,2-b]pyridin-5-one.

A flask was charged with 2-[(2-methyl-5-phenylpyridin-3-yl)carbonyl]benzaldehyde (6.2 mg, 0.02 mmol) and MeOH (1mL). LiHMDS (25 µl, 1.0 M in THF) was added and the vessel was heated in the Biotage Initiator series microwave for 30 min. at 100 °C. The mixture was then diluted with EtOAc, washed with water and brine, then dried over Na₂SO₄. The solution was concentrated *in vacuo* and purified by reverse phase HPLC (0-100% CH₃CN/water with a 0.1% TFA modifier) to afford the title compound. ¹H NMR (600 MHz, CD₃OD) δ 9.15 (d, 1H), 8.76 (d, 1H), 8.25 (d, 1H), 7.77 (m, 4H), 7.66 (t, 1H), 7.54 (t, 2H), 7.45 (m, 2H0, 7.36 (d, 1H). LRMS (APCI) calculated for C₂₀H₁₄NO [M+H]+, 284.1; found 283.8

The following compounds were made utilizing the experimental procedures described above, but using the appropriately substituted 2-formylphenylboronic acids, which were prepared according to literature methods.

**Table 1**

| Compound | Structrure | Name | MS(M+1) |
|---|---|---|---|
| 2 | 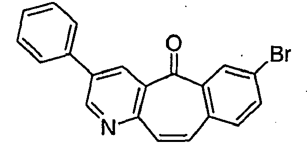 | 7-bromo-3-phenyl-5*H-*benzo[4,5]cyclohepta[1,2-*b*]pyridin-5-one | calc'd 362.0 (M+H)+; found 362.0 (M+H)+ |

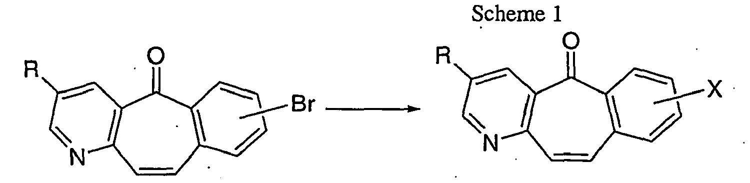

### Example 3

### 3-chloro-7-[(2,4-dimethoxybenzyl)aminol-5H-benzo[4,5]cyclohepta[1,2-b]pyridin-5-one.

7-bromo-3-chloro-5*H-*benzo[4,5]cyclohepta[1,2*-b*]pyridin-5-one (3.0 g, 9.40 mmol), tris(dibenzylideneacetone) dipalladium (0) (Pd₂(dba₃)) (43 mg, 0.047 mmol), *rac-*2,2'-bis(diphenylphosphino)-1,1'-binapthyl (BINAP) (88 mg, 0.141 mmol), and sodium *tert-*butoxide (1.08 g, 11.3 mmol) were combined in a dry flask through which argon was purged. The flask was charged with 100 mL of dry dioxane, 2,4-dimethoxybenzylamine (1.41 mL, 9.40 mmol) was added, and the mixture was sparged with argon for 5 minutes. The reaction was heated to 100°C and stirred under argon. After 2 h the reaction was concentrated and dissolved in 400 ml of ethyl acetate and washed with 100 mL of saturated aqueous ammonium chloride solution. The organic layer was separated, dried with magnesium sulfate, filtered, and concentrated. The resultant solids were slurried in 50 mL hot methanol, then allowed to cool to ambient temperature. The solids were filtered and dried to afford the title compound. LRMS (APCI) calculated for C₂₃H₂₀ClN₂O₃ [M+H]+, 407.1; found 407.1.

### Example 4

### 7-amino-3-chloro-5H-benzo[4.5]cyclohepta[1,2-b]pyridin-5-one.

### Method A:

7-bromo-3-chloro-5*H-*benzo[4,5]cyclohepta[1,2*-b*]pyridin-5-one (1.05 g, 3.30 mmol), Pd₂(dba)₃ (8 mg, 0.00825 mmol), BINAP (15 mg, 0.0248 mmol) and benzophenone imine (0.662 mL, 3.95 mmol) were combined in a dry flask. The flask was charged with 40 mL of dry toluene, followed by sodium *tert-*butoxide (0.444 g, 4.62 mmol). Argon was bubbled through the solution for 5 minutes. The reaction solution was heated to 110°C and stirred under argon. After 2.5 hours, the reaction was concentrated, 20 mL of THF and 1 mL of 6N hydrochloric acid were added and the resulting solution was stirred. After 2 hours, the solution was poured into 300 mL of ethyl acetate, 100 mL of saturated sodium bicarbonate and 200 mL of water. The organic layers were separated, dried with magnesium sulfate, filtered, concentrated, and purified by flash column chromatography (0-30% ethyl acetate/hexanes gradient) to afford the title compound. ¹H NMR (600 MHz, CDCl₃) δ 8.77 (d, 1H); 8.55 (d, 1H); 7.58 (d, 1H); 7.44 (d, 1H); 7.18 (d, 1H); 7.14 (d, 1H); 7.01 (dd, 1H); 4.15 (s, 2H). LRMS (APCI) calculated for C₁₄H₁₀ClN₂O [M+H]+, 257.0; found 257.1.

### Method B:

3-chloro-7-[(2,4-dimethoxybenzyl)amino]-5*H-*benzo[4,5]cyclohepta[1,2*-b*]pyridin-5-one (1.1 g, 2.7 mmol) was dissolved in 8 mL methanol and 30 mL dichloromethane. Then 10 mL of trifluoroacetic acid was added and the solution was stirred at ambient temperature. After 1 hour, reaction was concentrated, dissolved in 500 mL of ethyl acetate and washed with 200 mL saturated sodium bicarbonate. The organic layer was separated, dried with magnesium sulfate, filtered, and purified by flash column chromatography (0-10% methanol/dichloromethane gradient) and reverse phase HPLC (20-100% acetonitrile/water gradient, 0.1 % trifluoroacetic acid modifier) to afford title compound. LRMS (APCI) calculated for C₁₄H₁₀ClN₂O [M+H]+, 257.0; found 257.1.

### Example 5

### N-(3-chloro-5-oxo-5H-benzo[4.5]cyclohepta[1,2-b]pyridin-7-yl)methanesulfonamide.

### Method A:

7-bromo-3-chloro-5*H-*benzo[4,5]cyclohepta[1,2*-b*]pyridin-5-one (5.00 g. 15.7 mmol), methyl sulfonamide (1.49 g, 15.7 mmol), Pd₂(dba)₃ (0.714 g, 0.78 mmol), 9,9-dimethyl-4,5-bis(diphenylphosphino) xanthene (XANTPHOS) (1.36 g, 2.35 mmol) and cesium carbonate (15.3 g, 47.0 mmol) were added to a dry flask through which argon was purged. The flask was charged with 100 mL of dry dioxane and argon was bubbled through the solution for 10 minutes. The reaction mixture was heated to 95°C and stirred under argon. After 12 hours, the reaction mixture was concentrated and dissolved in 2000 mL ethyl acetate and 1000 mL water. The organic layer was separated and washed with 500 mL of brine, dried with magnesium sulfate, filtered, and concentrated. The resultant solids were dissolved in 150 mL of a 3:1 mixture of hot dichloromethane/methanol and allowed to cool to ambient temperature with stirring. After 3 h, 150 mL of hexanes were added and the resulting slurry was allowed to stir. After 12 hours, an additional 50 mL hexanes were added. After 4 hours, the solids were filtered and dried to afford the title compound.

### Method B:

7-amino-3-chloro-5*H-*benzo[4,5]cyclohepta[1,2*-b*]pyridin-5-one (0.70 g, 2.7 mmol), triethylamine (0.83 mL, 5.94 mmol) and methanesulfonylchloride (0.42 mL, 5.4 mmol) were added to 40 mL dichloromethane and cooled to 0°C. The solution was stirred and allowed to warm to ambient temperature. After 1 hour, the reaction was quenched with saturated sodium bicarbonate solution and stirred. After 30 minutes, the reaction mixture was poured into 300 mL ethyl acetate and 250 mL water. The organic layer was separated, dried with magnesium sulfate, filtered, and concentrated to afford crude *N*,*N*-(3-chloro-5-oxo-5*H-*benzo[4,5]cyclohepta[1,2*-b*]pyridin-7-yl)bis-methanesulfonamide.

The crude *N*,*N*-(3-chloro-5-oxo-5*H-*benzo[4,5]cyclohepta[1,2*-b*]pyridin-7-yl)bis-methanesulfonamide (1.1 g, 2.7 mmol) was dissolved in 150 mL methanol, then 5 mL of 5N sodium hydroxide solution was added and the solution was stirred at ambient temperature. After 1 hour, the reaction solution was partially concentrated and dissolved in 250 mLof ethyl acetate, 150 mL water, and 50 mL saturated aqueous ammonium chloride solution. The organic layer was separated, dried with magnesium sulfate, filtered, and concentrated to afford the title compound.
¹H NMR (600 MHz, CDCl₃) δ 8.82 (d, 1H); 8.54 (d, 1H); 7.98 (d, 1H); 7.70 (dd, 1H); 7.65 (d, 1H); 7.33 (d, 1H); 7.25 (d, 1H); 6.78 (s, 1H); 3.12 (s, 3H). LRMS (APCI) calculated for C₁₅H₁₂ClN₂O₃S [M+H]+, 335.0; found 335.1.

### Example 6

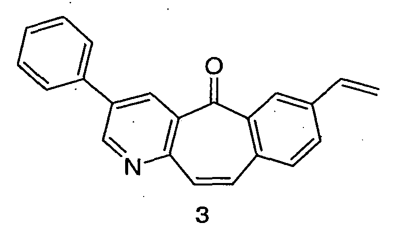

### 3-phenyl-7-vinyl-5H-benzo[4,5]cyclohepta[1,2-b]pyridin-5-one.

A test tube fitted with a Teflon lined septum was charged with compound **2** (100.0 mg, 0.276 mmol), PdCl₂(PPh₃)₂ (10 mg, 0.014 mmol), tri-n-butylvinyltin (0.089 mL, 0.30 mmol) and 3 mL of dioxane. The mixture was sparged with Ar for 10 min, then heated to 95°C overnight. The solution was diluted with EtOAc and washed with water and brine then dried over Na₂SO₄. The solution was concentrated *in vacuo* and purified by flash column chromatography (10-70% EtOAc/hexanes gradient) to afford a white solid. This sold was dissolved in 10 mL of 1:1:1 EtOAc/dichloromethane/water mixture and 82 mg of CsF was added. After 2h, the organic layer was separated and the aqueous layer was extracted with EtOAc and the organic layers dried over Na₂SO₄. The solution was concentrated *in vacuo* and purified by flash column chromatography (0-10-20-100% EtOAc/hexanes step gradient) to afford the title compound 3. ¹H NMR (600 MHz, CDCl₃) δ 9.14 (s, 1H); 8.78 (s, 1H); 8.30 (s, 1H); 7.75-7.78 (m, 1H); 7.70-7.74 (m, 2H); 7.59 (d, 1H); 7.50-7.55 (m, 2H); 7.40-7.47 (m, 2H); 7.30 (d, 1H); 6.85 (dd, 1H); 5.95 (d, 1H); 5.43 (d, 1H). LRMS (APCI) calc'd for (C₂₂H₁₆NO) [M+H]+, 310:1; found 310.2

### Example 7

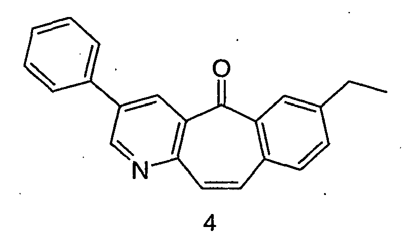

### 7-ethyl-3-phenyl-5H-benzo[4,5]cyclohepta[1,2-b]pyridin-5-one.

A flask was charged with compound 3 (20.0 mg, 0.065 mmol), 8 mg of 10% palladium on carbon, 3 mL of EtOH, 3 mL of EtOAc, and 0.5 mL of 1N HCl. The flask was fitted with a three-way stopcock with a hydrogen balloon, then evacuated and flushed with hydrogen four times. After 1 h the reaction mixture was filtered through a 0.45µ Nylon syringe filter, concentrated *in vacuo* and purified by reverse phase HPLC (20-100% CH₃CN/water with a 0.1 % TFA modifier) to afford the title compound **4.** ¹H NMR (600 MHz, CDCl₃) δ 9.13 (s, 1H); 8.75 (s, 1H); 8.13 (s, 1H); 7.70-7.73 (m, 2H); 7.49-7.55 (m, 4H); 7.42-7.46 (m, 1H); 7.37 (d, 1H); 7.28 (d, 1H); 2.81 (q, 2H); 1.31 (t, 3H). LRMS (APCI) calc'd for (C₂₂H₁₈NO) [M+H]+, 312.1; found 312.2

### Example 8

### Step 1: (3-chloro-5-oxo-5H-benzo[4,5]cyclohepta[1,2-b]pyridin-7-yl)boronic acid.

7-bromo-3-chloro-5*H-*benzo[4,5]cyclohepta[1,2*-b*]pyridin-5-one (1.00 g, 3.12 mmol), Pd₂(dba)₃ (0.146 g, 0.16 mmol), tricyclohexylphosphine (0.104 g, 0.37 mmol), bis(pinacolato)diboron (0.87 g, 3.43 mmol) and potassium acetate (0.61 g, 6.23 mmol) were mixed in a dry flask through which argon was purged. The flask was charged with 40 mL of dry dioxane and argon was bubbled through the solution for 15 minutes. The reaction was heated to 95°C and stirred under argon. After 6 h, the reaction mixture was poured into 500 mL of ethyl acetate and 100 mL of saturated aqueous ammonium chloride. The organic layer was separated, dried with magnesium sulfate, filtered, and concentrated to afford the title compound. LRMS (APCI) calculated for C₁₄H₁₀BClNO₃ [M+H]+, 286.0; found 286.1.

### Step 2: 3-chloro-7-hydroxy-5H-benzo[4,5]cyclohepta[1,2-b]pyridin-5-one

(3-chloro-5-oxo-5*H-*benzo[4,5]cyclohepta[1,2*-b*]pyridin-7-yl)boronic acid (1.00 g, 3.5 mmol) was dissolved in a 0°C solution of 25 mL of THF, 25 mL of water, 0.5 mL of acetic acid and 0.5 mL of 30% (w/w) hydrogen peroxide. The solution was stirred and allowed to warm to ambient temperature. After 6 h, the reaction mixture was partially concentrated and dissolved in 500 mL ethyl acetate. The organic layer was washed with water (2x100 mL), dried with magnesium sulfate, filtered, and concentrated to afford solids. The solids were dissolved in 20 mL dichloromethane and 60 mL hexanes and stirred as solids crystallized from the solution. After 2 hours, the crystalline solids were filtered and dried to afford the title compound. ¹H NMR (600 MHz, DMSO-D₆) δ 10.50 (s, 1H); 8.93 (s, 1H); 8.45 (s, 1H); 7.68 (d, 1H); 7.58 (d, 1H); 7.39 (d, 1H); 7.23 (d, 1H); 7.12 (d, 1H). LRMS (APCI) calculated for C₁₄H₉ClNO₂ [M+H]+, 258.0; found 258.1.

The following compounds were made according to Scheme 1. Additional synthetic modifications were employed in the preparation of some of the compounds.

**Table 2**

| Compound | Structure | Name | MS (M+1) |
|---|---|---|---|
| 5 | 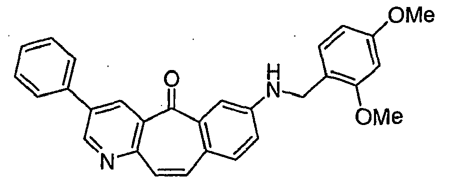 | 7-[(2,4-dimethoxybenzyl)amino]-3-phenyl-5H-benzo[4,5]cyclohepta[1,2-b]pyridin-5-one | calc'd 449.2 (M+H)+; found 449.2 (M+H)+ |
| 6 | 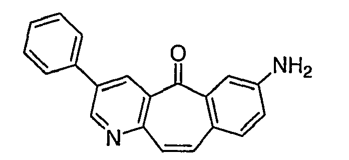 | 7-amino-3-phenyl-5*H-*benzo[4,5]cyclohepta[1,2-*b*]pyridin-5-one | calc'd 299.1 (M+H)+; found 299.1 (M+H)+ |
| 7 | 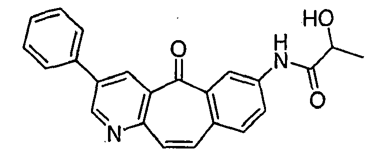 | 2-hydroxy-*N*-(5-oxo-3-phenyl-5*H-*benzo[4,5]cyclohepta[1,2-b]pyridin-7-yl)propanamide | calc'd 371.1(M+H)+; found 371.1 (M+H)+ |
| 8 | 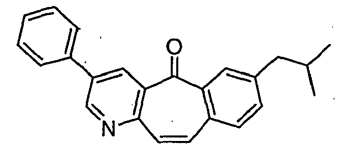 | 7-isobutyl-3-phenyl-5*H-*benzo[4,5]cyclohepta[1,2-*b*]pyridin-5-one (M+H)+ | calc'd 340.2 (M+H)+; found 340.2 |
| 9 | 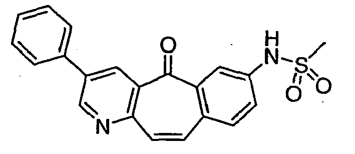 | *N*-(5-oxo-3-phenyl-5*H-*benzo[4,5]cyclohepta[1,2-*b*]pyridin-7-yl)methanesulfonamide | calc'd 377.1 (M+H)+; found 377.1 (M+H)+ |

### Example 9

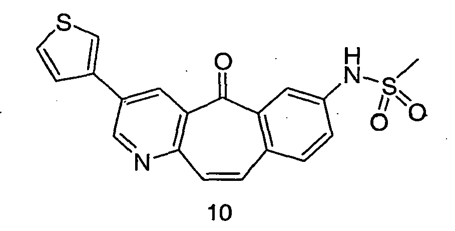

### N-[5-oxo-3-(3-thienyl)-5H-benzo[4,5]cyclohepta[1,2-b]pyridin-7-yl]methanesulfonamide.

*N*-(3-chloro-5-oxo-5*H-*benzo[4,5]cyclohepta[1,2*-b*]pyridin-7-yl)methanesultonamide (0.100 g, 0.30 mmol), 3-thienylboronic acid (0.077 g, 0.60 mmol), tetrakis(triphenylphosphine)palladium (0) (10mg, 0.009 mmol) and potassium carbonate (0.124 g, 0.90 mmol) were combined in a dry flask. The flask was purged with argon and 5 mL of dry dioxane was added. Argon was bubbled through the solution for 5 minutes and the solution was stirred and heated to 100°C. After 12 hours, the reaction mixture was poured into 100 mL of ethyl acetate, 100 mL of water, and 25 mL of saturated ammonium chloride. The organic layer was separated, dried with magnesium sulfate, filtered, concentrated, and purified by reverse phase HPLC (30-100% acetonitrile/water gradient, 0.1% trifluoroacetic acid modifier) to afford the title compound. ¹H NMR (600 MHz, DMSO-D₆) δ 10.40 (s, 1H); 9.34 (d, 1H);8.70 (d, 1H); 8.28 (s, 1H); 8.00 (d, 1H); 7.78 (m, 2H); 7.73 (m, 1H); 7.58 (dd, 1H); 7.38 (d, 1H); 7.26 (d; 1H); 3.08 (s, 3H). LRMS (APCI) calculated for C₁₉H₁₅N₂O₃S₂ [M+H]+, 383.0; found 383.1.

### Example 10

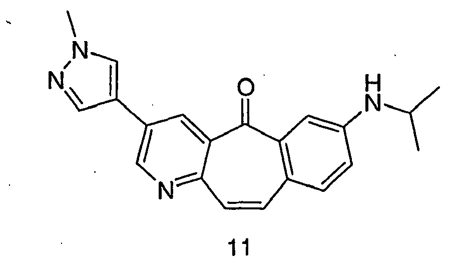

### 7-(isopropylamino)-3-(1-methyl-1H-pyrazol-4-yl)-5H-benzo[4,5]cyclohepta[1,2-b]pyridin-5-one.

3-chloro-7-(isopropylamino)-5*H-*benzo[4,5]cyclohepta[1,2-b]pyridin-5-one (0.030 g, .09 mmol), tris(dibenzylideneacetone)dipalladium (.004 g, 0.004 mmol), 1-methyl-4-(4,4,5,5-tetramethyl-1,3,2-dixaborolan-2-yl)-1*H-*pyrazole (.039 g, 0.19 mmol), potassium fluoride (0.018 g, 0.316 mmol) and tri-t-butylphosphonium tetrafluoroborate (0.003 g, 0.009 mmol) were combined in a microwave tube. The tube was purged with argon and 2 mL of dry DMF was added. The solution was stirred and heated to 180°C in the Biotage Initiator series microwave. After 30 minutes, saturated ammonium chloride was added and the mixture was extracted with ethyl acetate, washed with brine, dried over magnesium sulfate, filtered, concentrated *in vacuo,* and purified by reverse phase HPLC (20-70% acetonitrile/water gradient, 0.05% trifluoroacetic acid modifier) to afford the title compound. ¹H NMR (600 MHz, CDCl₃) δ 8.93 (d, 1H); 8.64 (bs, 1H); 7.86 (s, 1H); 7.75 (s, 1H); 7.45 (d, 1H); 7.39 (d, 1H); 7.14 (m, 2H); 6.85 (dd, 1H); 3.93 (s, 3H); 3.76 (septet, 1H); 1.22 (d, 6H). LRMS (APCI) calc'd for (C₂₁H₂₁N₄O) [M+H]+, 345.2; found 345.2.

### Example 11

### N,N-dimethyl-2-[4-(4,4,5,5-tetramethyl-1,3,2-dioxaborolan-2-yl)-1H-pyrazol-1-yl]ethanamine

4-(4,4,5,5-tetramethyl-1,3,2-dioxaborolan-2-yl)-1*H-*pyrazole (0.250 g, 1.29 mmol), dimethylamino ethyl chloride (0.37 g, 2.58 mmol) and potassium carbonate (0.534 g, 3.87 mmol) were dissolved in 3 mL of dry dimethylformamide. The reaction mixture was heated in a Biotage Initiator series microwave at 190°C for 1 hour. The reaction mixture was poured into 300 mL of ethyl acetate and 50 of mL brine. The organic layer was separated, dried with magnesium sulfate, filtered, and concentrated to afford the title compound. ¹H NMR (600 MHz, CDCl₃) δ 7.76 (s, 1H); 7.72 (s, 1H); 4.22 (t, 2H); 2.94 (s, 3H); 2.86 (s, 3H); 2.74 (t, 2H); 1.29 (s, 12 H).

### Example 12

### N'-(3-chloro-5-oxo-5H-benzo[4,5]cyclohepta[1,2-b]pyridin-7-yl)-N-[(2R)-1,4-dioxan-2-ylmethyll-N-methylsulfamide (Compound 12)

### Step 1: Benzyl (1,4-dioxan-2-ylmethyl)methylcarbamate

1-(1,4-dioxan-2-yl)-*N*-methylmethanamine hydrochloride (4.83 g, 29 mmol) was dissolved in 100 mL dichloromethane. Benzyl chloridocarbonate (4.9 mL, 35 mmol) and triethylamine (10 mL, 72 mmol) were added. The solution was stirred at ambient temperature. After 12 hours, the solution was concentrated, then diluted with ethyl acetate, and washed with saturated sodium bicarbonate and water. The organic layer was separated, dried with magnesium sulfate, filtered, concentrated *in vacuo,* and purified by silica chromatography (0-100% ethyl acetate/hexanes gradient) to afford the title compound (racemic mixture).

The racemic mixture (6.35 g) was dissolved in 24 mL heptane and 8 mL isopropanol. Material was resolved on chiral AD column (15% isopropanol/heptane) to afford 2.9 g enantiomer A [τ_{R} :9.43 min (analytical chiral HPLC, AD column, 0.46 cm x 25 cm cm id, 15% isopropanol/heptane, isocratic, flow rate = 0.75 mL/min)] and 2.9 g enantiomer B [τ_{R}: 10.92 min (analytical chiral HPLC, AD column, 0.46 cm x 25 cm cm id, 15% isopropanol/heptane, isocratic, flow rate = 0.75 mL/min)]. LRMS (APCI) calc'd for (C₁₄H₂₀NO₄) [M+H]+, 266.1; found, 266.2.

### Step 2: Benzyl [(2R)-1,4-dioxan-2-ylmethyl]methylcarbamate

Benzyl (1,4-dioxan-2-ylmethyl)methylcarbamate (Enantiomer A, 2.9 g, 10.9 mmol) was dissolved in 50 mL dry ethanol. 10% (w/w) palladium on carbon (0.29 g) and 1.0 mL 10N HCl were added. The flask was sealed and flushed with hydrogen. Stirred solution under a hydrogen balloon. After 12 hours, the solution was filtered through celite and concentrated *in vacuo* to afford the title compound. ¹H NMR (600 MHz, D⁶-DMSO) δ 8.64 (s, 2H); 3.82-3.75 (m, 2H); 3.69 (d, 1H); 3.64 (d, 1H); 3.59 (m, 1H); 3.44 (m, 1H); 3.22 (t, 1H); 2.94-2.84 (m, 2H); 2.51 (s, 3H).

### Step 3: tert-butyl {[((2R)-1,4-dioxan-2-ylmethyl)(methyl)amino]sulfonyl}carbamate

1-(1,4-dioxan-2-yl)-*N*-methylmethanamine hydrochloride (0.760 g, 4.55 mmol), *N*-[1-{[(*tert-*butoxycarbonyl)amino]sulfonyl}pyridin-4(1H)-ylidene]-*N*-methylmethanaminium (1.51 g, 5.00 mmol), and triethylamine (1.55 mL, 11.4 mmol) were slurried in 50 mL dichloromethane and stirred at ambient temperature. After 12 hours, the solution was concentrated *in vacuo* and purified by silica chromatography (50-100% ethyl acetate/hexanes gradient) to afford the title compound. LRMS (APCI) calc'd for (C₁₁H₂₂N₂O₆SNa) [M+Na]+, 333.1; found, 333.1.

### Step 4: {[((2R)1,4-dioxan-2-ylmethyl)(methyl)amino]sulfonyl}ammonium trifluoroacetate

*tert-*butyl {[(1,4-dioxan-2-ylmethyl)(methyl)amino]sulfonyl}carbamate (1.25 g, 4.03 mmol) was dissolved in 10 mL dichloromethane and 20 mL trifluoroacetic acid and stirred at ambient temperature. After 2 hours, the solution was concentrated and azeotroped twice with heptane to afford the title compound. LRMS (APCI) calc'd for (C₆H₁₅N₂O₄S) [M+H]+, 211.1; found, 211.1.

### Step 5: N'-(3-chloro-5-oxo-5H-benzo[4,5]cyclohepta[1,2-b]pyridin-7-yl)-N-[(2R)-1,4-dioxan-2-ylmethyl]-N-methylsulfamide

7-bromo-3-chloro-5*H-*benzo[4,5]cyclohepta[1,2*-b*]pyridin-5-one (1.41 g, 4.41 mmol), {[((2*R*)1,4-dioxan-2-ylmethyl)(methyl)amino]sulfonyl}ammonium trifluoroacetate (1.30 g, 4.01 mmol), tris(dibenzylideneacetone)dipalladium (0.183 g, 0.20 mmol), 9,9-dimethyl-4,5-bis(diphenylphosphino) xanthene (0.347 g, 0.60 mmol), and cesium carbonate (3.91 g, 12.0 mmol) were combined in a dry flask. 50 mL dry dioxane was added and argon was bubbled through the solution for five minutes. The solution was stirred and heated to 95°C. After 2 hours, the solution was concentrated *in vacuo,* diluted with ethyl acetate, and washed with water and brine. The organic layer was isolated, dried with magnesium sulfate, filtered, concentrated *in vacuo,* and purified by silica chromatography (0-100% ethyl acetate/hexanes gradient) to afford the title compound. LRMS (APCI) calc'd for (C₂₀H₂₁ClN₃O₅S) [M+H]+, 450.1; found, 450.1.

### Example 13

### Enantioselective synthesis of Benzyl [(2S)-1,4-dioxan-2-ylmethyl]methylcarbamate

### Step 1: (2S)-2-[(benzyloxy)methyl]-1,4-dioxane

(2*R*)-3-(benzyloxy)propane-1,2-diol (2.00 g, 11.0 mmol) and tetrabutylammonium bromide (708 mg, 2.20 mmol) were dissolved in 50 mL of 1,2-dichloroethane, then 50 mL of a 50% (w/w) aqueous sodium hydroxide solution was added quickly and the mixture was heated to 50 °C. After 18 h, an additional 50 mL of 1,2-dichloroethane and 50 mL of 50% (w/w) sodium hydroxide solution was added. After 8 h, additional 50 mL of 1,2-dichloroethane was added. After 72 h, the mixture was diluted in diethyl ether, washed with water and brine, then dried over sodium sulfate and concentrated. The residue was purified by flash column chromatography (silica, ethylacetate/hexanes) to afford the title compound. ¹H NMR (600 MHz, CDCl₃) δ 7.26-7.35 (m, 5H); 4.51-4.56 (m, 2H); 3.72-3.82 (m, 4H); 3.67-3.71 (m, 1H); 3.58-3.64 (m, 1H); 3.38-3.48 (m, 3H).

### Step 2: benzyl [(2S)-1,4-dioxan-2-ylmethyl]methylcarbamate

A round bottomed flask was charged with (2*S*)-2-[(benzyloxy)methyl]-1,4-dioxane (1.77 g, 8.48 mmol), 902 mg of 10% Pd/C and 50 mL of absolute ethanol. A three-way stopcock fitted with a hydrogen balloon was affixed to the flask, then the flask was evaporated and back-filled with hydrogen (4x) and stirred under the hydrogen atmosphere overnight. The mixture was filtered through Celite and concentrated to afford (2*S*)-1,4-dioxan-2-ylmethanol.

A round bottomed flask was charged with (2*S*)-1,4-dioxan-2-ylmethanol (115 mg, 0.973 mmol), triethylamine (0.204 mL, 1.46 mmol), and 5 mL of dichloromethane then cooled to -10 °C. Methanesulfonyl chloride (91 µL, 1.17 mmol) was added by syringe and the solution was stirred for 30 minutes at -10 °C. The solution was diluted in dichloromethane, washed with 1M HCl, and the aqueous layer was extracted with dichloromethane (2x). The combined organic layers were washed with saturated aquoues sodium bicarbonate (2x) and brine, then dried over sodium sulfate and concentrated to afford (2*R*)-1,4-dioxan-2-ylmethyl methanesulfonate.

Sodium hydride (29 mg, 0.74 mmol) was suspended in 2 mL of *N*,*N*-dimethylformamide (DMF) and cooled to 0 °C. A solution of benzyl methylcarbamate (81 mg, 0.49 mmol) in 2 mL of DMF was added via syringe. After 20 minutes, a solution of (2*R*)-1,4-dioxan-2-ylmethyl methanesulfonate (191 mg, 0.97 mmol) in 2 mL of DMF was added by syringe and the mixture was heated to 70°C. After 2 h the mixture was cooled to ambient temperature, then diluted in diethyl ether, washed with water and brine, then dried over sodium sulfate and concentrated. The residue was purified by flash column chromatography (silica, ethylacetate/hexanes) to afford the title compound. LRMS (APCI) calc'd for (C₁₄H₂₀NO₄) [M+H]+, 266.1; found, 266.2.

Analysis of benzyl [(2*S*)-1,4-dioxan-2-ylmethyl]methylcarbamate by analytical HPLC [τ_{R}: 10.85 min (analytical chiral HPLC, AD column, 0.46 cm x 25 cm id, 15% isopropanol/heptane, isocratic, flow rate = 0.75 mL/min)] and co-injection with Enantiomer A from Example 12 allowed for the following assignment of stereochemistry for the separated enantiomers of Example 12, Step 2.

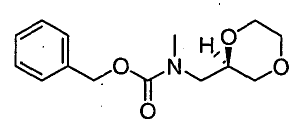

### Enantiomer A

### benzyl [(2R)-1,4-dioxan-2-ylmethyl]methylcarbamate

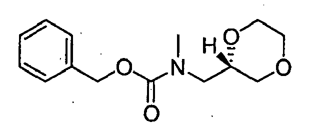

### Enantiomer B

### benzyl [(2S)-1,4-dioxan-2-ylmethyl]methylcarbamate

### Example 13A

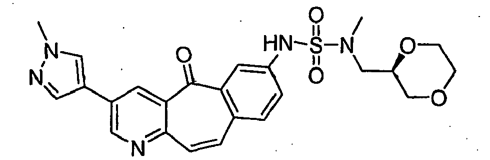

### N-[(2R)-1,4-dioxan-2-ylmethyl]-N-methyl-N'-[3-(1-methyl-1H-pyrazol-4-yl)-5-oxo-5H-benzo[4,5]cyclohepta[1,2-b]pyridin-7-yl]sulfamide (Compound 13)

*N*'-(3-chloro-5-oxo-5*H-*benzo[4,5]cyclohepta[1,2*-b*]pyridin-7-yl)-*N*-[(2R)1,4-dioxan-2-ylmethyl]-*N*-methylsulfamide (0.500 g, 1.11 mmol), 1-methyl-4-(4,4,5,5-tetramethyl-1,3,2-dioxaborolan-2-yl)-1*H-*pyrazole (0.692 g, 3.33 mmol), Pd₂(dba)₃ (0.051 g, 0.056 mmol), (tBu₃)PBF₄ (0.032 g, 0.11 mmol) and potassium fluoride (0.212 g, 3.66 mmol) were combined in a dry tube. 5 mL dry DMF was added and argon was bubbled through the solution for five minutes. The tube was sealed and heated in the Biotage Initiator series microwave to 135°C for 20 minutes. The solution was diluted with ethyl acetate and washed with saturated sodium bicarbonate, water, and brine. The organic layer was dried with magnesium sulfate, filtered, concentrated *in vacuo,* and purified by silica chromatography (0-100% ethyl acetate/hexanes gradient followed by 0-10% methanol/dichloromethane gradient) to afford the crude compound. The crude material was crystallized from a mixture of 10 mL methanol, 40 mL dichloromethane, and 70 mL hexanes to afford the title compound. ¹H NMR (600 MHz, D⁶-DMSO) δ 10.52 (s, 1H); 9.20 (d, 1H); 8.55 (d, 1H); 8.45 (s, 1H); 8.13 (s, 1H); 7.95 (d, 1H); 7.75 (d, 1H); 7.55 (d, 1H); 7.32 (d, 1H); 7.22 (d, 1H); 3.88 (s, 3H); 3.64-3.60 (m, 2H); 3.58-3.54 (m, 1H); 3.54-3.50 (m, 1H); 3.44-3.40 (m, 1H); 3.38-3.34 (m, 1H); 3.14-3.10 (m, 3H); 2.77 (s, 3H). LRMS (APCI) calc'd for (C₂₄H₂₆N₅O₅S) [M+H]+, 496.2; found, 496.2.

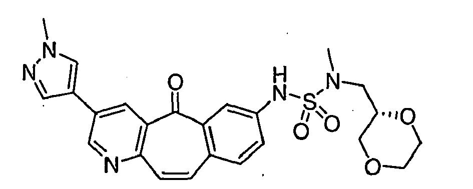

*N*-[(2*S*)-1,4-dioxan-2-ylmethyl]-*N*-methyl-*N*-[3-(1-methyl-1*H-*pyrazol-4-yl)-5-oxo-5*H-*benzo[4,5]cyclohepta[1,2*-b*]pyridin-7-yl]sulfamide (Compound 13S) was prepared using the procedure described in Examples 12 and 13A, but substituting benzyl [(2*S*)-1,4-dioxan-2-ylmethyl]methylcarbamate (Enantiomer B from Example 12, Step 1) for benzyl [(2*R*)-1,4-dioxan-2-ylmethyl]methylcarbamate in Example 12, Step 2.

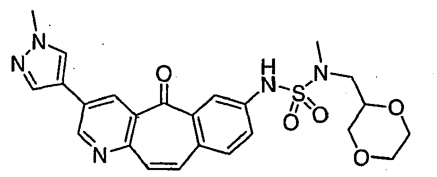

*N*-[1,4-dioxan-2-ylmethyl]-*N*-methyl-*N*'-[3-(1-methyl-1*H-*pyrazol-4-yl)-5-oxo-5*H-*benzo[4,5]cyclohepta[1,2*-b*]pyridin-7-yl]sulfamide racemic mixture (Compound 13RS) was prepared using the procedure described in Examples 12 and 13A, but substituting racemic benzyl [1,4-dioxan-2-ylmethyl]methylcarbamate for benzyl [(2*R*)-1,4-dioxan-2-ylmethyl]methylcarbamate in Example 12, Step 2.

The enantiomeric components of this racemic mixture of the instant compound were separated by the following procedure: The racemic Compound 12RS (0.083 g) was dissolved in a mixture of 2 mL methanol and 18 mL dichloromethane. Material was resolved on chiral OD column (70% isopropanol/heptane) to afford 0.030 g enantiomer A (Compound 13) [τ_{R} : 12.8 min (analytical chiral HPLC, OD column, 0.46 cm x 25 cm cm id, 60% isopropanol/heptane, isocratic, flow rate = 0.75 mL/min)] and 0.026 g enantiomer B (Compound 13S) [τ_{R} : 15.8 min (analytical chiral HPLC, OD column, 0.46 cm x 25 cm cm id, 60% isopropanol/heptane, isocratic, flow rate = 0.75 mL/min)].

### Example 14

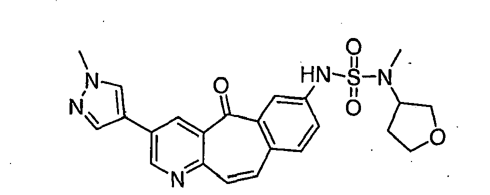

### N-methyl-N'-[3-(1-methyl-1H-pyrazol-4-yl)-5-oxo-5H-benzo[4,5]cyclohepta[1,2-b]pyridin-7-yl]-N-(tetrahydrofuran-3-yl)sulfamide (Compound 14)

### Step 1: N-methyl-N-(tetrahydrofuran-3-yl)sulfamide

A flask was charged with *N*-tert-butoxycarbonyl-*N*-[4-(dimethylazaniumylidene)-1,4-dihydropyridin-1-ylsulfonyl]azanide (2.19 g, 7.27 mmol), *N*-methyltetrahydrofuran-3-aminium chloride (1.00 g, 7.27 mmol) and triethyl amine (1.01 mL, 7.27 mmol) in 10 mL of CH₂Cl₂. After 2 h, the solution was concentrated *in vacuo* and purified by flash column chromatography (10-100% EtOAc/hexanes) to afford *tert-*butyl {[methyl(tetrahydrofuran-3-yl)amino]sulfonyl}carbamate.
*tert-*butyl {[methyl(tetrahydrofuran-3-yl)amino]sulfonyl}carbamate (1.47 g, 5.23 mmol) was dissolved in 70 mL of CH₂Cl₂ and 45 mL of trifluoroacetic acid. After 1 h the solution was concentrated *in vacuo,* diluted in CH₂Cl₂, washed with saturated aqueous NaHCO₃ and brine, then dried over Na₂SO₄. The solution was concentrated *in vacuo* to afford the title compound. ¹H NMR (600 MHz, DMSO-*d6*) δ 4.25-4.31 (m, 1H); 3.79-3.84 (m, 1H); 3.58-3.66 (m, 2H); 3.47-3.52 (m, 1H); 2.56 (s, 3H), 2.04-2.10 (m, 1H); 1.81-1.88 (m, 1H).

### Step 2: N'-(3-chloro-5-oxo-5H-benzo[4,5]cyclohepta[1,2-b]pyridin-7-yl)-N-methyl-N-({3R}-tetrahydrofuran-3-yl)sulfamide and N-(3-chloro-5-oxo-5H-benzo[4,5]cyclohepta[1,2-b]pyridin-7-yl)-N-methyl-N-({3R}-tetrahydrofuran-3-yl)sulfamide

7-bromo-3-chloro-5*H-*benzo[4,5]cyclohepta[1,2*-b*]pyridin-5-one (665 mg. 2.07 mmol), *N*-methyl-*N-*(tetrahydrofuran-3-yl)sulfamide (372 mg, 2.06 mmol), Pd₂(dba)₃ (95 mg, 0.10 mmol), 9,9-dimethyl-4,5- bis(diphenylphosphino) xanthene (XANTPHOS) (179 mg, 0.310 mmol) and cesium carbonate (2.02 g, 6.19 mmol) were added to a dry flask through which argon was purged. The flask was charged with 30 mL of dry dioxane and argon was bubbled through the solution for 10 minutes. The reaction mixture was heated to 95°C and stirred under argon. After 2h, the mixture was cooled to ambient temperature, diluted in ethyl acetate, washed with saturated aqueous NaHCO₃ and brine, then dried over Na₂SO₄. The solution was concentrated and purified via flash column chromatography (silica, ethylacetate/hexanes) to afford the title compound. ¹H NMR (600 MHz, DMSO-*d6*) δ 10.59 (s, 1H); 8.97 (d, 1H); 8.50 (d, 1H); 7.95 (d, 1H); 7.79 (d, 1H); 7.54 (dd, 1H); 7.40 (d, 1H); 7.22 (d, 1H); 4.47-4.53 (m, 1H); 3.75-3.80 (m, 1H); 3.42-3.53 (m, 3H); 2.67 (s, 3H); 1.93-2.00 (m, 1H); 1.50-1.72 (m, 1H);.LRMS (APCI) calc'd for (C₁₉H₁₉ClN₃O₄S) [M+H]+, 420.1; found, 420.1.
The racemic mixture was dissolved in 5 mg/mL of 5:1 methanol/dimethylsulfoxide and resolved on via chiral HPLC (Chiracel OJ-H column, 21 mm x 250 mm, 40% methanol/supercritical carbon dioxide, flow rate = 50 mL/min, 100 bar outlet pressure) to afford enantiomer A (τ_{R} = 6.33 min) and enantiomer B (τ_{R} =7.9 min)

### Step 3: N-methyl-N'-[3-(1-methyl-1H-pyrazol-4-yl)-5-oxo-5H-benzo[4,5]cyclohepta[1,2-b]pyridin-7-yl]-N-({3R}-tetrahydrofuran-3-yl)sulfamide and N-methyl-N'-[3-(1-methyl-1H-pyrazol-4-yl)-5-oxo-5H-benzo[4,5]cyclohepta[1,2-b]pyridin-7-yl]-N-({3S}-tetrahydrofuran-3-yl)sulfamide

The separated enantiomers were carried forward in the same manner. A procedure is described for Enantiomer B.
N'-(3-chloro-5-oxo-5H-benzo[4,5]cyclohepta[1,2*-b*]pyridin-7-yl)-N-methyl-N-(tetrahydrofuran-3-yl)sulfamide Enantiomer B (0.070 g, 0.17 mmol), 1-methyl-4-(4,4,5,5-tetramethyl-1,3,2-dioxaborolan-2-yl)-1H-pyrazole (0.069 g, 0.33 mmol), Pd₂(dba)₃ (0.008 g, 0.0085 mmol), (tBu₃)PBF₄ (0.005 g, 0.017 mmol) and potassium fluoride (0.032 g, 0.56 mmol) were combined in a dry tube. 1.0 mL dry DMF was added and argon was bubbled through the solution for five minutes. The tube was sealed and heated in a Biotage Initiator series microwave reactor to 100°C for 30 minutes. The solution was diluted with ethyl acetate and washed with saturated sodium bicarbonate. The organic layer was dried with magnesium sulfate, filtered, concentrated in vacuo, and purified by HPLC chromatography (20-100% acetonitrile/water gradient, 0.05% trifluoroacetic acid modifier) to afford the crude compound. The crude material was purified by silica chromatography (0-100% ethyl acetate/hexanes gradient, followed by 0-20% methanol/dichloromethane gradient). The isolated material was dissolved in minimal 25% methanol/dichloromethane and hexanes were added until precipitation occurred. Precipitate was filtered to afford title compound. ¹H NMR (600 MHz, D⁶-DMSO) □ 10.55 (s, 1H); 9.20 (d, 1H); 8.58 (d, 1H); 8.46 (s, 1H); 8.13 (s, 1H); 7.95 (d, 1H); 7.75 (d, 1H); 7.52 (dd, 1H); 7.32 (d, 1H); 7.22 (d, 1H);4.48-4.53 (m, 1H); 3.88 (s, 3H); 3.74-3.80 (m, 1H); 3.42-3.54 (m, 3H); 2.68 (s, 3H); 1.93-2.00 (m, 1H); 1.65-1.72 (m, 1H). LRMS (APCI) calc'd for (C₂₃H₂₄N₅O₄S) [M+H]+, 466.2; found, 466.2.
The racemic mixture of *N*-methyl-*N'*-[3-(1-methyl-1*H-*pyrazol-4-yl)-5-oxo-5*H-*benzo[4,5]cyclohepta[1,2-*b*]pyridin-7-yl]-*N*(tetrahydrofuran-3-yl)sulfamide was prepared using the above procedure starting with the racemic mixture from Step 2.

The following compounds were made according to the methods described above. Commercially unavailable 1-*H-*pyrazole-4-boronic esters were prepared in a manner similar to that described in Example **12**.

**Table 3**

| | | | |
|---|---|---|---|
| 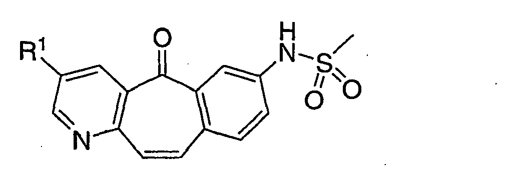 | | | |

| Comp. # | R¹ | Name | MS (M+1) |
|---|---|---|---|
| 15 | 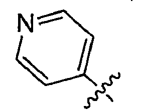 | *N*-(5-oxo-3-pyridin-4-yl-5*H-*benzo[4,5]cyclohepta[1,2*-b*]pyridin-7-yl)methanesulfonamide | calc'd 378.1 (M+H)+; found 378.1 (M+H)+ |
| 16 | 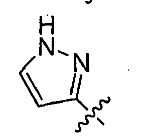 | *N*-[5-oxo-3-(1*H-*pyrazol-3-yl)-5*H-*benzo[4,5]cyclohepta[1,2*-b*]pyridin-7-yl]methanesulfonamide | calc'd 367.1 (M+H)+; found 367.1 (M+H)+ |
| 17 | 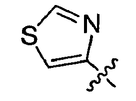 | *N*-[5-oxo-3-(1,3-thiazol-4-yl)-5*H-*benzo[4,5]cyclohepta[1,2-b]pyridin-7-yl]methanesulfonamide | calc'd 384.0 (M+H)+; found 384.0 (M+H)+ |
| 18 | 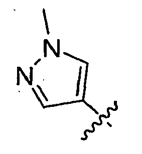 | *N*-[3-(1-methyl-1*H-*pyrazol-4-yl)-5-oxo-5*H-*benzo[4,5]cyclohepta[1,2-*b*]pyridin-7-yl]methanesulfonamide | calc'd 381.1 (M+H)+; found 380.7 (M+H)+ |
| 18A | 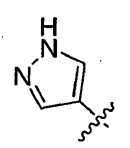 | *N*-[5-oxo-3-(1*H-*pyrazol-4-yl)-5*H-* benzo[4,5]cyclohepta[1,2*-b*]pyridin-7-yl]methanesulfonamide | calc'd 367.1 (M+H)+; found 366.7 (M+H)+ |
| 19 | 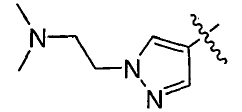 | *N*-(3-{1-[2-(dimethylamino)ethyl]-1*H-* pyrazol-4-yl}-5-oxo-5*H-*benzo[4,5]-cyclohepta[1,2*-b*]pyridin-7-yl)methanesulfonamide, 3TFA or 3HCl | calc'd 438.2 (M+H)+; found 437.7 (M+H)+ |
| 20 | 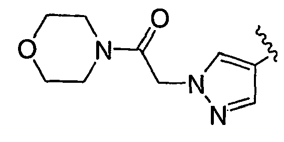 | *N*-{3-[1-(2-morpholin-4-yl-2-oxoethyl)-1*H-*pyrazol-4-yl]-5-oxo-5*H-*benzo[4,5]cyclohepta[1,2*-b*]pyridin-7-yl}methanesulfonamide | calc'd 494.1 (M+H)+; found 493.6 (M+H)+ |
| 21 | 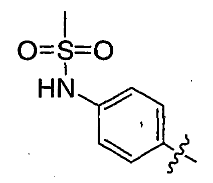 | *N*-(4-{7-[(methylsulfonyl)amino]-5-oxo-5*H-*benzo[4,5]cyclohepta[1,2-b]pyridin-3-yl}phenyl)methanesulfonamide | calc'd 470.1 (M+H)+; found 470.1 (M+H)+ |
| 22 | 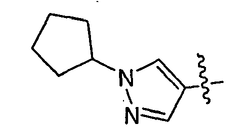 | *N*-[3-(1-cyclopentyl-1*H-*pyrazol-4-yl)-5-oxo-5*H-*benzo[4,5]cyclohepta[1,2*-b*]pyridin-7-yl]methanesulfonanide | calc'd 435.1 (M+H)+; found 435.1 (M+H)+ |
| 23 | 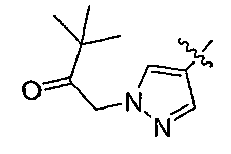 | *N*-{3-[1-(3,3-dimethyl-2-oxobutyl)-1*H-*pyrazol-4-yl]-5-oxo-5*H-*benzo[4,5]cyclohepta[1,2*-b*]pyridin-7-yl}methanesulfonamide | calc'd 465.2 (M+H)+; found 465.2 (M+H)+ |
| 24 | 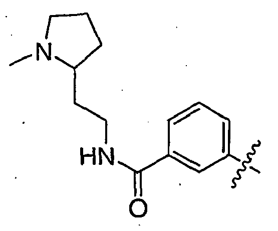 | *N*-[2-(1-methylpyrrolidin-2-yl)ethyl]-3-{7-[(methylsulfonyl)amino]-5-oxo-5*H-* benzo[4,5]cyclohepta[1,2-b]pyridin-3-yl}benzamide | calc'd 531.2(M+H) +; found 531.0(M+H) + |

**Table 3A**

| Comp. # | Structure | Name | MS (M+1) |
|---|---|---|---|
| 25 | 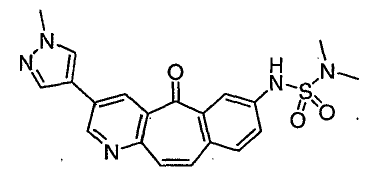 | *N,N*-dimethyl-*N*-[3-(1-methyl-1*H-*pyrazol-4-yl)-5-oxo-5*H-*benzo[4,5]cyclohepta[1,2 *-b*]pyridin-7-yl]sulfamide | MS (M+1) calc'd 410.1 (M+H)+; found 409.7 (M+H)+ |
| 26 | 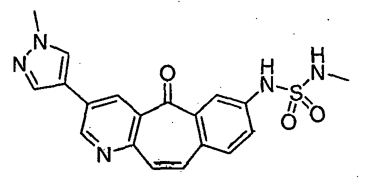 | *N*-methyl-*N*'-[3-(1-methyl-1*H-*pyrazol-4-yl)-5-oxo-5*H-*benzo[4,5]cyclohepta[1,2 *-b*]pyridin-7-yl]sulfamide | calc'd 382.1 (M+H)+; found 382.1 (M+H)+ |
| 26A | 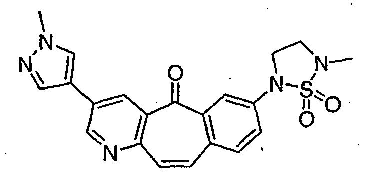 | 7-(5-methyl-1,1-dioxido-1,2,5-thiadiazolidin-2-yl)-3-(1-methyl-1*H-*pyrazol-4-yl)-5*H-*benzo[4,5]cyclohepta[1,2 *-b*]pyridin-5-one | calc'd 422.1 (M+H)+; found 422.1 (M+H)+ |
| 27 | 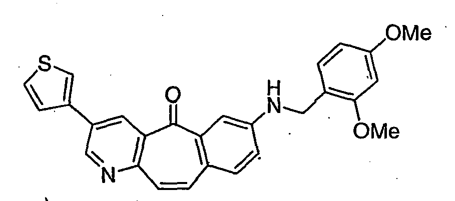 | 7-[(2,4-dimethoxybenzyl)amino]-3-(3-thienyl)-5*H-*benzo[4,5]cyclohepta[1,2 *-b*]pyridin-5-one | calc'd 455.1 (M+H)+; found 455.2 (M+H)+ |
| 28 | 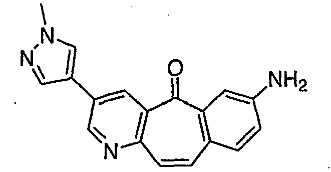 | 7-amino-3-(1-methyl-1*H-*pyrazol-4-yl)-5*H-*benzo[4,5]cyclohepta[1,2 *-b*]pyridin-5-one, isolated as free base and 3HCl salt | calc'd 303.1 (M+H)+; found 303.1 (M+H)+ |
| 29 | 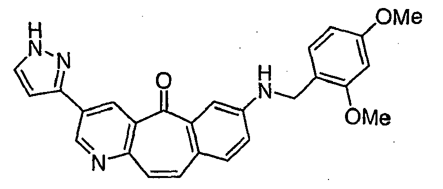 | 7-[(2,4-dimethoxybenzyl)amino]-3-(1*H-*pyrazol-3-yl)-5*H-*benzo[4,5]cyclohepta[1,2 *-b*]pyridin-5-one | calc'd 439.2 (M+H)+; found 439.2 (M+H)+ |
| 30 | 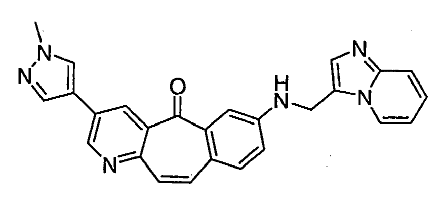 | 7-[(imidazo[1,2-a]pyridin-3-ylmethyl)amino]-3-(1-methyl-1*H-*pyrazol-4-yl)-*5*H-benzo[4,5]cyclohepta[1,2 *-b*]pyridin-5-one | calc'd 433.2(M+H) +; found 433.2(M+H) + |
| 31 | 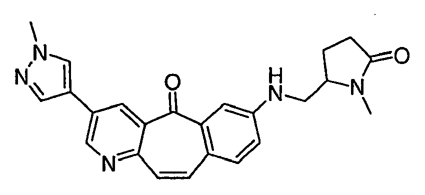 | 7-{[(1-methyl-5-oxopyrrolidin-2-yl)methyl]amino}-3-(1-methyl-1*H-*pyrazol-4-yl)-5*H-*benzo[4,5]cyclohepta[1 ,2*-b*]pyridin-5-one | calc'd 414.2(M+H) +; found 414.1 (M+H) + |
| 32 | 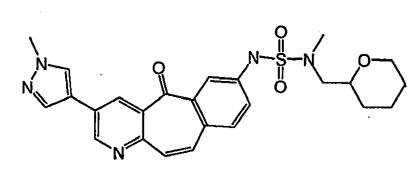 | *N*-methyl-*N*-[3-(1-methyl-1*H-*pyrazol-4-yl)-5-oxo-5*H-*benzo[4,5]cyclohepta[1 ,2*-b*]pyridin-7-yl]-*N*-(tetrahydro-2*H-*pyran-2-ylmethyl)sulfamide | calc'd 494.2 (M+H)+; found 494.2 (M+H)+ |
| 33 | 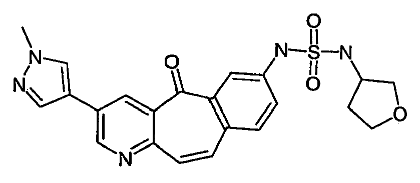 | *N-[3-(1-methyl-1H-pyrazol-4-yl)-5-oxo-5H*-*benzo[4,5]cyclohepta[1 ,2-b]pyridin-7-yl]-N'-(tetrahydrofuran-3-yl)sulfamide* | calc'd 452.1 (M+H)+; found 452.2 (M+H)+ |
| 34 | 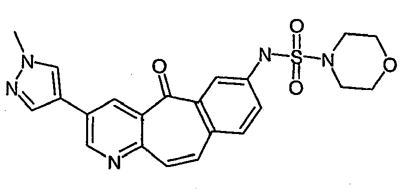 | *N-[3-(1-methyl-1H-pyrazol-4-yl)-5-oxo-5H-benzo[4,5]cyclohepta[1 ,2-blpyridin-7-yl]morpholine-4-sulfonamide* | calc'd 452.1 (M+H)+; found 452.2 (M+H)+ |

### Example 15

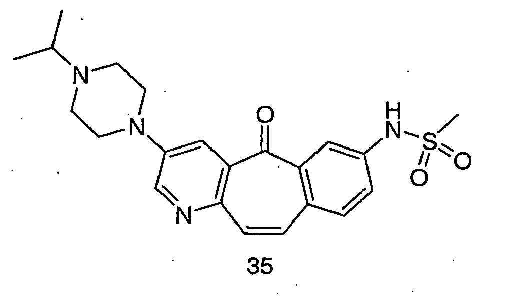

### N-[3-(4-isopropylpiperazin-1-yl)-5-oxo-5H-benzo[4,5]cyclohepta[1,2-b]pyridin-7-yllmethanesulfonamide.

### Method A:

*N*-(3-chloro-5-oxo-5*H-*benzo[4,5]cyclohepta[1,2*-b*]pyridin-7-yl)methanesulfonamide (0.050 g, 0.15 mmol), isopropylpiperazine (0.038 g, 0.30 mmol), Pd₂(dba)₃ (1.5 mg, 0.0015 mmol), BINAP (3.0 mg, 0.0045 mmol), and sodium *tert-*butoxide (0.043 g, 0.45 mmol) were added to a dry flask through which argon was purged. 3.0 mL dry dioxane was added and argon was bubbled through the solution for 5 minutes. The reaction was stirred and heated to 105°C. After 12 hours, the reaction mixture was poured into 100 mL ethyl acetate, 100 mL water, and 25 mL saturated ammonium chloride. The organic layer was separated, dried with magnesium sulfate, filtered, concentrated, and purified by reverse phase HPLC (20-100% acetonitrile/water gradient, 0.1 % trifluoroacetic acid modifier) to afford the title compound.

### Method B:

*N*-(3-chloro-5-oxo-5*H-*benzo[4,5]cyclohepta[1,2*-b*]pyridin-7-yl)methanesulfonamide (0.100 g, 0.30 mmol), Pd₂(dba)₃ (6 mg, 0.006 mmol), *rac-*BINAP (11 mg, 0.018 mmol), and cesium carbonate (0.490 g, 1.50 mmol) combined mixed in a dry tube. Isopropylpiperazine (0.170 mL, 1.20 mmol) and 0.70 mL of dry dimethylformamide were added and the tube was sealed. The reaction contents were heated in the Biotage Initiator series microwave at 180°C for 15 minutes. The reaction contents were partially concentrated and purified by reverse phase HPLC (10-100% acetonitrile/water gradient, 0.1% trifluoroacetic acid modifier) to afford the title compound.

¹H NMR (600 MHz, CD₃OD) δ 8.64 (d, 1H); 8.18 (s, 1H); 7.96 (d, 1H); 7.68 (d, 1H); 7.60 (dd, 1H); 7.20 (d, 1H); 7.18 (d, 1H); 3.45 (m, 4H); 3.04 (s, 3H); 2.78 (m, 5H); 1.14 (d, 6H). LRMS (APCI) calculated for C₂₂H₂₇N₄O₃S [M+H]+, 427.2; found 427.2.

The following compounds were made as illustrated above. Additional synthetic modifications were employed in the preparation of some of the compounds.

**Table 4**

| Comp. # | Structure | Name | MS (M+1) |
|---|---|---|---|
| 36 | 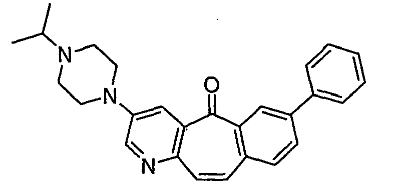 | 3-(4-isopropylpiperazin-1-yl)-7-phenyl-5H-benzo[4,5]cyclohepta[1,2-b]pyridin-5-one | calc'd 410.2 (M+H)+; found 410.2 (M+H)+ |
| 37 | 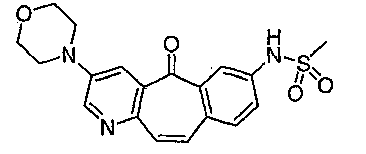 | *N*-(3-morpholin-4-yl-5-oxo-5*H-*benzo[4,5]cyclohepta[1,2-b]pyridin-7-yl)methanesulfonamide | calc'd 386.1 (M+H)+; found 386.1 (M+H)+ |
| 38 | 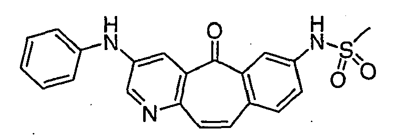 | *N*-(3-anilino-5-oxo-5*H-*benzo[4,5]cyclohepta[1,2-b]pyridin-7-yl)methanesulfonamide | calc'd 392.1 (M+H)+; found 392.1 (M+H)+ |
| 39 | 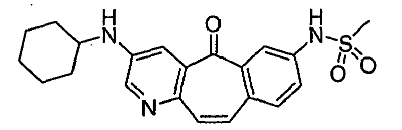 | *N*-[3-(cyclohexylamino)-5-oxo-5*H-*benzo[4,5]cyclohepta[1,2-b]pyridin-7-yl]methanesulfonamide | calc'd 398.2 (M+H)+; found 398.2 (M+H)+ |
| 40 | 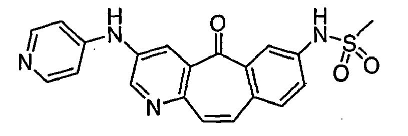 | *N*-[5-oxo-3-(pyridin-4-ylamino)-5*H-*benzo[4,5]cyclohepta[1,2-b]pyridin-7-yl]methanesulfonamide | calc'd 393.1 (M+H)+; found 393.1 (M+H)+ |

### Example 16

### N-(3-chloro-5-oxo-5H-benzo[4,5]cyclohepta[1,2-b]pyridin-7-yl)-2-methoxyacetamide.

7-amino-3-chloro-5*H-*benzo[4,5]cyclohepta[1,2-b]pyridin-5-one (0.70 g, 2.7 mmol) was dissolved in 20 mL of dry dichloromethane and 5 mL of dry acetonitrile. Methoxyacetic acid (0.32 mL, 4.1 mmol), N-(3-Dimethylaminopropyl)-N'-ethylcarbodiimide hydrochloride (EDCI) (0.79 g, 4.1 mmol) and 1-hydroxybenzotriazole hydrate (HOBt) (0.55 g, 4.1 mmol) were added and the solution was stirred at ambient temperature. After 12 hours, the reaction solution was poured into 300 mL of ethyl acetate and 100 mL of water. The organic layer was separated and washed with 100 mL of brine. The organic layer was separated, dried with magnesium sulfate, filtered, concentrated and purified by flash column chromatography (0-100% ethyl acetate/hexanes gradient) to afford the title compound. ¹H NMR (600 MHz, CDCl₃) δ 10.29 (s, 1H); 8.94 (d, 1H); 8.58 (d, 1H); 8.46 (d, 1H); 8.10 (dd, 1H); 7.77 (d, 1H); 7.40 (d, 1H); 7.20 (d, 1H); 4.04 (s, 2H); 3.36 (s, 3H). LRMS (APCI) calculated for C₁₇H₁₄ClN₂O₃ [M+H]+, 329.1; found 329.1.

### Example 17

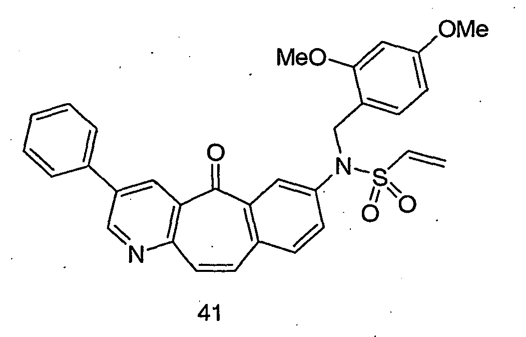

### N-(2,4-dimethoxybenzyl)-N-(5-oxo-3-phenyl-5H-benzo[4,5]cyclohepta[1,2-b]pyridin-7-yl)ethylenesulfonamide.

A flask was charged with compound **12** (194 mg, 0.434 mmol) and 8 mL of CH₂Cl₂ and cooled to 0 °C. *N*-methylmorpholine (0.19 mL, 1.74 mmol) and 2-chloroethanesulfonyl chloride (90 µL, 0.87 mmol) were added and the solution was allowed to warm to room temperature. After 18 h, the solution was diluted with EtOAc, washed with water and brine, then dried over Na₂SO₄. The solution was concentrated *in vacuo* and purified by flash column chromatography (10-100% EtOAc/hexanes gradient) to afford the title compound **41**. ¹H NMR (600 MHz, CDCl₃) δ 9.14 (d, 1H); 8.73 (d, 1H); 8.19 (d, 1H); 7.69-7.72 (m, 2H); 7.58 (dd, 1H); 7.49-7.54 (m, 3H); 7.44-7.47 (m, 1H); 7.39 (d, 1H); 7.19-7.25 (m, 2H); 6.57 (dd, 1H); 6.37 (dd, 1H); 6.30 (app d, 1H); 6.21 (d, 1H); 6.00 (d, 1H); 4.86 (s, 2H); 3.72 (s, 3H); 3.65 (s, 3H). LRMS (APCI) calc'd for (C₃₁H₂₇N₂O₅S) [M+H]+, 539.2; found 539.2

### Example 18

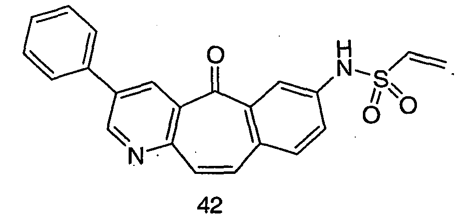

### N-(5-oxo-3-henyl-5H-benzo[4,5]cyclohepta[1,2-b]pyridin-7-yl)ethylenesulfonamide.

Compound **42** was prepared from **41** via, the method described for Example **4B.** ¹H NMR (600 MHz, DMSO-d6) δ 10.69 (s, 1H); 9.29 (257, 1H); 8.68 (d, 1H); 7.98 (d, 1H); 7.86-7.90 (m, 2H); 7.78 (d, 1H); 7.52-7.58 (m, 3H); 7.45-7.48 (m, 1H); 7.41 (d, 1H); 7.29 (d, 1H); 6.86 (dd, 1H); 6.20 (d, 1H); 6.06-6.10 (m, 1H). LRMS (APCI) calc'd for (C₂₂H₁₇N₂O₃S) [M+14]+, 389.1; found 389.1

### Example 19

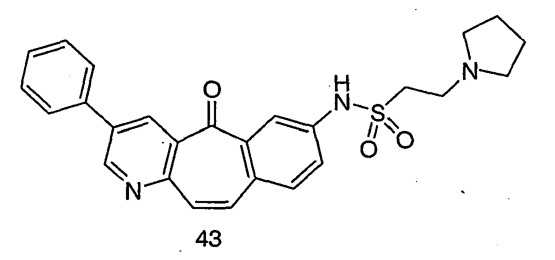

### N-(5-oxo-3-phenyl-5H-benzo[4,5]cyclohepta[1,2-b]pyridin-7-yl)-2-pyrrolidin-1-ylethanesulfonamide.

Compound **42** (20.0 mg, 0.051 mmol) and pyrrolidine (13 µL, 0.15 mmol) were dissolved in 2 mL of MeOH and 1 mL of CH₂Cl₂. After 18 h, the solution was concentrated under a stream of nitrogen and purified by purified by reverse phase HPLC (20-100% CH₃CN/water with a 0.1% TFA modifier) to afford the title compound **43.** ¹H NMR (600 MHz, CDCl₃) δ 9.13 (d, 1H); 8.74 (d, 1H); 7.92 (d, 1H); 7.73 (dd, 1H); 7.68-7.73 (m, 2H); 7.58 (d, 1H); 7.49-7.53 (m, 2H); 7.42-7.45 (m, 1H); 7.36 (d, 1H); 7.23 (d, 1H); 3.28-3.32 (m, 2H); 3.08-3.12 (m, 2H); 2.60-2.65 (m, 4H); 1.88-1.94 (m, 4H). LRMS (APCI) calc'd for (C₂₆H₂₆N₃O₃S) [M+H]₊, 460.2; found 460.

### Example 20

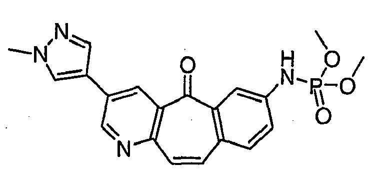

### dimethyl [3-(1-methyl-1H-pyrazol-4-yl)-5-oxo-5H-benzo[4,5]cyclohepta[1,2-b]pyridin-7-yl]amidophosphate. (Compound 44)

Compound 27A (10 mg, 0.033 mmol) and triethylamine (14 µL, 0.10 mmol) was suspended in 2 mL dichloromethane and dimethyl chloridophosphate (7 µL, 0.066 mmol) was added. After 30 min, the suspension was heated to 40 °C. After an additional 2 h, dimethyl chloridophosphate (36 µL, 0.33 mmol) was added. After an additional 18 h the yellow solution was poured into ethylacetate and the organic layer was washed with saturated aqueous sodium bicarbonate and brine, then dried over sodium sulfate and concentrated. The residue was purified via reverse phase HPLC (20-100% acetonitrile/water gradient, 0.05% trifluoroacetic acid modifier) to afford the title compound. ¹H NMR (600 MHz, CDCl₃) δ 8.89 (d, 1H); 8.63 (s, 1H);.7.88 (s, 1H); 7.82 (d, 1H); 7.79 (s, 1H); 7.51 (d, 1H); 7.32-7.38 (m, 2H); 7.18-7.22 (m, 1H); 6.06 (br d, 1H); 3.94 (s, 3H); 3.79(s, 3H); 3.77 (s, 3H);LRMS (APCI) calc'd for (C₂₀H₂₀N₄O₄P) [M+H]+, 411.1; found 411.1.

### Example 21

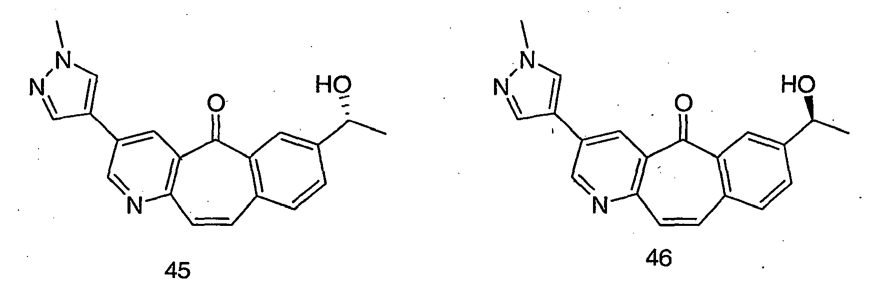

### Step 1: 3-chloro-7-vinyl-5H-benzo[4,5]cyclohepta[1,2-b]pyridin-5-one

A test tube fitted with a teflon septum was charged with compound 1 (1.0 g, 3.1 mmol), PdCl₂(dppf) (0.12 g, 0.16 mmol), and potassium vinyltrifluoroborate (0.42 g, 3.1 mmol). The tube was evacuated and backfilled with argon three times. Fully degassed n-PrOH (30 mL) was added followed by the addition of triethylamine (1.3 mL, 9.4 mmol). The mixture was heated to 100°C for 3 hours. The solution was diluted with ethyl acetate and washed with water and brine and dried over magnesium sulfate. The solution was concentrated *in vacuo* and purified via flash chromatography (silica, 0-25% ethyl acetate/hexanes) to afford the title compound. ¹H NMR (600 MHz, CDCl₃) δ 8.80 (d, 1H); 8.53 (d, 1H); 8.27 (d, 1H); 7.76 (dd, 1H); 7.57 (d, 1H); 7.32 (d, 1H); 7.26 (d, 1H); 6.83 (dd; 1H); 5.94 (d, 1H); 5.43 (d, 1H). LRMS (APCI) calc'd for (C₁₆H₁₁ClNO) [M+H]+, 268.1; found 268.1.

### Step2: 3-chloro-7-oxiran-2-yl-5H-benzo[4,5]cyclohepta[1,2-b]pyridin-5-one

3-chloro-7-vinyl-5*H*-benzo[4,5]cyclohepta[1,2-*b*]pyridin-5-one (0.30 g, 1.12 mol) was dissolved in 17 mL DMSO and 3.0 mL water. N-Bromosuccinimide (0.20 g, 1.12 mmol) was added and the reaction was heated in an oil bath at 60°C for 1 hour at which time an additional 0.1 g N-Bromosuccinimide (0.56 mmol) was added and the mixture was stirred an additional 45 min at 60°C. The resulting mixture was diluted with water and extracted with ethyl acetate three times. The combined organics were washed with brine, dried over sodium sulfate, filtered, and concentrated *in vacuo.* The resulting crude residue was dissolved in 30 mL tetrahydrofuran and 6mL *t*-BuOH. *t*-BuOK (2.24 mL of 1.0 M in THF, 2.24 mmol) was added dropwise and the resulting orange slurry was stirred at room temperature for 45 min. The reaction was diluted with water and extracted with ethyl acetate three times. The combined organics were washed with brine, dried over magnesium sulfate, concentrated *in vacuo,* and purified via flash chromatography (silica, 0-25% ethyl acetate/hexanes) to afford the title compound. ¹H NMR (600 MHz, CDCl₃) δ 8.79 (d, 1H); 8.49 (d, 1H); 8.20 (d, 1H); 7.57 (d, 1H); 7.55 (dd, 1H); 7.27 (d, 1H); 7.23 (d, 1H); 3.99 (dd, 1H); 3.21 (dd, 1H); 2.84 (dd, 1H).

### Step 3: 3-chloro-7-(1-hydroxyethyl)-5H-benzo[4,5]cyclohepta[1,2-b]pyridin-5-ol and 3-chloro-7-(2-hydroxyethyl)-5H-benzo[4,5]cyclohepta[1,2-b]pyridin-5-ol

3-chloro-7-oxiran-2-yl-5*H*-benzo[4,5]cyclohepta[1,2-*b*]pyridin-5-one (0.67 g, 2.4 mmol) was dissolved in 30 mL THF. LiAlH₄ (90 mg, 2.4 mmol) was added and the reaction was stirred at room temperature for 2 hours. The reaction was quenched via the dropwise addition of water followed by slow addition of 1N HCl. The mixture was extracted with ethyl acetate. The combined organics were washed with brine, dried over magnesium sulfate, filtered, and concentrated *in vacuo.* The crude mixture was used without further purification.
3-chloro-7-(1-hydroxyethyl)-5*H*-benzo[4,5]cyclohepta[1,2-*b*]pyridin-5-ol
LRMS (APCI) calc'd for (C₁₆H₁₅ClNO₂) [M+H]+, 288.1; found 288.1.
3-chloro-7-(2-hydroxyethyl)-5*H*-benzo[4,5]cyclohepta[1,2-*b*]pyridin-5-ol
LRMS (APCI) calc'd for (C₁₆H₁₅ClNO₂) [M+H]+, 288.1; found 288.1.

### Step 4: 7-(1-{[tert-butyl(dimethyl)silyl]oxy}ethyl)-3-chloro-5H-benzo[4,5]cyclohepta[1,2-b]pyridin-5-one and 3-chloro-7-(2-{[tert-butyl(dimethyl)silyl]oxy}ethyl)-3-chloro-5H-benzo[4,5]cyclohepta[1,2-b]pyridin-5-one

A crude mixture of 3-chloro-7-(1-hydroxyethyl)-5*H*-benzo[4,5]cyclohepta[1,2-*b*]pyridin-5-ol and 3-chloro-7-(2-hydroxyethyl)-5*H*-benzo[4,5]cyclohepta[1,2-*b*]pyridin-5-ol (0.67 g, 2.4 mmol) was dissolved in 30 mL N.N'-dimethylformamide. Imidazole (0.82 g, 6.0 mmol) and TBSCl (0.45 g, 3.0 mmol) were added sequentially and the reaction was stirred at 50°C for 2 hours at which time additional imidazole (0.82 g, 12 mmol) and TBSCl (0.45 g, 3.0 mmol) were added and the reaction was stirred an additional 2 hours. The mixture was diluted with water and saturated aqueous ammonium chloride and extracted three times with ethyl acetate. The combined organics were washed five times with brine, dried over magnesium sulfate and concentrated *in vacuo.* The resulting crude material was dissolved in 30 mL dichloromethane. MnO₂ (4.0 g, 46.5 mmol) was added and the reaction was stirred overnight at room temperature. The resulting slurry was filtered through a plug of celite with dichloromethane, concentrated *in vacuo,* and purified via flash chromatography (silica, 0-20% ethyl acetate/hexanes) to afford the title compounds as a mixture. The compounds were separated via flash chromatography (silica, 0-10% ethyl acetate/hexanes).
7-(1-{[*tert*-butyl(dimethyl)silyl]oxy}ethyl)-3-chloro-5*H*-benzo[4,5]cyclohepta[1,2-*b*]pyridin-5-one. ¹H NMR (600 MHz, CDCl₃) δ 8.79 (d, 1H); 8.53 (d, 1H); 8.18 (d, 1H); 7.76 (dd, 1H); 7.58 (d, 1H); 7.29 (d, 1H); 7.26 (d, 1H); 5.01 (q, 1H); 1.44 (d, 3 H); 0.91 (s, 9H); 0.07 (s, 3H); -0.01 (s, 3H). 7-(2-{[*tert*-butyl(dimethyl)silyl]oxy}ethyl)-3-chloro-5*H*-benzo[4,5]cyclohepta[1,2-*b*]pyridin-5-one. ¹H NMR (600 MHz, CDCl₃) δ 8.80 (d, 1H); 8.52 (d, 1H); 8.12 (d, 1H); 7.58 (dd, 1H); 7.53 (d, 1H); 7.29-7.27 (m, 2H); 3.86 (t, 2H); 2.96 (t, 2H); 0.84 (s, 9H); -0.04 (s, 6H).

### Step 5: 7-(1-{[tert-butyl(dimethyl)silyl]oxy}ethyl)-3-(1-methyl-1H-pyrazol-4-yl)-5H-benzo[4.5]cyclohepta[1,2-b]pyridin-5-one

A test tube fitted with a teflon septum was charged with 7-(1-{[*tert-*butyl(dimethyl)silyl]oxy}ethyl)-3-chloro-5*H*-benzo[4,5]cyclohepta[1,2-*b*]pyridin-5-one (50 mg, 0.13 mmol), PdCl₂(PPh₃)₂ (9 mg, 0.013 mmol), 1-methyl-4-(4,4,5,5-tetramethyl-1,3,2-dioxaborolan-2-yl)-1*H-*pyrazole (78 mg, 0.38 mmol), and sodium carbonate (40 mg, 0.38 mmol). The tube was evacuated and backfilled with argon three times. Fully degassed dioxane (1.2 mL) was added and the mixture was stirred at 100°C overnight. The solution was diluted with ethyl acetate, washed with water and brine, dried over magnesium sulfate, concentrated *in vacuo,* and purified via flash chromatography (silica, 20-100% ethyl acetate/hexanes) to afford the title compound. ¹H NMR (600 MHz, CDCl₃) δ 8.99 (d, 1H); 8.58 (d, 1H); 8.19 (d, 1H); 7.90 (d, 1H); 7.97 (s, 1H); 7.72 (dd, 1H); 7.56 (d, 1H); 7.32 (d, 1H); 7.22 (d, 1H); 5.01 (q, 1H); 3.97 (s, 3H); 1.44 (d, 3H); 0.90 (s, 9H); 0.06 (s, 3H); -0.02 (s, 3H). LRMS (APCI) calc'd for (C₂₆H₃₂N₃O₂Si) [M+H]+, 446.2; found, 446.2.

### Step 6: 7-[(1R)-1-hydroxyethyl]-3-(1-methyl-1H-pyrazol-4-yl)-5H-benzo[4,5]cyclohepta[1,2-b]pyridin-5-one and 7-[(1S)-1-hydroxyethyl]-3-(1-methyl-1H-pyrazol-4-yl)-5H-benzo[4,5]cyclohepta[1,2-b]pyridin-5-one

7-(1-{[*tert*-butyl(dimethyl)silyl]oxy}ethyl)-3-(1-methyl-1*H*-pyrazol-4-yl)-5*H-*benzo[4,5]cyclohepta[1,2-*b*]pyridin-5-one (51 mg, 0.114 mmol) was dissolved in 2 mL of tetrahydrofuran. Tetrabutylammonium fluoride (0.14 mL of 1.0M in THF, 0.14 mmol) was added and the mixture was stirred at room temperature for 1 hour. The reaction was diluted with ethyl acetate and brine and washed with brine twice. The organic layer was dried over magnesium sulfate, filtered and concentrated *in vacuo.* Purification via reverse phase HPLC (10-70% acetonitrile/water gradient, 0.05% trifluoroacetic acid modifier) afforded the title compound. The two enantiomers were separated via preparative chiral HPLC (AS column, 18% ethanol/heptane isocratic). Absolute stereochemistry was determined via formation of the Mosher's esters.
7-[(1*R*)-1-hydroxyethyl]-3-(1-methyl-1*H*-pyrazol-4-yl)-5*H*-benzo[4,5]cyclohepta[1,2-*b*]pyridin-5-one. ¹H NMR (600 MHz, CDCl₃) δ 8.98 (d, 1H); 8.55 (d, 1H); 8.24 (d, 1H); 7.89 (d, 1H); 7.80 (s, 1H); 7.74 (dd, 1H); 7.58 (d, 1H); 7.34 (d, 1H); 7.22 (d, 1H); 5.06 (q, 1H); 3.98 (s, 3H); 1.55 (s, 3H). Hydroxyl proton was not observed. LRMS (APCI) calc'd for (C₂₀H₁₈N₃O₂) [M+H]+, 332.1; found 332.1. τ_{R}: 18.9 min (analytical chiral HPLC, AS column, 0.46 cm x 25 cm, 18% ethanol/heptane, isochratic, flow rate = 0.75 mL/min).
7-[(1*S*)-1-hydroxyethyl]-3-(1-methyl-1*H*-pyrazol-4-yl)*-*5*H*-benzo[4,5]cyclohepta[1,2-*b*]pyridin-5-one. ¹H NMR and LRMS data matched 7-[(1*R*)-1-hydroxyethyl]-3-(1-methyl-1*H*-pyrazol-4-yl)-5*H-*benzo[4,5]cyclohepta[1,2-*b*]pyridin-5-one. τ_{R}: 21.5 min (analytical chiral HPLC, AS column, 0.46 cm x 25 cm, 18% ethanol/heptane, isochratic, flow rate = 0.75 mL/min).

### Example 22

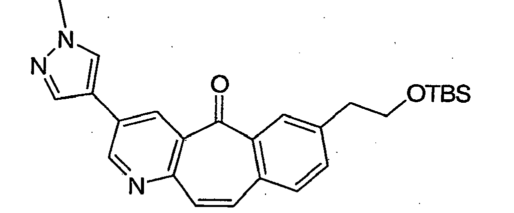

### 7-(2-{[tert-butyl(dimethyl)silyl]oxy}ethyl)-3-(1-methyl-1H-pyrazol-4-yl)-5H-benzo[4,5]cyclohepta[1,2-b]pyridin-5-one (Compound 47)

A test tube fitted with a teflon septum was charged with 7-(2-{[*tert*-butyl(dimethyl)silyl]oxy}ethyl)-3-chloro-5*H*-benzo[4,5]cyclohepta[1,2-*b*]pyridin-5-one (9 mg, 0.023 mmol), PdCl₂(PPh₃)₂ (2 mg, 0.002 mmol), 1-methyl-4-(4,4,5,5-tetramethyl-1,3,2-dioxaborolan-2-yl)-1*H*-pyrazole (14 mg, 0.068 mmol), and sodium carbonate (7 mg, 0.068 mmol). The tube was evacuated and backfilled with argon three times. Fully degassed dioxane (0.5 mL) was added and the mixture was stirred at 100°C overnight. The solution was diluted with ethyl acetate, washed with water and brine, dried over magnesium sulfate, concentrated *in vacuo,* and purified via flash chromatography (silica, 20-100% ethyl acetate/hexanes) to afford the title compound. LRMS (APCI) calc'd for (C₂₆H₃₂N₃O₂Si) [M+H]+, 446.2; found 446.2.

### Example 23

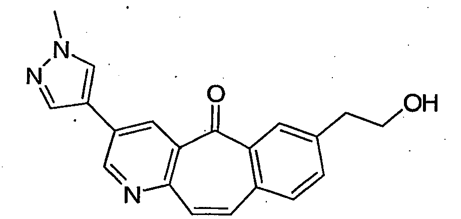

### 7-(2-hydroxyethyl)-3-(1-methyl-1H-pyrazol-4-yl)-5H-benzo[4,5]cyclohepta[1,2-b]pyridin-5-one (Compound 48)

7-(2-{[*tert*-butyl(dimethyl)silyl]oxy}ethyl)-3-(1-methyl-1*H*-pyrazol-4-yl)-5*H*-benzo[4,5]cyclohepta[1,2-*b*]pyridin-5-one (5 mg, 0.011 mmol) was dissolved in 0.5 mL of tetrahydrofuran. Tetrabutylammonium fluoride (0.013 mL of 1.0M in THF, 0.013 mmol) was added and the mixture was stirred at room temperature for 1 hour. The reaction was diluted with ethyl acetate and brine and washed with brine twice. The organic layer was dried over magnesium sulfate, filtered and concentrated *in vacuo.* Purification via reverse phase HPLC (10-70% acetonitrile/water gradient, 0.05% trifluoroacetic acid modifier) afforded the title compound. ¹H NMR (600 MHz, CDCl₃) δ 9.01 (d, 1H); 8.58 (s, 1H); 8.16 (d, 1H); 7.92 (s, 1H); 7.81 (s, 1H); 7.59 (dd, 1H); 7.56 (d, 1H); 7.35 (d, 1H); 7.24 (d, 1H); 3.99 (s, 3H); 3.96 (t, 2H); 3.03 (t, 2H). Hydroxyl proton was not observed. LRMS (APCI) calc'd for (C₂₀H₁₈N₃O₂) [M+H]+, 332.1; found 332.1.

### Example 24

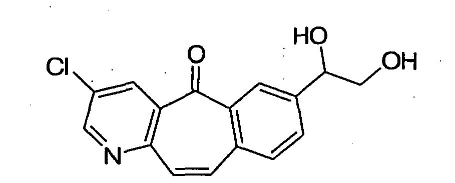

### 3-chloro-7-(1,2-dihydromethyl)-5H-benzo[4,5]cyclohepta[1,2-b]pyridin-5-one (Compound 49)

3-chloro-7-vinyl-5*H*-benzo[4,5]cyclohepta[1,2-*b*]pyridin-5-one (0.10 g, 0.37 mmol) was dissolved in 4 mL of tetrahydrofuran and 2 mL water. 4-Methylmorpholine *N*-oxide (0.105 mL of a 50% w/w aqueous solution, 0.45 mmol) was added followed by osmium tetroxide (0.24 mL of a 4% w/w aqueous solution, 0.037 mmol). The resulting mixture was stirred at room temperature for 3 hours at which time it was quenched via the addition of a 10% w/w aqueous sodium thiosulfate solution and stirred for 10 minutes. The mixture was extracted with ethyl acetate two times. The combined organics were dried over magnesium sulfate, filtered, concentrated *in vacuo,* and purified via flash chromatography (silica, 20-100% ethyl acetate/hexanes) to afford the title compound. ¹H NMR (600 MHz, CDCl₃) δ 8.80 (d, 1H); 8.49 (d, 1H); 8.24 (d, 1H); 7.76 (dd, 1H); 7.60 (d, 1H); 7.32 (d, 1H); 7.25 (d, 1H); 4.99 (dd, 1H); 3.87 (dd, 1H); 3.70 (dd, 1H). Hydroxyl protons were not observed. LRMS (APCI) calc'd for (C₁₆H₁₃ClNO₃) [M+H]+, 302.1; found 302.1.

### Example 25

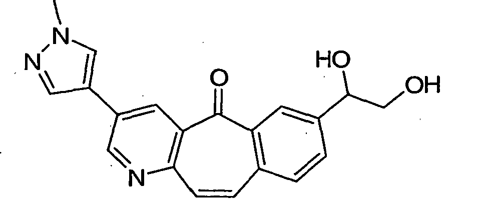

### 7-(1,2-dihydroxyethyl)-3-(1-methyl-1H-pyrazol-4-yl)-5H-benzo[4,5]cyclohepta[1,2-b]pyridin-5-one (Compound 50)

A test tube fitted with a teflon septum was charged with 3-chloro-7-(1,2-dihydroxyethyl)-5*H-*benzo[4,5]cyclohepta[1,2-*b*]pyridin-5-one (9 mg, 0.03 mmol), 1-methyl-4-(4,4,5,5-tetramethyl-1,3,2-dioxaborolan-2-yl)-1*H*-pyrazole (12 mg, 0.060mmol), Pd₂(dba)₃ (1 mg, 0.001 mmol), (tBu₃)PBF₄ (1 mg, 0.003 mmol) and potassium fluoride (6 mg, 0.098 mmol). The tube was evacuated and backfilled with argon three times. Fully degassed DMF (0.9mL) was added and the reaction was heated in a microwave at 180°C for 30 min. The reaction was poured into an ethyl acetate/brine mixture and washed twice with brine. The organic layer was dried over magnesium sulfate, filtered, concentrated *in vacuo,* and purified via reverse phase HPLC (10-70% acetonitrile/water gradient, 0.05% trifluoroacetic acid modifier) to afford the title compound. ¹H NMR (600 MHz, CDCl₃) δ 9.00 (d, 1H); 8.58 (d, 1H); 8.27 (s, 1H); 7.91 (s, 1H); 7.81 (s, 1H); 7.75 (d, 1H); 7.61 (d, 1H); 7.38 (d, 1H); 7.24 (d, 1H); 5.0 (dd, 1H); 3.99 (s, 3H); 3.87 (dd, 1H); 3.72 (d, 1H). LRMS (APCI) calc'd for (C₂₀H₁₈N₃O₃) [M+H]+, 348.1; found 348.1.

### Example 26

### 3-chloro-5-oxo-5H-benzo[4,5]cyclohepta[1,2-b]pyridine-7-carbaldehyde

3-chloro-7-(1,2-dihydroxyethyl)-5*H*-benzo[4,5]cyclohepta[1,2-*b*]pyridin-5-one (60 mg, 0.20 mmol) was dissolved in 1.8 mL of tetrahydrofuran and 0.9 mL water. Sodium periodate (51 mg, 0.24 mmol) was added and the reaction was stirred at room temperature for 1 hour. The reaction was then diluted with water and extracted with ethyl acetate three times. The combined organics were dried over magnesium sulfate, filtered, concentrated *in vacuo,* and purified via flash chromatography (silica, 10-100% ethyl acetate/hexanes) to afford the title compound. ¹H NMR (600 MHz, CDCl₃) δ 10.15 (s, 1H); 8.85 (d, 1H); 8.73 (d, 1H); 8.54 (d, 1H); 8.20 (dd, 1H); 7.75 (d, 1H); 7.47 (d, 1H); 7.31 (d, 1H).

### Example 27

### 3-chloro-7-(1-hydroxypronyl)-5H-benzo[4,5]cyclohepta[1,2-b]pyridin-5-one

3-chloro-5-oxo-5*H-*benzo[4,5]cyclohepta[1,2-*b*]pyridine-7-carbaldehyde (25 mg, 0.093 mmol) was dissolved in 4 mL of hot dichloromethane. The solution was cooled to room temperature and ethylmagnesium chloride (0.047 mL of 2.0 M in THF, 0.093 mmol) was added. The reaction was stirred at room temperature for 1.5 hours at which point it was quenched via the addition of saturated aqueous ammonium chloride. The mixture was extracted three times with dichloromethane. The combined organic layers were dried over magnesium sulfate, filtered, concentrated *in vacuo,* and purified via flash chromatography (5-60% ethyl acetate/hexanes) to afford the title compound. ¹H NMR (600 MHz, CDCl₃) δ 8.80 (d, 1 H); 8.52 (d, 1H); 8.21 (d, 1H); 7.73 (dd, 1H); 7.60 (d, 1H); 7.32 (d, 1H); 7.27 (d, 1H); 4.79 (t, 1H); 1.99 (s, 1H); 1.89-1.79 (m, 2H); 0.94 (t, 3H). LRMS (APCI) calc'd for (C₁₇H₁₅ClNO₂) [M+H]+, 300.1; found 300.1.

### Example 28

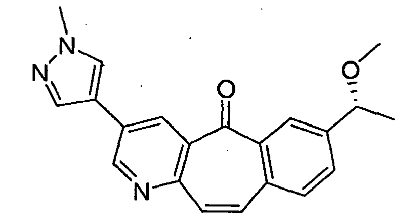

### 7-[(1R)-1-methoxyethyl]-3-(1-methyl-1H-pyrazol-4-yl)-5H-benzo[4,5]cyclohepta[1,2-b]pyridin-5-one (Compound 51)

7-[(1*R*)-1-hydroxyethyl]-3-(1-methyl-1*H*-pyrazol-4-yl)-5*H*-benzo[4,5]cyclohepta[1,2-*b*]pyridin-5-one (7 mg, 0.02 mmol) was dissolved in 1 mL of tetrahydrofuran. Sodium hydride (10 mg of 60% dispersion in oil) was added and the reaction was stirred at room temperature for 2 hours. Methyl iodide (26 µL, 0.42 mmol) was added and the reaction was stirred an additional 3 hours. The reaction was then poured into a mixture of ethyl acetate and saturated aqueous ammonium chloride. The aqueous layer was extracted twice with ethyl acetate. The combined organics were dried over magnesium sulfate, filtered, concentrated *in vacuo,* and purified via reverse phase HPLC (10-100% acetonitrile/water gradient, 0.05% trifluoroacetic acid modifier) to afford the title compound. ¹H NMR (600 MHz, CDCl₃) δ 9.05 (d, 1H); 8.73 (s, 1H); 8.21 (d, 1H); 7.94 (s, 1H); 7.85 (s, 1H); 7.73 (dd, 1H); 7.65 (d, 1H); 7.51 (d, 1H); 7.34 (d, 1H); 4.47 (q, 1H); 4.01 (s, 3H); 3.27 (s, 3H); 1.46 (d, 3H). LRMS (APCI) calc'd for (C₂₁H₂₀N₃O₂) [M+H]+, 346.2; found 346.2.

### Example 29

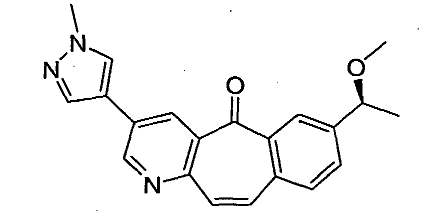

### 7-[(1S)-1-methoxyethyl]-3-(1-methyl-1H-pyrazol-4-yl)-5H-benzo[4,5]cyclohepta[1,2-b]pyridin-5-one (Compound 52)

7-[(1*S*)-1-hydroxyethyl]-3-(1-methyl-1*H*-pyrazol-4-yl)-5*H*-benzo[4,5]cyclohepta[1,2-*b*]pyridin-5-one (7 mg, 0.02 mmol) was dissolved in 1 mL of tetrahydrofuran. Sodium hydride (10 mg of 60% dispersion in oil) was added and the reaction was stirred at room temperature for 2 hours. Methyl iodide (26 µL, 0.42 mmol) was added and the reaction was stirred an additional 3 hours. The reaction was then poured into a mixture of ethyl acetate and saturated aqueous ammonium chloride. The aqueous layer was extracted twice with ethyl acetate. The combined organics were dried over magnesium sulfate, filtered, concentrated *in vacuo,* and purified via reverse phase HPLC (10-100% acetonitrile/water gradient, 0.05% trifluoroacetic acid modifier) to afford the title compound. ¹H NMR and LRMS data matched 7-[(1R)-1-methoxyethyl]-3-(1-methyl-1*H*-pyrazol-4-yl)-5*H*-benzo[4,5]cyclohepta[1,2-*b*]pyridin-5-one.

### Example 30

### tert-butyl 4-[2-(3-chloro-5-oxo-5H-benzo[4,5]cyclohepta[1,2-b]pyridin-7-yl)-2-hydroxyethyl]piperazine-1-carboxylate

3-chloro-7-oxiran-2-yl-5*H*-benzo[4,5]cyclohepta[1,2-*b*]pyridin-5-one (60 mg, 0.21 mmol) was suspended in 2.5 mL of methanol. *tert*-Butyl piperazine-1-carboxylate (98 mg, 0.53 mmol) was added and the reaction was heated to reflux for 8 hours. The resulting mixture was concentrated *in vacuo* and purified directly via flash chromatography (15-100% ethyl acetate/hexanes) to afford the title compound. ¹H NMR (600 MHz, CDCl₃) δ 9.79 (d, 1H); 8.49 (d, 1H); 8.22 (d, 1H); 7.77 (dd, 1H); 7.60 (d, 1H); 7.31 (d, 1H); 7.25 (d, 1H); 4.91 (dd, 1H); 3.50-3.45 (m, 4H); 2.72 (broad s, 2H); 2.63 (dd, 1H); 2.50 (dd, 1H); 2.44 (broad s, 2H); 1.46 (s, 9H). Hydroxyl proton was not observed. LRMS (APCI) calc'd for (C₂₅H₂₉ClN₃O₄) [M+H]+, 470.2; found 470.2.

### Example 31

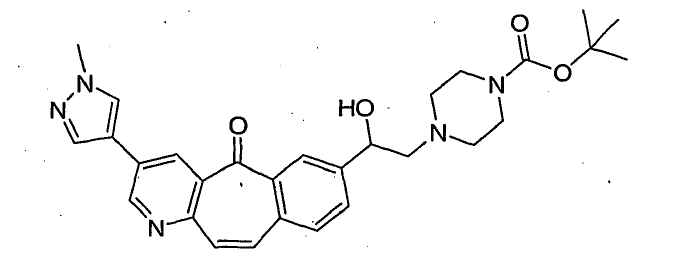

### tert-butyl4-{2-hydroxy-2-[3-(1-methyl-1H-pyrazol-4-yl)-5-oxo-5H-benzo[4,5]cyclohepta[1,2-b]pyridin-7-yl]ethyl}piperazine-1-carboxylate (Compound 53)

*tert*-Butyl 4-[2-(3-chloro-5-oxo-5*H*-benzo[4,5]cyclohepta[1,2-*b*]pyridin-7-yl)-2-hydroxyethyl]piperazine-1-carboxylate (65 mg, 0.138 mmol), 1-methyl-4-(4,4,5,5-tetramethyl-1,3,2-dioxaborolan-2-yl)-1*H-*pyrazole (58 mg, 0.28 mol), Pd₂(dba)₃ (6 mg, 0.007 mmol), (tBu₃)PBF₄ (4 mg, 0.014 mmol), and potassium fluoride (27 mg, 0.46 mmol) were combined in a sealed tube which was evacuated and backfilled with argon three times. Fully degassed DMF (1.5 mL) was added. The tube was placed in an oil bath at 115°C and stirred for 19 hours. The reaction mixture was poured into an ethyl acetate/brine mixture and extracted with ethyl acetate. Combined organics were dried over magnesium sulfate, filtered, concentrated *in vacuo,* and purified via reverse phase HPLC (10-42% acetonitrile/water gradient, 0.05% trifluoroacetic acid modifier) to afford the title compound. ¹H NMR (600 MHz, CDCl₃) δ 9.00 (d, 1H); 8.55 (d, 1H); 8.23 (d, 1H); 7.91 (s, 1H); 7.80 (s, 1H); 7.76 (dd, 1H); 7.60 (d, 1H); 7.34 (d, 1H); 7.23 (d, 1H); 4.95 (d, 1H); 3.98 (s, 3H); 3.56-3.51 (m, 4H); 2.78-2.53 (m, 6 H); 1.46 (s, 9H). Hydroxyl proton was not observed. LRMS (APCI) calc'd for (C₂₉H₃₄N₅O₄) [M+H]+, 516.3; found 516.3.

### Example 32

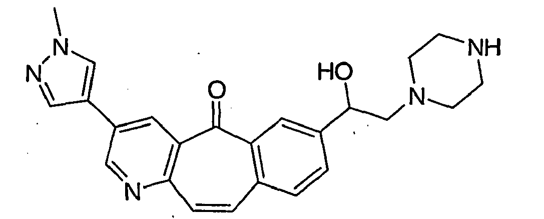

### 7-(1-hydroxy-2-piperazin-1-ylethyl)-3-(1-methyl-1H-pyrazol-4-yl)-5H-benzo[4,5]cyclohepta[1,2-b]pyridin-5-one (Compound 54)

*tert*-butyl 4-{2-hydroxy-2-[3-(1-methyl-1*H*-pyrazol-4-yl)-5-oxo-5*H*-benzo[4,5]cyclohepta[1,2-*b*]pyridin-7-yl]ethyl}piperazine-1-carboxylate (30 mg, 0.058 mmol) was dissolved in 0.5 mL of dichloromethane. Trifluroacetic acid (53 µL, 0.53 mmol) was added and the reaction was stirred at room temperature for 8 hours. The resulting mixture was concentrated *in vacuo* and directly purified via reverse phase HPLC (10-100% acetonitrile/water gradient, 0.05% trifluoroacetic acid modifier) to afford the title compound as the TFA salt. ¹H NMR (600 MHz, CD₃OD) δ 9.11 (d, 1H); 8.67 (d, 1H); 8.31 (d, 1H); 8.25 (s, 1H); 8.05 (d, 1H); 7.83 (dd, 1H); 7.75 (d; 1H); 7.39 (d, 1H); 7.32 (d, 1H); 5.12 (dd, 1H); 3.97 (s, 3H); 3.39-3.34 (m, 4H); 3.18-3.16 (m, 4H); 3.04-2.96 (m, 2H). Hydroxyl and amine protons were not observed. LRMS (APCI) calc'd for (C₂₄H₂₆N₅O₂) [M+H]+, 416.2; found 416.2.

## Claims

1. A compound of Formula I: 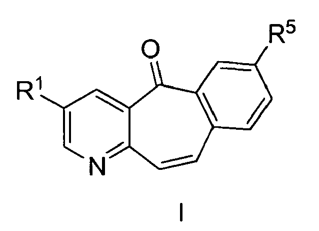 or a pharmaceutically acceptable salt or stereoisomer thereof, wherein
a is independently 0 or 1;
b is independently 0 or 1;
m is independently 0, 1, or 2;
R¹ is selected from aryl, heterocyclyl and NR¹⁰R¹¹; said aryl and heterocyclyl group optionally substituted with one to five substituents, each substituent independently selected from R⁸;
R5 is selected from C₁₋₆alkyl, C₂₋₆ alkenyl, OH, -O-C₁₋₆alkyl, - O-C(=O)C₁₋₆ alkyl, -O-aryl, S(O)ₘR^{a}, -C(=O)NR¹⁰R¹¹, -NHS(O)₂NR¹⁰R¹¹ and NR¹⁰R¹¹, each alkyl, alkenyl and aryl optionally substituted with one to five substituents, each substituent independently selected from R⁸;
R⁸ independently is: (C=O)ₐO_{b}C₁-C₁₀ alkyl, (C=O)ₐO_{b}aryl, C₂-C₁₀ alkenyl, C₂-C₁₀ alkynyl, (C=O)ₐO_{b} heterocyclyl, CO₂H, halo, CN, OH, O_{b}C₁-C₆ perfluoroalkyl, Oₐ(C=O)_{b}NR¹⁰R¹¹, S(O)ₘR^{a}, S(O)₂NR¹⁰R¹¹, OS(=O)R^{a}, oxo, CHO, (N=O)R¹⁰R¹¹, or (C=O)ₐO_{b}C₃-C₈ cycloalkyl,
said alkyl, aryl, alkenyl, alkynyl, heterocyclyl, and cycloalkyl optionally substituted with one, two or three substituents selected from R⁹;
R9 is independently selected from: (C=O)ₐO_{b}(C₁-C₁₀)alkyl, O_{b}(C₁-C₃)perfluoroalkyl, oxo, OH, halo, CN, (C₂-C₁₀)alkenyl, (C₂-C₁₀)alkynyl, (C=O)ₐO_{b}(C₃-C₆)cycloalkyl, (C=O)ₐO_{b}(C₀-C₆)alkylene-aryl, (C=O)ₐO_{b}(C₀-C₆)alkylene-heterocyclyl, (C=O)ₐO_{b}(C₀-C₆)alkylene-N(Rb)₂, C(O)R^{a}, (C₀-C₆)alkylene-CO₂R^{a}, C(O)H, (C₀-C₆)alkylene-CO₂H, C(O)N(Rb)₂, S(O)ₘR^{a}, and S(O)₂NR¹⁰R¹¹;
said alkyl, alkenyl, alkynyl, cycloalkyl, aryl, and heterocyclyl is optionally substituted with one, two or three substituents selected from R^{b}, OH, (C₁-C₆)alkoxy, halogen, CO₂H, CN, O(C=O)C₁-C₆ alkyl, oxo, and N(R^{b})₂;
R¹⁰ and R¹¹ are independently selected from: H, (C=O)O_{b}C₁-C₁₀ alkyl, (C=O)O_{b}C₃-C₈ cycloalkyl, (C=O)O_{b}aryl, (C=O)O_{b}heterocyclyl, C₁-C₁₀ alkyl, aryl, C₂-C₁₀ alkenyl, C₂-C₁₀ alkynyl, heterocyclyl, C₃-C₈ cycloalkyl, SO₂R^{a}, and (C=O)NR^{b}₂,
said alkyl, cycloalkyl, aryl, heterocylyl, alkenyl, and alkynyl is optionally substituted with one, two or three substituents selected from R⁸, or
R¹⁰ and R¹¹ can be taken together with the nitrogen to which they are attached to form a monocyclic or bicyclic heterocycle with 5-7 members in each ring and optionally containing, in addition to the nitrogen, one or two additional heteroatoms selected from N, O and S, said monocyclic or bicyclic heterocycle optionally substituted with one, two or three substituents selected from R⁹;
R^{a} is independently selected from: (C₁-C₆)alkyl, (C₂-C₆)alkenyl, (C₃-C₆)cycloalkyl, aryl, -(C₁-C₆)alkylenearyl, heterocyclyl and -(C₁-C₆)alkyleneheterocyclyl; and
R^{b} is independently selected from: H, (C₁-C₆)alkyl, aryl, -(C₁-C₆)alkylenearyl, heterocyclyl, - (C₁-C₆)alkyleneheterocyclyl, (C₃-C₆)cycloalkyl, (C=O)OC₁-C₆ alkyl, (C=O)C₁-C₆ alkyl or S(O)₂R^{a}.

2. A compound of claim 1 selected from:
3-phenyl-7-vinyl-5*H*-benzo[4,5]cyclohepta[1,2-*b*]pyridin-5-one;
7-ethyl-3-phenyl-5*H*-benzo[4,5]cyclohepta[1,2-*b*]pyridin-5-one;
7-[(2,4-dimethoxybenzyl)amino]-3-phenyl-5*H*-benzo[4,5]cyclohepta[1,2-*b*]pyridin-5-one;
7-amino-3-phenyl-5*H*-benzo[4,5]cyclohepta[1,2-*b*]pyridin-5-one;
2-hydroxy-*N*-(5-oxo-3-phenyl-5*H*-benzo[4,5]cyclohepta[1,2-*b*]pyridin-7-yl)propanamide;
*N*-methyl-5-oxo-3-phenyl-5*H*-benzo[4,5]cyclohepta[1*,2-b*]pyridine-7-carboxamide;
7-isobutyl-3-phenyl-5*H*-benzo[4,5]cyclohepta[1,2-*b*]pyridin-5-one;
*N*-(5-oxo-3-phenyl-5*H*-benzo[4,5]cyclohepta[1,2-*b*]pyridin-7-yl)methanesulfonamide;
*N*-[5-oxo-3-(3-thienyl)-5H-benzo[4,5]cyclohepta[1,2-b]pyridin-7-yl]methanesulfonamide;
7-amino-3-(1-methyl-1*H*-pyrazol-4-yl)-5*H*-benzo[4,5]cyclohepta[1,2-*b*]pyridin-5-one;
7-(isopropylamino)-3-(1-methyl-1*H*-pyrazol-4-yl)-5*H*-benzo[4,5]cyclohepta[1,2-*b*]pyridin-5-one;
*N*-[(2*R*)-1,4-dioxan-2-ylmethyl]-*N*-methyl-*N'*-[3-(1-methyl-1*H*-pyrazol-4-yl)-5-oxo-5*H-*benzo[4,5]cyclohepta[1,2-*b*]pyridin-7-yl]sulfamide;
*N*-[(2*S*)-1,4-dioxan-2-ylmethyl]-*N*-methyl-*N*'-[3-(1-methyl-1*H*-pyrazol-4-yl)-5-oxo-5*H-*benzo[4,5]cyclohepta[1,2-*b*]pyridin-7-yl]sulfamide;
*N*-[1,4-dioxan-2-ylmethyl]-*N*-methyl-*N*-[3-(1-methyl-1*H*-pyrazol-4-yl)-5-oxo-5*H-*benzo[4,5]cyclohepta[1,2-*b*]pyridin-7-yl]sulfamide racemic;
*N*-methyl-*N*-[3-(1-methyl-1*H*-pyrazol-4-yl)-5-oxo-5*H*-benzo[4,5]cyclohepta[1,2-*b*]pyridin-7-yl]-*N*-(tetrahydrofuran-3-yl)sulfamide;
*N*-methyl-*N'*-[3-(1-methyl-1*H*-pyrazol-4-yl)-5-oxo-5*H*-benzo[4,5]cyclohepta[1,2-*b*]pyridin-7-yl]-*N*-({3R}-tetrahydrofuran-3-yl)sulfamide;
*N*-methyl-*N'*-[3-(1-methyl-1*H*-pyrazol-4-yl)-5-oxo-5*H*-benzo[4,5]cyclohepta[1,2-*b*]pyridin-7-yl]-*N*-({3S}-tetrahydrofuran-3-yl)sulfamide;
*N-(*5-oxo-3-pyridin-4-yl-5*H*-benzo[4,5]cyclohepta[1,2-*b*]pyridin-7-yl)methanesulfonamide;
*N*-[5-oxo-3-(1*H*-pyrazol-3-yl)-5*H*-benzo[4,5]cyclohepta[1,2-*b*]pyridin-7-yl]methanesulfonamide;
*N*-[5-oxo-3-(1,3-thiazol-4-yl)-5*H*-benzo[4,5]cyclohepta[1,2-*b*]pyridin-7-yl]methanesulfonamide;
*N*-[3-(1-methyl-1*H*-pyrazol-4-yl)-5-oxo-5*H*-benzo[4,5]cyclohepta[1,2-*b*]pyridin-7-yl]methanesulfonamide;
*N*-[5-oxo-3-(1*H*-pyrazol-4-yl)-5*H*-benzo[4,5]cyclohepta[1,2-*b*]pyridin-7-yl]methanesulfonamide;
*N-*(3-{1-[2-(dimethylamino)ethyl]-1*H-*pyrazol-4-yl}-5-oxo-5*H-*benzo[4,5]cyclohepta[1,2-*b*]pyridin-7-yl)methanesulfonamide;
*N-*{3-[1-(2-morpholin-4-yl-2-oxoethyl)-1*H-*pyrazol-4-yl]-5-oxo-5*H-*benzo[4,5]cyclohepta[1,2-*b*]pyridin-7-yl}methanesulfonamide;
*N*-(4-{7-[(methylsulfonyl)amino]-5-oxo-5*H*-benzo[4,5]cyclohepta[1,2-*b*]pyridin-3-yl}phenyl)methanesulfonamide;
*N*-[3-(1-cyclopentyl-1*H-*pyrazol-4-yl)-5-oxo-5*H*-benzo[4,5]cyclohepta[1,2-*b*]pyridin-7-yl]methanesulfonamide;
*N-*{3-[1-(3,3-dimethyl-2-oxobutyl)-1*H*-pyrazol-4-yl]-5-oxo-5*H-*benzo[4,5]cyclohepta[1,2-*b*]pyridin-7-yl}methanesulfonamide;
*N*-[2-(1-methylpyrrolidin-2-yl)ethyl]-3-{7-[(methylsulfonyl)amino]-5-oxo-5*H-*benzo[4,5]cyclohepta[1,2-*b*]pyridin-3-yl}benzamide;
*N*,*N*-dimethyl-*N'*-[3-(1-methyl-1*H*-pyrazol-4-yl)-5-oxo-5*H*-benzo[4,5]cyclohepta[1,2-*b*]pyridin-7-yl]sulfamide;
7-(5-methyl-1,1-dioxido-1,2,5-thiadiazolidin-2-yl)-3-(1-methyl-1*H-*pyrazol-4-yl)-5*H-*benzo[4,5]cyclohepta[1,2-*b*]pyridin-5-one;
7-[(2,4-dimethoxybenzyl)amino]-3-(3-thienyl)-5*H*-benzo[4,5]cyclohepta[1,2-*b*]pyridin-5-one;
7-[(2,4-dimethoxybenzyl)amino]-3-(1*H-*pyrazol-3-yl)-5*H*-benzo[4,5]cyclohepta[1,2-*b*]pyridin-5-one;
N-methyl-N'-[3-(1-methyl-1H-pyrazol-4-yl)-5-oxo-5H-benzo[4,5]cyclohepta[1,2-b]pyridin-7-yl]-N-(tetrahydrofuran-3-yl)sulfamide;
7-[(imidazo[1,2-*a*]pyridin-3-ylmethyl)amino]-3-(1-methyl-1*H*-pyrazol-4-yl)-5*H-*benzo[4,5]cyclohepta[1,2-*b*]pyridin-5-one;
7-{[(1-methyl-5-oxopyrrolidin-2-yl)methyl]amino}-3-(1-methyl-1*H*-pyrazol-4-yl)-5*H-*benzo[4,5]cyclohepta[1,2-*b*]pyridin-5-one;
*N*-methyl-*N'*-[3-(1-methyl-1*H-*pyrazol-4-yl)-5-oxo-5*H-*benzo[4,5]cyclohepta[1,2-*b*]pyridin-7-yl]-*N*-(tetrahydro-2*H*-pyran-2-ylmethyl)sulfamide;
*N*-[3-(1-methyl-1*H*-pyrazol-4-yl)-5-oxo-5*H-*benzo[4,5]cyclohepta[1,2-*b*]pyridin-7-yl]-*N'-*(tetrahydrofuran-3-yl)sulfamide;
*N*-[3-(1-methyl-1*H-*pyrazol-4-yl)-5-oxo-5*H-*benzo[4,5]cyclohepta[1,2-*b*]pyridin-7-yl]morpholine-4-sulfonamide;
*N*-[3-(4-isopropylpiperazin-1-yl)-5-oxo-5*H-*benzo[4,5]cyclohepta[1,2-*b*]pyridin-7-yl]methanesulfonamide;
3-(4-isopropylpiperazin-1-yl)-7-phenyl-5H-benzo[4,5]cyclohepta[1,2-b]pyridin-5-one;
*N*-(3-morpholin-4-yl-5-oxo-5*H*-benzo[4,5]cyclohepta[1,2-*b*]pyridin-7-yl)methanesulfonamide;
*N*-(3-anilino-5-oxo-5*H*-benzo[4,5]cyclohepta[1,2-*b*]pyridin-7-yl)methanesulfonamide;
*N*-[3-(cyclohexylamino)-5-oxo-5*H*-benzo[4,5]cyclohepta[1,2-*b*]pyridin-7-yl]methanesulfonamide;
*N*-[5-oxo-3-(pyridin-4-ylamino)-5*H*-benzo[4,5]cyclohepta[1,2-*b*]pyridin-7-yl]methanesulfonamide;
*N*-(2,4-dimethoxybenzyl)-*N*-(5-oxo-3-phenyl-5*H*-benzo[4,5]cyclohepta[1,2-*b*]pyridin-7-yl)ethylenesulfonamide;
*N*-(5-oxo-3-phenyl-5*H*-benzo[4,5]cyclohepta[1,2-*b*]pyridin-7-yl)ethylenesulfonamide;
*N-(*5-oxo-3-phenyl-5*H*-benzo[4,5]cyclohepta[1,2-*b*]pyridin-7-yl)-2-pyrrolidin-1-ylethanesulfonamide;
dimethyl [3-(1-methyl-1H-pyrazol-4-yl)-5-oxo-5H-benzo[4,5]cyclohepta[1,2-b]pyridin-7-yl]amidophosphate;
7-[(1*R*)-1-hydroxyethyl]-3-(1-methyl-1*H*-pyrazol-4-yl)-5*H*-benzo[4,5]cyclohepta[1,2-*b*]pyridin-5-one;
7-[(1*S*)-1-hydroxyethyl]-3-(1-methyl-1*H*-pyrazol-4-yl)-5*H*-benzo[4,5]cyclohepta[1,2-*b*]pyridin-5-one;
7-(2-{[*tert*-butyl(dimethyl)silyl]oxy}ethyl)-3-(1-methyl-1*H*-pyrazol-4-yl)-5*H-*benzo[4,5]cyclohepta[1,2-*b*]pyridin-5-one;
7-(2-hydroxyethyl)-3-(1-methyl-1*H-*pyrazol-4-yl)-5*H*-benzo[4,5]cyclohepta[1,2-*b*]pyridin-5-one;
7-(1,2-dihydroxyethyl)-3-(1-methyl-1*H-*pyrazol-4-yl)-5*H*-benzo[4,5]cyclohepta[1,2-*b*]pyridin-5-one;
7-[(1*R*)-1-methoxyethyl]-3-(1-methyl-1*H*-pyrazol-4-yl)-5*H*-benzo[4,5]cyclohepta[1,2-*b*]pyridin-5-one;
7-[(1*S*)-1-methoxyethyl]-3-(1-methyl-1*H*-pyrazol-4-yl)-5*H*-benzo[4,5]cyclohepta[1,2-*b*]pyridin-5-one;
*tert*-butyl 4-[2-(3-chloro-5-oxo-5*H*-benzo[4,5]cyclohepta[1,2-*b*]pyridin-7-yl)-2-hydroxyethyl]piperazine-1-carboxylate;
*tert*-butyl 4-{2-hydroxy-2-[3-(1-methyl-1*H*-pyrazol-4-yl)-5-oxo-5*H*-benzo[4,5]cyclohepta[1,2-*b*]pyridin-7-yl]ethyl} piperazine-1-carboxylate;
7-(1-hydroxy-2-piperazin-1-ylethyl)-3-(1-methyl-1*H*-pyrazol-4-yl)-5*H-*benzo[4,5]cyclohepta[1,2-*b*]pyridin-5-one;
or a pharmaceutically acceptable salt or stereoisomer thereof.

3. A compound of claim 1 which is: 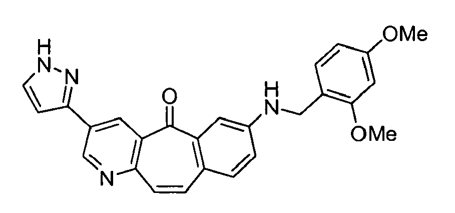 7-[(2,4-dimethoxybenzyl)amino]-3-(1*H-*pyrazol-3-yl)-5*H*-benzo[4,5]cyclohepta[1,2-*b*]pyridin-5-one
or a pharmaceutically acceptable salt thereof.

4. A compound of claim 1 which is: 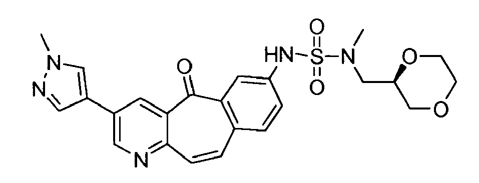 *N*-[(2*R*)-1,4-dioxan-2-ylmethyl]-*N-*methyl-*N'*-[3-(1-methyl-1*H*-pyrazol-4-yl)-5-oxo-5*H-*benzo[4,5]cyclohepta[1,2-*b*]pyridin-7-yl]sulfamide
or a pharmaceutically acceptable salt or stereoisomer thereof.

5. A compound of claim 1 which is: 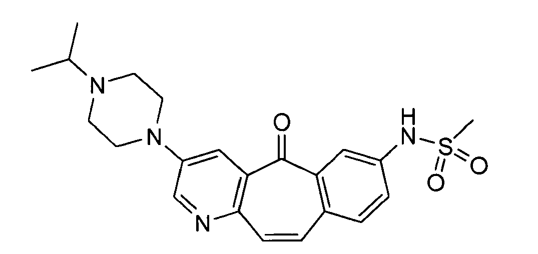 *N*-[3-(4-isopropylpiperazin-1-yl)-5-oxo-5*H*-benzo[4,5]cyclohepta[1,2-*b*]pyridin-7-yl]methanesulfonamide
or a pharmaceutically acceptable salt thereof.

6. A compound of claim 1 which is: 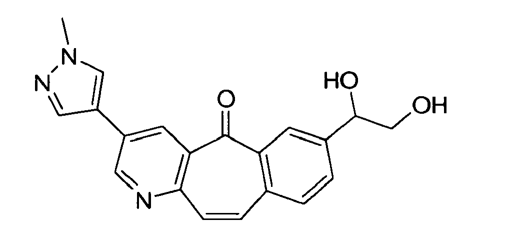 7-(1,2-dihydroxyethyl)-3-(1-methyl-1*H*-pyrazol-4-yl)-5*H*-benzo[4,5]cyclohepta[1,2-*b*]pyridin-5-one
or a pharmaceutically acceptable salt or stereoisomer thereof.

7. A pharmaceutical composition that is comprised of a compound in accordance with any previous claim, or a pharmaceutically acceptable salt or stereoisomer thereof and a pharmaceutically acceptable carrier.

8. A compound according to any one of Claims 1 to 6, or a pharmaceutically acceptable salt or steroisomer thereof for use in therapy.

9. A compound according to any one of Claims 1 to 6, or a pharmaceutically acceptable salt or stereoisomer thereof for use in the treatment or prevention of cancer.

10. A compound according to any one of Claims 1 to 6, or a pharmaceutically acceptable salt or stereoisomer thereof for use in preventing or modulating metastasis of cancer.

11. A compound for use according to Claim 9 wherein the cancer is selected from ovarian cancer, childhood hepatocellular carcinoma, metastatic head and neck squamous cell carcinomas, gastric cancer, breast cancer, colorectal cancer, cervical cancer, lung cancer, nasopharyngeal cancer, pancreatic cancer, glioblastoma and sarcomas.

12. A compound for use according to Claim 9 wherein the cancer is selected from cancers of the brain, genitourinary tract, lymphatic system, stomach, larynx and lung.

13. A compound for use according to Claim 9 wherein the cancer is selected from histiocytic lymphoma, lung adenocarcinoma, small cell lung cancers, pancreatic cancer, liver cancer, gastric cancer, colon cancer, multiple myeloma, glioblastomas and breast carcinoma.

14. The use of a compound according to any one of claims 1 to 8, or a pharmaceutically acceptable salt or stereoisomer thereof for the preparation of a medicament for use according to any one of claims 9 to 13.

15. A combination of a compound of any one of claims 1 to 6, or a pharmaceutically acceptable salt or stereoisomer thereof and known therapeutic agents and anti-cancer agents.

## Patentansprüche

1. Eine Verbindung der Formel I: 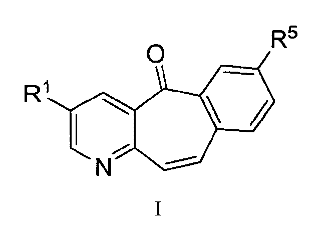 oder ein pharmazeutisch annehmbares Salz oder Stereoisomer davon, wobei
a unabhängig 0 oder 1 ist,
b unabhängig 0 oder 1 ist,
m unabhängig 0, 1 oder 2 ist,
R¹ ausgewählt ist aus Aryl, Heterocyclyl und NR¹⁰R¹¹, wobei die Aryl- und Heterocyclylgruppe gegebenenfalls substituiert ist mit einem bis fünf Substituenten, wobei jeder Substituent unabhängig ausgewählt ist aus R⁸,
R⁵ ausgewählt ist aus C₁₋₆-Alkyl, C₂₋₆-Alkenyl, OH, -O-C₁-₆-Alkyl, -O-C(=O)C₁₋₆-Alkyl, -O-Aryl, S(O)ₘR^{a}, -C(=O)NR¹⁰R¹¹, -NHS(O)₂NR¹⁰R¹¹ und NR¹⁰R¹¹, wobei jedes Alkyl, Alkenyl und Aryl gegebenenfalls substituiert ist mit einem bis fünf Substituenten, wobei jeder Substituent unabhängig ausgewählt ist aus R⁸,
R⁸ unabhängig ist: (C=O)ₐO_{b}C₁-C₁₀-Alkyl, (C=O)ₐO_{b}-Aryl, C₂-C₁₀-Alkenyl, C₂-C₁₀-Alkinyl, (C=O)ₐO_{b}-Heterocyclyl, CO₂H, Halogen, CN, OH, O_{b}C₁-C₆-Perfluoralkyl, Oₐ(C=O)_{b}NR¹⁰R¹¹, S(O)ₘR^{a}, S(O)₂NR¹⁰R¹¹, OS(=O)R^{a}, Oxo, CHO, (N=O)R¹⁰R¹¹ oder (C=O)ₐO_{b}C₃-C₈-Cycloalkyl,
wobei das Alkyl, Aryl, Alkenyl, Alkinyl, Heterocyclyl und Cycloalkyl gegebenenfalls substituiert sind mit einem, zwei oder drei Substituenten, ausgewählt aus R⁹,
R⁹ unabhängig ausgewählt ist aus: (C=O)ₐO_{b}(C₁-C₁₀)-Alkyl, O_{b}(C₁-C₃)-Perfluoralkyl, Oxo, OH, Halogen, CN, (C₂-C₁₀)-Alkenyl, (C₂-C₁₀)-Alkinyl, (C=O)ₐO_{b}(C₃-C₆)-Cycloalkyl, (C=O)ₐO_{b}(C₀-C₆)-Alkylenaryl, (C=O)ₐO_{b}(C₀-C₆)-Alkylenheterocyclyl, (C=O)ₐO_{b}(C₀-C₆)-Alkylen-N(R^{b})₂, C(O)R^{a}, (C₀-C₆)-Alkylen-CO₂R^{a}, C(O)H, (C₀-C₆)-Alkylen-CO₂H, C(O)N(R^{b})₂, S(O)ₘR^{a} und S(O)₂NR¹⁰R¹¹,
wobei das Alkyl, Alkenyl, Alkinyl, Cycloalkyl, Aryl und Heterocyclyl gegebenenfalls substituiert ist mit einem, zwei oder drei Substituenten, ausgewählt aus R^{b}, OH, (C₁-C₆)-Alkoxy, Halogen, CO₂H, CN, O(C=O)C₁-C₆-Alkyl, Oxo und N(R^{b})₂,
R¹⁰ und R¹¹ unabhängig ausgewählt sind aus: H, (C=O)O_{b}C₁-C₁₀-Alkyl, (C=O)O_{b}C₃-C₈-Cycloalkyl, (C=O)O_{b}-Aryl, (C=O)O_{b}-Heterocyclyl, C₁-C₁₀₋Alkyl, Aryl, C₂-C₁₀-Alkenyl, C₂-C₁₀-Alkinyl, Heterocyclyl, C₃-C₈-Cycloalkyl, SO₂R^{a} und (C=O)NR^{b}₂,
wobei das Alkyl, Cycloalkyl, Aryl, Heterocyclyl, Alkenyl und Alkinyl gegebenenfalls substituiert sind mit einem, zwei oder drei Substituenten, ausgewählt aus R⁸, oder
R¹⁰ und R¹¹ mit dem Stickstoff, an den sie gebunden sind, zusammengenommen werden können unter Bildung eines monocyclischen oder bicyclischen Heterocyclus mit 5-7 Gliedern in jedem Ring und der gegebenenfalls zusätzlich zum Stickstoff ein oder zwei zusätzliche Heteroatome, ausgewählt aus N, O und S, enthält, wobei der monocyclische oder bicyclische Heterocyclus gegebenenfalls substituiert ist mit einem, zwei oder drei Substituenten, ausgewählt aus R⁹,
R^{a} unabhängig ausgewählt ist aus: (C₁-C₆)-Alkyl, (C₂-C₆)-Alkenyl, (C₃-C₆)-Cycloalkyl, Aryl, -(C₁-C₆)-Alkylenaryl, Heterocyclyl und -(C₁-C₆)-Alkylenheterocyclyl, und
R^{b} unabhängig ausgewählt ist aus: H, (C₁-C₆)-Alkyl, Aryl, -(C₁-C₆)-Alkylenaryl, Heterocyclyl, -(C₁-C₆)-Alkylenheterocyclyl, (C₃-C₆)-Cycloalkyl, (C=O)OC₁-C₆-Alkyl, (C=O)C₁-C₆-Alkyl oder S(O)₂R^{a}.

2. Eine Verbindung nach Anspruch 1, ausgewählt aus:
3-Phenyl-7-vinyl-5*H*-benzo[4,5]cyclohepta[1,2-*b*]pyridin-5-on,
7-Ethyl-3-phenyl-5H-benzo[4,5]cyclohepta[1,2-b]pyridin-5-on,
7-[(2,4-Dimethoxybenzyl)amino]-3-phenyl-5*H*-benzo[4,5]cyclohepta[1,2-*b*]pyridin-5-on,
7-Amino-3-phenyl-5*H*-benzo[4,5]cyclohepta[1,2-*b*]pyridin-5-on,
2-Hydroxy-N-(5-oxo-3-phenyl-5*H-*benzo[4,5]cyclohepta[1,2-*b*]pyridin-7-yl)propanamid,
N-Methyl-5-oxo-3-phenyl-5*H-*benzo[4,5]cyclohepta[1,2-*b*]pyridin-7-carboxamid,
7-Isobutyl-3-phenyl-5*H-*benzo[4,5]cyclohepta[1,2-*b*]pyridin-5-on,
N-(5-Oxo-3-phenyl-5*H-*benzo[4,5]cyclohepta[1,2-b]pyridin-7-yl)methansulfonamid,
N-[5-Oxo-3-(3-thienyl)-5*H-*benzo[4,5]cyclohepta[1,2-b]pyridin-7-yl]methansulfonamid,
7-Amino-3-(1-methyl-1*H*-pyrazol-4-yl)-5*H*-benzo[4,5]cyclohepta[1,2-*b*]pyridin-5-on,
7-(Isopropylamino)-3-(1-methyl-1*H*-pyrazol-4-yl)-5*H*-benzo[4,5]cyclohepta[1,2-*b*]pyridin-5-on,
*N*-[(2*R*)-1,4-Dioxan-2-ylmethyl]-*N*-methyl-*N*'-[3-(1-methyl-1*H*-pyrazol-4-yl)-5-oxo-5*H-*benzo[4,5]cyclohepta[1,2-*b*]pyridin-7-yl]sulfamid,
*N*-[(2*S*-1,4-Dioxan-2-ylmethyl]-*N*-methyl-*N*'-[3-(1-methyl-1*H*-pyrazol-4-yl)-5-oxo-5*H-*benzo[4,5]cyclohepta[1,2-*b*]pyridin-7-yl]sulfamid,
*N*-[1,4-Dioxan-2-ylmethyl]-*N*-methyl-*N*'-[3-(1-methyl-1*H*-pyrazol-4-yl)-5-oxo-5*H-*benzo[4,5]cyclohepta[1,2-*b*]pyridin-7-yl]sulfamid, racemisch,
*N*-Methyl-*N*'-[3-(1-methyl-1*H*-pyrazol-4-yl)-5-oxo-5*H*-benzo[4,5]cyclohepta[1,2-*b*]pyridin-7-yl]-*N-*(tetrahydrofuran-3-yl)sulfamid,
*N*-Methyl-*N*'-[3-(1-methyl-1*H*-pyrazol-4-yl)-5-oxo-5*H*-benzo[4,5]cyclohepta[1,2-*b*]pyridin-7-yl]-*N-*({3R}-tetrahydrofuran-3-yl)sulfamid,
*N*-Methyl-*N*'-[3-(1-methyl-1*H-*pyrazol-4-yl)-5-oxo-5*H*-benzo[4,5]cyclohepta[1,2-*b*]pyridin-7-yl]-*N-*({3S}-tetrahydrofuran-3-yl)sulfamid,
*N*-(5-Oxo-3-pyridin-4-yl-5*H*-benzo[4,5]cyclohepta[1,2-*b*]pyridin-7-yl)methansulfonamid,
*N*-[5-Oxo-3-(1*H*-pyrazol-3-yl)-5*H*-benzo[4,5]cyclohepta[1,2-*b*]pyridin-7-yl]methansulfonamid,
*N*-[5-Oxo-3-(1,3-thiazol-4-yl)-5*H*-benzo[4,5]cyclohepta[1,2-*b*]pyridin-7-yl]methansulfonamid,
*N*-[3-(1-Methyl-1*H-*pyrazol-4-yl)-5-oxo-5*H*-benzo[4,5]cyclohepta[1,2-*b*]pyridin-7-yl]methansulfonamid,
*N*-[5-Oxo-3-(1*H*-pyrazol-4-yl)-5*H*-benzo[4,5]cyclohepta[1,2-*b*]pyridin-7-yl]methansulfonamid,
*N*-(3-{1-[2-(Dimethylamino)ethyl]-1*H-*pyrazol-4-yl}-5-oxo-5*H*-benzo[4,5]cyclohepta[1,2-*b*]pyridin-7-yl)methansulfonamid,
*N*-{3-[1-(2-Morpholin-4-yl-2-oxoethyl)-1*H*-pyrazol-4-yl]-5-oxo-5*H*-benzo[4,5]cyclohepta[1,2-*b*]pyridin-7-yl}methansulfonamid,
*N*-(4-{7-[(Methylsulfonyl)amino]-5-oxo-5*H*-benzo[4,5]cyclohepta[1,2-*b*]pyridin-3-yl}phenyl)methan-sulfonamid,
*N*-[3-(1-Cyclopentyl-1*H-*pyrazol-4-yl)-5-oxo-5*H*-benzo[4,5]cyclohepta[1,2-*b*]pyridin-7-yl]methan-sulfonamid,
*N*-{3-[1-(3,3-Dimethyl-2-oxobutyl)-1*H*-pyrazol-4-yl]-5-oxo-5*H*-benzo[4,5]cyclohepta[1,2-*b*]pyridin-7-yl}methansulfonamid,
*N*-[2-(1-Methylpyrrolidin-2-yl)ethyl]-3-{7-[(methylsulfonyl)amino]-5-oxo-5*H*-benzo[4,5]cyclohepta[1,2-*b*]pyridin-3-yl}benzamid,
*N*,*N*-Dimethyl-*N*'-[3-(1-methyl-1*H-*pyrazol-4-yl)-5-oxo-5*H*-benzo[4,5]cyclohepta[1,2-*b*]pyridin-7-yl]sulfamid,
7-(5-Methyl-1,1-dioxido-1,2,5-thiadiazolidin-2-yl)-3-(1-methyl-1*H*-pyrazol-4-yl)-5*H*-benzo[4,5]-cyclohepta[1,2-*b*]pyridin-5-on,
7-[(2,4-Dimethoxybenzyl)amino]-3-(3-thienyl)-5*H*-benzo[4,5]cyclohepta[1,2-*b*]pyridin-5-on,
7-[(2,4-Dimethoxybenzyl)amino]-3-(1*H*-pyrazol-3-yl)-5*H*-benzo[4,5]cyclohepta[1,2-*b*]pyridin-5-on,
*N*-Methyl-*N*'-[3-(1-methyl-1*H*-pyrazol-4-yl)-5-oxo-5*H*-benzo[4,5]cyclohepta[1,2-*b*]pyridin-7-yl]-*N-*(tetrahydrofuran-3-yl)sulfamid,
7-[(Imidazo[1,2-*a*]pyridin-3-ylmethyl)amino]-3-(1-methyl-1*H*-pyrazol-4-yl)-5*H*-benzo[4,5]cyclohepta[1,2-b]pyridin-5-on,
7-{[(1-Methyl-5-oxopyrrolidin-2-yl)methyl]amino}-3-(1-methyl-1*H*-pyrazol-4-yl)-5*H*-benzo[4,5]-cyclohepta[1,2-*b*]pyridin-5-on,
*N*-Methyl-*N*'-[3-(1-methyl-1*H*-pyrazol-4-yl)-5-oxo-5*H*-benzo[4,5]cyclohepta[1,2-*b*]pyridin-7-yl]-*N-*(tetrahydro-2*H*-pyran-2-ylmethyl)sulfamid,
*N*-[3-(1-Methyl-1*H*-pyrazol-4-yl)-5-oxo-5*H*-benzo[4,5]cyclohepta[1,2-*b*]pyridin-7-yl]-*N*'-(tetrahydrofuran-3-yl)sulfamid,
*N*-[3-(1-Methyl-1*H-*pyrazol-4-yl)-5-oxo-5*H*-benzo[4,5]cyclohepta[1,2-*b*]pyridin-7-yl]morpholin-4-sulfonamid,
*N*-[3-(4-Isopropylpiperazin-1-yl)-5-oxo-5*H*-benzo[4,5]cyclohepta[1,2-*b*]pyridin-7-yl]methansulfonamid,
3-(4-Isopropylpiperazin-1-yl)-7-phenyl-5*H*-benzo[4,5]cyclohepta[1,2-*b*]pyridin-5-on,
*N*-(3-Morpholin-4-yl-5-oxo-5*H*-benzo[4,5]cyclohepta[1,2-*b*]pyridin-7-yl)methansulfonamid,
*N*-(3-Anilino-5-oxo-5*H*-benzo[4,5]cyclohepta[1,2-*b*]pyridin-7-yl)methansulfonamid,
*N*-[3-(Cyclohexylamino)-5-oxo-5*H*-benzo[4,5]cyclohepta[1,2-*b*]pyridin-7-yl]methansulfonamid,
*N*-[5-Oxo-3-(pyridin-4-ylamino)-5*H*-benzo[4,5]cyclohepta[1,2-*b*]pyridin-7-yl]methansulfonamid,
*N*-(2,4-Dimethoxybenzyl)-*N*-(5-oxo-3-phenyl-5*H*-benzo[4,5]cyclohepta[1,2-*b*]pyridin-7-yl)-ethylensulfonamid,
*N*-(5-Oxo-3-phenyl-5*H*-benzo[4,5]cyclohepta[1,2-*b*]pyridin-7-yl)ethylensulfonamid,
*N*-(5-Oxo-3-phenyl-5*H*-benzo[4,5]cyclohepta[1,2-*b*]pyridin-7-yl)-2-pyrrolidin-1-ylethansulfonamid,
Dimethyl-[3-(1-methyl-1*H*-pyrazol-4-yl)-5-oxo-5*H*-benzo[4,5]cyclohepta[1,2-*b*]pyridin-7-yl]amidophosphat,
7-[(1*R*)-1-Hydroxyethyl]-3-(1-methyl-1*H*-pyrazol-4-yl)-5*H*-benzo[4,5]cyclohepta[1,2-*b*]pyridin-5-on,
7-[(1*S*)-1-Hydroxyethyl]-3-(1-methyl-1*H*-pyrazol-4-yl)-5*H*-benzo[4,5]cyclohepta[1,2-*b*]pyridin-5-on,
7-(2-{[*tert*-Butyl(dimethyl)silyl]oxy}ethyl)-3-(1-methyl-1*H-*pyrazol-4-yl)-5*H*-benzo[4,5]cyclohepta[1,2-*b*]pyridin-5-on,
7-(2-Hydroxyethyl)-3-(1-methyl-1*H*-pyrazol-4-yl)-5*H*-benzo[4,5]cyclohepta[1,2-*b*]pyridin-5-on,
7-(1,2-Dihydroxyethyl)-3-(1-methyl-1*H*-pyrazol-4-yl)-5*H*-benzo[4,5]cyclohepta[1,2-*b*]pyridin-5-on,
7-[(1*R*)-1-Methoxyethyl]-3-(1-methyl-1*H*-pyrazol-4-yl)-5*H*-benzo[4,5]cyclohepta[1,2-*b*]pyridin-5-on,
7-[(1*S*)-1-Methoxyethyl]-3-(1-methyl-1*H*-pyrazol-4-yl)-5*H*-benzo[4,5]cyclohepta[1,2-*b*]pyridin-5-on,
*tert*-Butyl-4-[2-(3-chlor-5-oxo-5H-benzo[4,5]cyclohepta[1,2-b]pyridin-7-yl)-2-hydroxyethyl]piperazin-1-carboxylat,
*tert*-Butyl-4-(2-hydroxy-2-[3-(1-methyl-1*H*-pyrazol-4-yl)-5-oxo-5*H*-benzo[4,5]cyclohepta[1,2-*b*]pyridin-7-yl]ethyl)piperazin-1-carboxylat,
7-(1-Hydroxy-2-piperazin-1-ylethyl)-3-(1-methyl-1*H*-pyrazol-4-yl)-5*H*-benzo[4,5]cyclohepta[1,2-*b*]-pyridin-5-on,
oder ein pharmazeutisch annehmbares Salz oder Stereoisomer davon.

3. Eine Verbindung nach Anspruch 1, die ist: 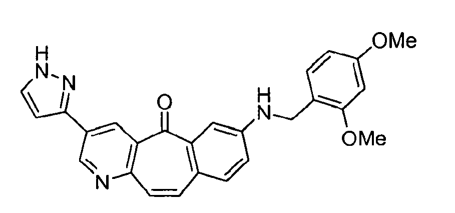 7-[(2,4-Dimethoxybenzyl)amino]-3-(1*H*-pyrazol-3 -yl)-5*H*-benzo[4,5]cyclohepta[1,2-*b*]pyridin-5-on
oder ein pharmazeutisch annehmbares Salz davon.

4. Eine Verbindung nach Anspruch 1, die ist: 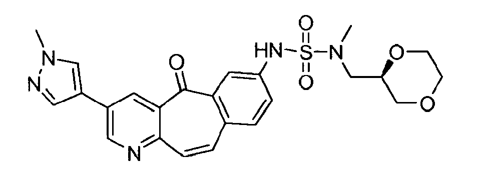 *N*-[(2*R*)-1,4-Dioxan-2-ylmethyl]-*N*-methyl-*N*'-[3-(1-methyl-1*H*-pyrazol-4-yl)-5-oxo-5*H-*benzo[4,5]cyclohepta[1,2-*b*]pyridin-7-yl]sulfamid
oder ein pharmazeutisch annehmbares Salz oder Stereoisomer davon.

5. Eine Verbindung nach Anspruch 1, die ist: 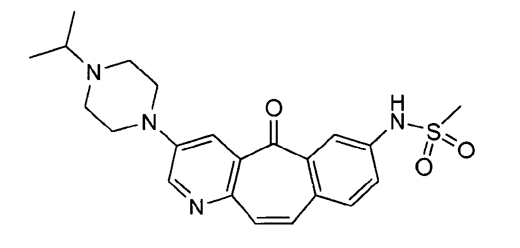 *N*-[3-(4-Isopropylpiperazin-1-yl)-5-oxo-5*H*-benzo[4,5]cyclohepta[1,2-*b*]pyridin-7-yl]methansulfonamid
oder ein pharmazeutisch annehmbares Salz davon.

6. Eine Verbindung nach Anspruch 1, die ist: 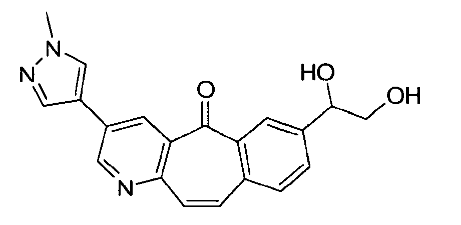 7-(1,2-Dihydroxyethyl)-3-(1-methyl-1*H*-pyrazol-4-yl)-5*H*-benzo[4,5]cyclohepta[1,2-*b*]pyridin-5-on
oder ein pharmazeutisch annehmbares Salz oder Stereoisomer davon.

7. Eine pharmazeutische Zusammensetzung, die aus einer Verbindung gemäß einem vorhergehenden Anspruch oder einem pharmazeutisch annehmbaren Salz oder Stereoisomer davon und einem pharmazeutisch annehmbaren Träger besteht.

8. Eine Verbindung gemäß einem der Ansprüche 1 bis 6 oder ein pharmazeutisch annehmbares Salz oder Stereoisomer davon zur Verwendung in der Therapie.

9. Eine Verbindung gemäß einem der Ansprüche 1 bis 6 oder ein pharmazeutisch annehmbares Salz oder Stereoisomer davon zur Verwendung bei der Behandlung oder Prävention von Krebs.

10. Eine Verbindung gemäß einem der Ansprüche 1 bis 6 oder ein pharmazeutisch annehmbares Salz oder Stereoisomer davon zur Verwendung bei der Prävention oder Modulierung von Krebsmetastasen.

11. Eine Verbindung zur Verwendung gemäß Anspruch 9, wobei der Krebs ausgewählt ist aus Ovarialkarzinom, Leberzellkarzinom bei Kindern, metastatischen Kopf- und Hals-Plattenepithelkarzinomen, Magenkrebs, Brustkrebs, Dickdarmkrebs, Gebärmutterhalskrebs, Lungenkrebs, Nasen-Rachen-Krebs, Bauchspeicheldrüsenkrebs, Glioblastomen und Sarkomen.

12. Eine Verbindung zur Verwendung gemäß Anspruch 9, wobei der Krebs ausgewählt ist aus Karzinomen des Gehirns, des Urogenitaltrakts, des Lymphsystems, des Magens, des Kehlkopfes und der Lunge.

13. Eine Verbindung zur Verwendung gemäß Anspruch 9, wobei der Krebs ausgewählt ist aus histiozytärem Lymphom, Lungenadenokarzinom, kleinzelligen Lungenkarzinomen, Bauchspeicheldrüsenkrebs, Leberkrebs, Magenkrebs, Darmkrebs, multiplem Myelom, Glioblastomen und Brustkrebs.

14. Die Verwendung einer Verbindung gemäß einem der Ansprüche 1 bis 8 oder eines pharmazeutisch annehmbaren Salzes oder Stereoisomers davon zur Herstellung eines Medikaments zur Verwendung gemäß einem der Ansprüche 9 bis 13.

15. Eine Kombination aus einer Verbindung nach einem der Ansprüche 1 bis 6 oder einem pharmazeutisch annehmbaren Salz oder Stereoisomer davon und bekannten therapeutischen Mitteln und Mitteln gegen Krebs.

## Revendications

1. Composé de formule I: 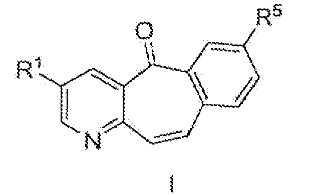 ou un de ses sels ou stéréoisomères pharmaceutiquement acceptables, dans lequel
a est indépendamment 0 ou 1 ;
b est indépendamment 0 ou 1 ;
m est indépendamment 0, 1, ou 2;
R¹ est choisi parmi les groupes aryle, hétérocyclyle et NR¹⁰R¹¹; lesdits groupes aryle et hétérocyclyle étant éventuellement substitués par un à cinq substituants, chaque substituant indépendamment choisi parmi R⁸;
R⁵ est choisi parmi les groupes alkyle en C₁₋₆, alcényle en C₂₋₆, OH, -O-alkyle en C₁₋₆, -O-C(=O)-alkyle en C₁₋₆, -O-aryle, S(O)ₘR^{a}, -C(=O)NR¹⁰R¹¹, -NHS(O)₂NR¹⁰R¹¹ et NR¹⁰R¹¹, chaque groupe alkyle, alcényle et aryle étant éventuellement substitué par un à cinq substituants, chaque substituant indépendamment choisi parmi R⁸;
R⁸ est indépendamment: un groupe (C=O)ₐO_{b}-alkyle en C₁-C₁₀, (C=O)ₐO_{b}-aryle, alcényle en C₂-C₁₀, alcynyle en C₂-C₁₀, (C=O)ₐO_{b}-hétérocyclyle, CO₂H, halogène, CN, OH, O_{b}-perfluoroalkyle en C₁-C₆, Oₐ(C=O)_{b}NR¹⁰R¹¹, S(O)ₘR^{a}, S(O)₂NR¹⁰R¹¹, OS(=O)R^{a}, oxo, CHO, (N=O)R¹⁰R¹¹, ou (C=O)ₐO_{b}-cycloalkyle en C₃-C₈,
lesdits groupes alkyle, aryle, alcényle, alcynyle, hétérocyclyle, et cycloalkyle étant éventuellement substitués par un, deux ou trois substituants choisis parmi R⁹;
R⁹ est indépendamment choisi parmi: un groupe (C=O)ₐO_{b}-alkyle en C₁-C₁₀, O_{b}-perfluoroalkyle en C₁-C₃, oxo, OH, halogène, CN, alcényle en C₂-C₁₀, alcynyle en C₂-C₁₀, (C=O)ₐO_{b}-cycloalkyle en C₃-C₆, (C=O)ₐO_{b}-(alkylène en C₀-C₆)-aryle, (C=O)ₐO_{b}-(alkylène en C₀-C₆)-hétérocyclyle, (C=O)ₐO_{b}-(alkylène en C₀-C₆)-N(R^{b})₂, C(O)R^{a}, (alkylène en C₀-C₆)-CO₂R^{a}, C(O)H, (alkylène en C₀-C₆)-CO₂H, C(O)N(R^{b})₂, S(O)ₘR^{a}, et S(O)₂NR¹⁰R¹¹;
lesdits groupes alkyle, alcényle, alcynyle, cycloalkyle, aryle, et hétérocyclyle étant éventuellement substitués par un, deux ou trois substituants choisis parmi R^{b}, OH, alcoxy en C₁-C₆, halogène, CO₂H, CN, O(C=O)-alkyle en C₁-C₆, oxo, et N(R^{b})₂;
R¹⁰ et R¹¹ sont indépendamment choisis parmi: H, un groupe (C=O)O_{b}-alkyle en C₁-C₁₀, (C=O)O_{b}-cycloalkyle en C₃-C₈, (C=O)O_{b}-aryle, (C=O)O_{b}-hétérocyclyle, alkyle en C₁-C₁₀, aryle, alcényle en C₂-C₁₀, alcynyle en C₂-C₁₀, hétérocyclyle, cycloalkyle en C₃-C₈, SO₂R^{a}, et (C=O)NR^{b}₂,
lesdits groupes alkyle, cycloalkyle, aryle, hétérocyclyle, alcényle, et alcynyle, étant éventuellement substitués par un, deux ou trois substituants choisis parmi R⁸ ou
R¹⁰ et R¹¹ peuvent être pris ensemble avec l'azote auquel ils sont attachés pour former un hétérocycle monocyclique ou bicyclique ayant 5-7 chaînons dans chaque cycle et contenant éventuellement, en plus de l'azote, un ou deux hétéroatomes supplémentaires choisis parmi N, O et S, ledit hétérocycle monocyclique ou bicyclique étant éventuellement substitué par un, deux ou trois substituants choisis parmi R⁹,
R^{a} est indépendamment choisi parmi: un groupe alkyle en C₁-C₆, alcényle en C₂-C₆, cycloalkyle en C₃-C₆, aryle, -(alkylène en C₁-C₆)aryle, hétérocyclyle et -(alkylène en C₁-C₆)hétérocyclyle; et
R^{b} est indépendamment choisi parmi: H, un groupe alkyle en C₁-C₆, aryle, - (alkylène en C₁-C₆)aryle, hétérocyclyle, -(alkylène en C₁-C₆)hétérocyclyle, cycloalkyle en C₃-C₆, (C=O)O-alkyle en C₁-C₆, (C=O)-alkyle en C₁-C₆ ou S(O)₂R^{a}.

2. Composé selon la revendication 1 choisi parmi les suivants:
3-phényl-7-vinyl-5H-benzo[4,5]cyclohepta[1,2-b]pyridin-5-one;
7-éthyl-3-phényl-5H-benzo[4,5]cyclohepta[1,2-b]pyridin-5-one;
7-[(2,4-diméthoxybenzyl)amino]-3-phényl-5H-benzo[4,5]cyclohepta[1,2-b]pyridin-5-one;
7-amino-3-phényl-5H-benzo[4,5]cyclohepta[1,2-b]pyridin-5-one;
2-hydroxy-N-(5-oxo-3-phényl-5H-benzo[4,5]cyclohepta[1,2-b]pyridin-7-yl)propanamide;
N-méthyl-5-oxo-3-phényl-5H-benzo[4,5]cyclohepta[1,2-b]pyridine-7-carboxamide;
7-isobutyl-3-phényl-5H-benzo[4,5]cyclohepta[1,2-b]pyridin-5-one;
N-(5-oxo-3-phényl-5H-benzo[4,5]cyclohepta[1,2-b]pyridin-7-yl)méthanesulfonamide;
N-[5-oxo-3-(3-thiényl)-5H-benzo[4,5]cyclohepta[1,2-b]pyridin-7-yl]méthanesulfonamide;
7-amino-3-(1-méthyl-1H-pyrazol-4-yl)-5H-benzo[4,5]cyclohepta[1,2-b]pyridin-5-one;
7-(isopropylamino)-3-(1-méthyl-1H-pyrazol-4-yl)-5H-benzo[4,5]cyclohepta[1,2-b]pyridin-5-one;
N-[(2R)-1,4-dioxan-2-ylméthyl]-N-méthyl-N'-[3-(1-méthyl-1H-pyrazol-4-yl)-5-oxo-5H-benzo[4,5]cyclohepta[1,2-b]pyridin-7-yl]sulfamide;
N-[(2S)-1,4-dioxan-2-ylméthyl]-N-méthyl-N'-[3-(1-méthyl-1H-pyrazol-4-yl)-5-oxo-5H-benzo[4,5]cyclohepta[1,2-b]pyridin-7-yl]sulfamide;
N-[1,4-dioxan-2-ylméthyl]-N-méthyl-N'-[3-(1-méthyl-1H-pyrazol-4-yl)-5-oxo-5H-benzo[4,5]cyclohepta[1,2-b]pyridin-7-yl]sulfamide racémique;
N-méthyl-N'-[3-(1-méthyl-1H-pyrazol-4-yl)-5-oxo-5H-benzo[4,5]cyclohepta[1,2-b]pyridin-7-yl]-N-(tétrahydrofuran-3-yl)sulfamide;
N-méthyl-N'-[3-(1-méthyl-1H-pyrazol-4-yl)-5-oxo-5H-benzo[4,5]cyclohepta[1,2-b]pyridm-7-yl]-N-({3R}-tétrahydrofuran-3-yl)sulfamide;
N-méthyl-N'-[3-(1-méthyl-1H-pyrazol-4-yl)-5-oxo-5H-benzo[4,5]cyclohepta[1,2-b]pyridin-7-yl]-N-({3S}-tétrahydrofdran-3-yl)sulfamide;
N-(5-oxo-3-pyridin-4-yl-5H-benzo[4,5]cyclohepta[1,2-b]pyridin-7-yl)méthanesulfonamide;
N-[5-oxo-3-(1H-pyrazol-3-yl)-5H-benzo[4,5]cyclohepta[1,2-b]pyridin-7-yl]méthanesulfonamide;
N-[5-oxo-3-(1,3-thiazol-4-yl)-5H-benzo[4,5]cyclohepta[1,2-b]pyridin-7-yl]méthanesulfonamide;
N-[3-(1-méthyl-1H-pyrazol-4-yl)-5-oxo-5H-benzo[4,5]cyclohepta[1,2-b]pyridin-7-yl]méthanesulfonamide;
N-[5-oxo-3-(1H-pyrazol-4-yl)-5H-benzo[4,5]cyclohepta[1,2-b]pyridin-7-yl]méthanesulfonamide;
N-(3-{1-[2-(diméthylamino)éthyl]-1H-pyrazol-4-yl}-5-oxo-5H-benzo[4,5]cyclohepta[1,2-b]pyridin--yl)méthanesulfonamide;
N-{3-[1-(2-morpholin-4-yl-2-oxoéthyl)-1H-pyrazol-4-yl]-5-oxo-5H-benzo[4,5]cyclohepta[1,2-b]pyridin-7-yl}méthanesulfonamide,
N-(4-{7-[(méthylsulfonyl)amino]-5-oxo-5H-benzo[4,5]cyclohepta[1,2-b]pyridin-3-yl}phényl)méthanesulfonamide;
N-[3-(1-cyclopentyl-1H-pyrazol-4-yl)-5-oxo-5H-benzo[4,5]cyclohepta[1,2-b]pyridin-7-yl]méthanesulfonamide;
N-{3-[1-(3,3-diméthyl-2-oxobutyl)-1H-pyrazol-4-yl]-5-oxo-5H-benzo[4,5]cyclohepta[1,2-b]pyridin-7-yl}méthanesulfonamide;
N-[2-(1-méthylpyrrolidin-2-yl)éthyl]-3-{7-[(méthylsulfonyl)amino]-5-oxo-5H-benzo[4,5]cyclohepta[1,2-b]pyridin-3-yl}benzamide;
N,N-diméthyl-N'-[3-(1-méthyl-1H-pyrazol-4-yl)-5-oxo-5H-benzo[4,5]cyclohepta[1,2-b]pyridin-7-yl] sulfamide;
7-(5-méthyl-1,1-dioxydo-1,2,5-thiadiazolidin-2-yl)-3-(1-méthyl-1H-pyrazol-4-yl)-5H-benzo[4,5]cyclohepta[1,2-b]pyridin-5-one;
7-[(2,4-diméthoxybenzyl)amino]-3-(3-thiényl)-5H-benzo[4,5]cyclohepta[1,2-b]pyridin-5-one;
7-[(2,4-diméthoxybenzyl)amino]-3-(1H-pyrazol-3-yl)-5H-benzo[4,5]cyclohepta[1,2-b]pyridin-5-one;
N-méthyl-N'-[3-(1-méthyl-1H-pyrazol-4-yl)-5-oxo-5H-benzo[4,5]cyclohepta[1,2-b]pyridin-7-yl]-N-(tétrahydrofuran-3-yl)sulfamide;
7-[(imidazo[1,2-a]pyridin-3-ylméthyl)amino]-3-(1-méthyl-1H-pyrazol-4-yl)-5H-benzo [4,5]cyclohepta[1,2-b]pyridin-5-one;
7-{[(1-méthyl-5-oxopyrrolidin-2-yl)méthyl]amino}-3-(1-méthyl-1H-pyrazol-4-yl)-5H-benzo[4,5]cyclohepta[1,2-b]pyridin-5-one,
N-méthyl-N'-[3-(1-méthyl-1H-pyrazol-4-yl)-5-oxo-5H-benzo[4,5]cyclohepta[1,2-b]pyridin-7-yl]-N-(tétrahydro-2H-pyran-2-ylméthyl)sulfamide;
N-[3-(1-méthyl-1H-pyrazol-4-yl)-5-oxo-5H-benzo[4,5]cyclohepta[1,2-b]pyridin-7-yl]-N'-(tétrahydrofuran-3-yl)sulfamide;
N-[3-(1-méthyl-1H-pyrazol-4-yl)-5-oxo-5H-benzo[4,5]cyclohepta[1,2-b]pyridin-7-yl]morpholine-4-sulfonamide;
N-[3-(4-isopropylpipérazin-1-yl)-5-oxo-5H-benzo[4,5]cyclohepta[1,2-b]pyridin-7-yl]méthanesulfonamide;
3-(4-isopropylpipérazin-1-yl)-7-phényl-1H-benzo[4,5]cyclohepta[1,2-b]pyridin-5-one;
N-(3-morpholin-4-yl-5-oxo-5H-benzo[4,5]cyclohepta[1,2b]pyridin-7-yl)méthanesulfonamide;
N-(3-anilino-5-oxo-5H-benzo[4,5]cyclohepta[1,2-b]pyridin-7-yl)méthanesulfonamide;
N-[3-(cyclohexylamino)-5-oxo-5H-benzo[4,5]cyclohepta[1,2-b]pyridin-7-yl]méthanesulfonamide;
N-[5-oxo-3-(pyridin-4-ylamino)-5H-benzo[4,5]cyclohepta[1,2-b]pyridin-7-yl]méthanesulfonamide;
N-(2,4-diméthoxybenzyl)-N-(5-oxo-3-phényl-5H-benzo[4,5]cyclohepta[1,2-b]pyridin-7-yl)éthylènesulfonamide;
N-(5-oxo-3-phényl-5H-benzo[4,5]cyclohepta[1,2b]pyridin-7-yl)éthylènesulfonamide;
N-(5-oxo-3-phényl-5H-benzo[4,5]cyclohepta[1,2-b]pyridin-7-yl)-2-pyrrolidin-1-yléthanesulfonamide,
[3-(1-méthyl-1H-pyrazol-4-yl)-5-oxo-5H-benzo[4,5]cyclohepta[1,2b]pyridin-7-yl]amidophosphate de diméthyle;
7-[(1R)-1-hydroxyéthyl]-3-(1-méthyl-1H-pyrazol-4-yl)-5H-benzo[4,5]cyclohepta[1,2-b]pyridin-5-one;
7-[(1S)-1-hydroxyéthyl]-3-(1-méthyl-1H-pyrazol-4-yl)-5H-benzo[4,5]cyclohepta[1,2-b]pyridin-5-one;
7-(2-{[tert-butyl(diméthyl)silyl]oxy}éthyl)-3-(1-méthyl-1H-pyrazol-4-yl)-5H-benzo[4,5]cyclohepta[1,2-b]pyridin-5-one;
7-(2-hydroxyéthyl)-3-(1-méthyl-1H-pyrazol-4-yl)-5H-benzo[4,5]cyclohepta[1,2-b]pyridin-5-one;
7-(1,2-dihydroxyéthyl)-3-(1-méthyl-1H-pyrazol-4-yl)-5H-benzo[4,5]cyclohepta[1,2-b]pyridin-5-one;
7-[(1R)-1-méthoxyéthyl]-3-(1-méthyl-1H-pyrazol-4-yl)-5H-benzo[4,5]cyclohepta[1,2-b]pyridin-5-one;
7-[(1S)-1-méthoxyéthyl]-3-(1-méthyl-1H-pyrazol-4-yl)-5H-benzo[4,5]cyclohepta[1,2-b]pyridin-5-one;
4-[2-(3-chloro-5-oxo-5H-benzo[4,5]cyclohepta[1,2-b]pyridin-7-yl)-2-hydroxyéthyl]pipérazine-1-carboxylate de tert-butyle;
4-{2-hydroxy-2-[3-(1-méthyl-1H-pyrazol-4-yl)-5-oxo-5H-benzo[4,5]cyclohepta[1,2-b]pyridin-7-yl]éthyl}pipérazine-1-carboxylate de tert-butyle;
7-(1-hydroxy-2-pipérazin-1-yléthyl)-3-(1-méthyl-1H-pyrazol-4-yl)-5H-benzo[4,5]cyclohepta[1,2-b]pyridin-5-one;
ou un de ses sels ou stéréoisomères pharmaceutiquement acceptables.

3. Composé selon la revendication 1 qui est: 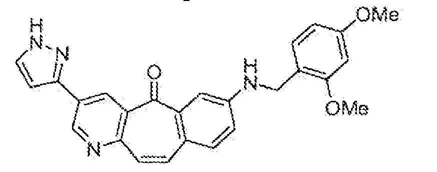 la 7-[(2,4-diméthoxybenzyl)amino]-3-(1H-pyrazol-3-yl)-5H-benzo[4,5]cyclohepta[1,2-b]pyridin-5-one
ou un de ses sels pharmaceutiquement acceptables.

4. Composé selon la revendication 1 qui est: 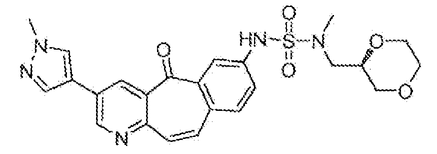 le N-[(2R)-1,4-dioxan-2-ylméthyl]-N-méthyl-N'-[3-(1-méthyl-1H-pyrazol-4-yl)-5-oxo-5H-benzo[4,5]cyclohepta[1,2-b]pyridin-7-yl]sulfamide
ou un de ses sels ou stéréoisomères pharmaceutiquement acceptables.

5. Composé selon la revendication 1 qui est: 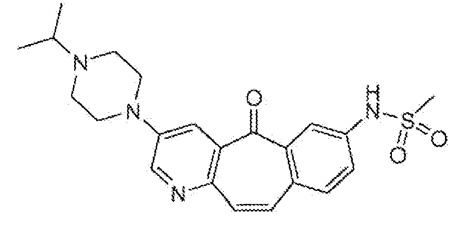 le N-[3-(4-isopropylpipérazin-1-yl)-5-oxo-5H-benzo[4,5]cyclohepta[1,2-b]pyridin-7-yl]-méthanesulfonamide
ou un de ses sels pharmaceutiquement acceptables.

6. Composé selon la revendication 1 qui est: 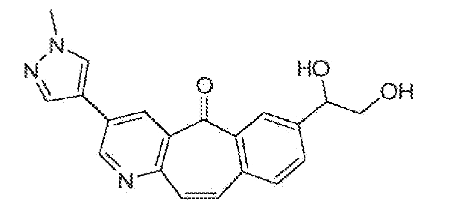 la 7-(1,2-dihydroxyéthyl)-3-(1-méthyl-1H-pyrazol-4-yl)-5H-benzo[4,5]cyclohepta[1,2-b]pyridin-5-one
ou un de ses sels ou stéréoisomères pharmaceutiquement acceptables.

7. Composition pharmaceutique qui est constituée d'un composé selon l'une quelconque des revendications précédentes, ou d'un de ses sels ou stéréoisomères pharmaceutiquement acceptables, et d'un véhicule pharmaceutiquement acceptable.

8. Composé selon l'une quelconque des revendications 1 à 6, ou un de ses sels ou stéréoisomères pharmaceutiquement acceptables, destiné à être utilisé en thérapeutique.

9. Composé selon l'une quelconque des revendications 1 à 6, ou un de ses sels ou stéréoisomères pharmaceutiquement acceptables, destiné à être utilisé dans le traitement ou la prévention du cancer.

10. Composé selon l'une quelconque des revendications 1 à 6, ou un de ses sels ou stéréoisomères pharmaceutiquement acceptables, destiné à être utilisé dans la prévention ou la modulation des métastases du cancer.

11. Composé à utiliser selon la revendication 9, le cancer étant choisi parmi le cancer des ovaires, le carcinome hépatocellulaire de l'enfant, l'épithélioma malpighien spinocellulaire de la tête et du cou métastatique, le cancer gastrique, le cancer du sein, le cancer colorectal, le cancer du col de l'utérus, le cancer du poumon, le cancer du nasopharynx, le cancer du pancréas, le glioblastome et les sarcomes.

12. Composé à utiliser selon la revendication 9, le cancer étant choisi parmi les cancers du cerveau, de l'appareil génito-urinaire, du système lymphatique, de l'estomac, du larynx et du poumon.

13. Composé à utiliser selon la revendication 9, le cancer étant choisi parmi le lymphome histiocytaire, l'adénocarcinome pulmonaire, les cancers bronchiques à petites cellules, le cancer du pancréas, le cancer du foie, le cancer gastrique, le cancer du côlon, le myélome multiple, les glioblastomes et le cancer du sein.

14. Utilisation d'un composé selon l'une quelconque des revendications 1 à 8, ou d'un de ses sels ou stéréoisomères pharmaceutiquement acceptables, pour la préparation d'un médicament à utiliser selon l'une quelconque des revendications 9 à 13.

15. Association d'un composé selon l'une quelconque des revendications 1 à 6, ou d'un de ses sels ou stéréoisomères pharmaceutiquement acceptables, et d'agents thérapeutiques et agents anticancéreux connus.
